# EUROPÄISCHE PATENTSCHRIFT

(11) **EP 1 461 335 B1**
(45) Veröffentlichungstag und Bekanntmachung des Hinweises auf die Patenterteilung: **28.02.2007**
(21) Anmeldenummer: 02793023.9
(22) Anmeldetag: 16.12.2002
(51) Int. Cl.: C07D 453/02, C07D 519/00, A61K 31/435

(54) **2-HETEROARYLCARBONSÄUREAMIDE**
2-HETEROARYLCARBOXYLIC ACID AMIDES
AMIDES D'ACIDE 2-HETEROARYLCARBOXYLIQUE

(30) Priorität: 27.12.2001 DE 10164139
(43) Veröffentlichungstag der Anmeldung: 29.09.2004
(73) Patentinhaber: Bayer HealthCare AG, 51368 Leverkusen (DE)
(72) Erfinder: HENDRIX, Martin, 51519 Odenthal (DE); BÖSS , Frank-Gerhard, Berkshire SL5 7DF (GB); ERB, Christina, 65830 Kriftel (DE); FLESSNER, Timo, 42113 Wuppertal (DE); VAN KAMPEN, Marja, 63263 Neu-Isenburg (DE); LUITHLE, Joachim, 42489 Wülfrath (DE); METHFESSEL, Christoph, 42327 Wuppertal (DE); WIESE, Welf-Burkhard, 42929 Wermelskirchen (DE)
(86) Internationale Anmeldenummer: PCT/EP2002/014288
(87) Internationale Veröffentlichungsnummer: WO 2003/055878

(56) Entgegenhaltungen:
- EP-A- 0 512 350
- WO-A-96/33186
- WO-A-02/100857
- US-A- 4 605 652
- US-A- 5 599 937
- PATENT ABSTRACTS OF JAPAN vol. 2002, no. 05, 3. Mai 2002 (2002-05-03) & JP 2002 030084 A (MITSUBISHI PHARMA CORP), 29. Januar 2002 (2002-01-29) in der Anmeldung erwähnt

## Beschreibung

Die Erfindung betrifft neue 2-Heteroarylcarbonsäureamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi and Changeux, *Neuropharmacol.* **1995,** *34,* 563-582). Ein funktioneller nAChR besteht aus fünf Untereinheiten, die unterschiedlich (bestimmte Kombinationen von α1-9 und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee and Role, *Annu. Rev. Physiol.,* **1995,** *57,* 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte wie z.B. die Stimulierung von nAChR in der Muskulatur vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani and Levin, *Biol. Psychiatry* **2001,** *49*, 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., *J Neurosci.* **1993,** *13,* 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide and Leslie, *Mol. Neurobiol.* **1999,** *20***,** 1-16).

Bestimmte N-(1-Aza-bieyclo[2.2.2]oct-3-yl)-heteroarylcarbonsäureamide zur Behandlung von u.a. Psychosen sind in der DE-A-37 24 059 beschrieben.

N-(Aza-bicycloalkyl)-heteroarylcarbonsäureamide, insbesondere N-(1-Aza-bicyclo-[2.2.2]oct-4-yl)-benzothiophen-3-carbonsäureamide, werden in der WO 93/15073 bzw. in der EP-A-0 485 962 als Zwischenstufen für die Synthese von pharmazeutisch wirksamen Verbindungen offenbart.

Aus der JP 14030084A sind 1-Azabicycloalkane und ihre Wirkung auf den nikotinischen α7-Rezeptor bekannt.

Aus US-A-4,605,652 und der EP-A-0 372 335 sind beispielsweise N-(1-Aza-bicyclo-[2.2.2]oct-3-yl)-thiophen-2-carbonsäureamid und seine gedächtnisverbessernde Wirkung bekannt.

Die vorliegende Erfindung betrifft Verbindungen der Formel (I) 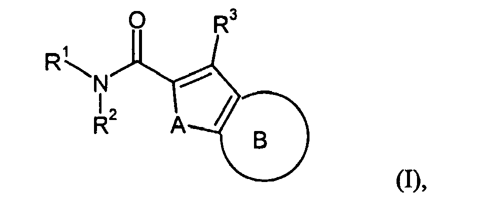 in welcher
- R¹: für 1-Aza-bicyclo[2.2.2]oct-3-yl steht,
- R²: für Wasserstoff oder C₁-C₆-Alkyl steht,
- R³: für Wasserstoff, Halogen oder C₁-C₆-Alkyl steht,
- A: für Sauerstoff oder Schwefel steht,
- A: für Sauerstoff oder Schwefel steht,
und
- der Ring B: für Benzo, Pyrimido, Pyridazo oder Pyridazino, die gegebenenfalls durch Reste ausgewählt aus der Gruppe Wasserstoff, Halogen, C₁-C₆-Alkanoyl, Carbamoyl, Cyano, Trifluormethyl, Trifluormethoxy, Nitro, Amino, C₁-C₆-Acylamino, C₁-C₆-Alkyl, C₁-C₆-Alkoxy, C₁-C₆-Alkylthio, C₁-C₆-Alkylamino, Heteroarylcarbonylamino, Arylcarbonylamino, C₁-C₄-Alkylsulfonylamino, Di-(C₁-C₄-alkylsulfonyl)amino, Arylsulfonylamino, Di-(arylsulfonyl)amino, C₃-C₆-Cycloalkylcarbonylmethyl, 1,3-Dioxa-propan-1,3-diyl, Amino(hydroxyimino)methyl und Benzo substituiert sind, steht,
und deren Salze, Solvate und Solvate der Salze.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze, Solvate oder Solvate der Salze vorliegen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren; Carbonsäuren oder Sulfonwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder N-Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten im Allgemeinen die folgende Bedeutung:
C₁-C₆- und C₁-C₄-Alkoxy stehen für einen geradkettigen oder verzweigten Alkoxy-Rest mit 1 bis 6 bzw. 1 bis 4 Kohlenstoffatomen. Bevorzugt ist ein geradkettiger oder verzweigter Alkoxy-Rest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, tert.-Butoxy, n-Pentoxy und n-Hexoxy.
C₁-C₆- und C₁-C₄-Alkyl stehen für einen geradkettigen oder verzweigten Alkyl-Rest mit 1 bis 6 bzw. 1 bis 4 Kohlenstoffatomen. Bevorzugt ist ein geradkettiger oder verzweigter Alkyl-Rest mit 1 bis 4, besonders bevorzugt mit 1 bis 3 Kohlenstoffatomen. Beispielsweise und vorzugsweise seien genannt: Methyl, Ethyl, n-Propyl, Isopropyl, tert.-Butyl, n-Pentyl und n-Hexyl.
(C₁-C₆-Alkanoyl steht für einen geradkettigen oder verzweigten Alkyl-Rest mit 1 bis 6 Kohlenstoffatomen, der in der 1-Position ein doppelt gebundenes Sauerstoffatom trägt und über die 1-Position verknüpft ist. Bevorzugt ist ein geradkettiger oder verzweigter Alkanoyl-Rest mit 1 bis 4, besonders bevorzugt mit 1 bis 2 Kohlenstoffatomen. Beispielhaft und vorzugsweise seien genannt: Formyl, Acetyl, Propionyl, n-Butyryl, Isobutyryl, Pivaloyl und n-Hexanoyl.
C₁-C₆-Alkylamino steht für einen geradkettigen oder verzweigten Alkylamino-Rest mit 1 bis 6 Kohlenstoffatomen, bevorzugt mit 1 bis 4, besonders bevorzugt mit 1 bis 3 Kohlenstoffatomen. Nicht-limitierende Beispiele umfassen Methylamino, Ethylamino, n-Propylamino, Isopropylamino und tert.-Butylamino.
(C₁-C₆)-Acylamino steht für eine Amino-Gruppe mit einem geradkettigen oder verzweigten Alkanoyl-Substituenten, der 1 bis 6 Kohlenstoffatome aufweist und über die Carbonylgruppe verknüpft ist. Bevorzugt ist ein Acylamino-Rest mit 1 bis 4, besonders bevorzugt mit 1 bis 2 Kohlenstoffatomen. Beispielhaft und vorzugsweise seien genannt: Formamido, Acetamido, Propionamido, n-Butyramido und Pivaloylamido.
C₁-C₄-Alkylsulfonylamino steht für einen geradkettigen oder verzweigten Alkylsulfonylamino-Rest mit 1 bis 4, bevorzugt mit 1 bis 3 Kohlenstoffatomen. Nicht-limitierende Beispiele umfassen Methylsulfonylamino, Ethylsulfonylamino, n-Propylsulfonylamino, Isopropylsulfonylamino, tert.-Butylsulfonylamino.
Arylsulfonylamino steht für einen Naphthyl- oder Phenylsulfonylamino-Rest und bevorzugt für einen Phenylsulfonylamino-Rest.
C₁-C₆-Alkylthio steht für einen geradkettigen oder verzweigten Alkylthio-Rest mit 1 bis 6 Kohlenstoffatomen. Bevorzugt ist ein geradkettiger oder verzweigter Alkylthio-Rest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.-Butylthio, n-Pentylthio und n-Hexylthio.
Arylcarbonyl steht für einen Naphthyl- oder Phenylcarbonyl-Rest und bevorzugt für einen Phenylcarbonyl-Rest (Benzoyl-Rest).
Heteroarylcarbonyl steht für einen Heteroarylcarbonyl-Rest mit einem 5- bis 6-gliedrigen, bevorzugt 5-gliedrigen Heteroarylring mit bis zu 2 Heteroatomen ausgewählt aus der Gruppe O, S und N. Nicht-limitierende Beispiele umfassen Thienylcarbonyl, Furylcarbonyl, Pyrrolylcarbonyl, Thiazolylcarbonyl, Oxazolylcarbonyl, Imidazolylcarbonyl, Pyridylcarbonyl, Pyrimidylcarbonyl.
C₃-C₆-Cycloalkylcarbonylmethyl steht für eine monocyclische Cycloalkyl-Gruppe mit 3 bis 6 Kohlenstoffatomen, die über eine Carbonylmethyl-Gruppe [-C(=O)-CH₂-] verknüpft ist. Nicht-limitierende Beispiele umfassen Cyclopropylcarbonylmethyl, Cyclopentylearbonylmethyl und Cyclohexylcarbonylmethyl.
Halogen steht für Fluor, Chlor, Brom und Jod. Bevorzugt sind Fluor, Chlor und Brom. Besonders bevorzugt sind Fluor und Chlor.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der Formel (I),
in welcher
- R¹: für 1-Aza-bicyclo[2.2.2]oct-3-yl steht,
- R²: für Wasserstoff oder (C₁-C₆)-Alkyl steht,
- R³: für Wasserstoff, Halogen oder (C₁-C₆)-Alkyl steht,
- A: für Sauerstoff oder Schwefel steht,
und
- der Ring B: für Benzo, Pyrimido, Pyridazo oder Pyridazino, die gegebenenfalls durch Reste ausgewählt aus der Gruppe Wasserstoff, Halogen, Formyl, Carbamoyl, Cyano, Trifluormethyl, Trifluormethoxy, Nitro, Amino, Formamido, Acetamido, (C₁-C₆)-Alkyl, (C₁-C₆)-Alkoxy, (C₁-C₆)-Alkylthio und Benzo substituiert sind, steht.

Besonders bevorzugt sind Verbindungen der Formel (I),
in welcher
- R¹: für 1-Aza-bicyclo[2.2.2]oct-3-yl steht,
- R²: für Wasserstoff steht,
- R³: für Wasserstoff, Chlor, Fluor oder Methyl steht,
- A: für Sauerstoff oder Schwefel steht,
und
- der Ring B: für Benzo, welches gegebenenfalls durch 1 bis 3 Reste ausgewählt aus der Gruppe Wasserstoff, Halogen, Amino, Formamido, Acetamido, C₁-C₄-Alkyl, C₁-C₄-Alkoxy, C₁-C₄-Alkylthio, C₁-C₄-Alkylamino, Furylcarbonylamino, Phenylcarbonylamino, Methylsulfonylamino, Di-(phenylsulfonyl)amino, Cyclopropylcarbonylmethyl, 1,3-Dioxapropan-1,3-diyl, Amino(hydroxyimino)methyl und Benzo substituiert sind, steht.

Insbesondere bevorzugt sind Verbindungen der Formel (Ia) 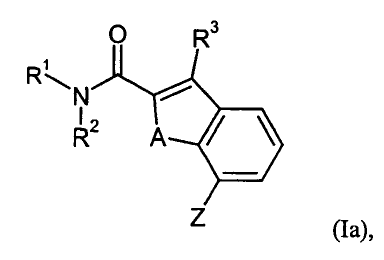 in welcher
- R¹: für 1-Aza-bicyclo[2.2.2]oct-3-yl steht,
- R²: für Wasserstoff oder C₁-C₆-Alkyl steht,
- R³: für Wasserstoff, Halogen oder C₁-C₆-Alkyl steht,
- A: für Sauerstoff oder Schwefel steht,
und
- Z: für Wasserstoff, Halogen, Formyl, Carbamoyl, Cyano, Trifluormethyl, Trifluormethoxy, Nitro, Amino, Formamido, Acetamido, C₁-C₆-Alkyl, C₁-C₆-Alkoxy, C₁-C₆-Alkylthio, C₁-C₆-Alkylamino, Heteroarylcarbonylamino, Arylcarbonylamino, C₁-C₄-Alkylsulfonylamino, Di-(arylsulfonyl)amino, C₃-C₆-Cycloalkylcarbonylmethyl oder Amino(hydroxyimino)methyl steht,

Ganz besonders bevorzugt sind Verbindungen der Formel (Ia),
in welcher
- R¹: für 1-Aza-bicyclo[2.2.2]oct-3-yl steht,
- R²: für Wasserstoff steht,
- R³: für Wasserstoff, Chlor, Fluor oder Methyl steht,
- A: für Sauerstoff oder Schwefel steht,
und
- Z: für Wasserstoff, Halogen, Formyl, Carbamoyl, Cyano, Trifluormethyl, Trifluormethoxy, Nitro, Amino, Formamido, Acetamido, Methyl, Ethyl, Methoxy, Ethoxy, C₁-C₄-Alkylamino, Furylcarbonylamino, Phenylcarbonylamino, Methylsulfonylamino, Di-(phenylsulfonyl)amino, Cyclopropylcarbonylmethyl oder Amino(hydroxyimino)methyl steht.

Vor allem bevorzugt sind Verbindungen der Formel (Ia),
in welcher
- R¹: für (*3R*)-1-Aza-bicyclo[2.2.2]oct-3-yl steht,
- R²: für Wasserstoff steht,
- R³: für Wasserstoff, Chlor, Fluor oder Methyl steht,
- A: für Sauerstoff oder Schwefel steht,
und
- Z: für Wasserstoff, Halogen, Formyl, Carbamoyl, Cyano, Trifluormethyl, Trifluormethoxy, Nitro, Amino, Formamido, Acetamido, Methyl, Ethyl, Methoxy, Ethoxy, C₁-C₄-Alkylamino, Furylcarbonylamino, Phenylcarbonylamino, Methylsulfonylamino, Di-(phenylsulfonyl)amino, Cyclopropyl-carbonylmethyl oder Amino(hydroxyimino)methyl steht.

Ebenfalls bevorzugt sind Verbindungen der Formel (I),
in welcher
- R¹: für (3*R*)-1-Aza-bicyclo[2.2.2]oct-3-yl steht,
und R², R³, A und der Ring B die oben angegebenen Bedeutungen haben.

Besonders bevorzugt sind Verbindungen der Formel (Ia),
in welcher
- R¹: für (3*R*)-1-Aza-bicyclo[2.2.2]oct-3-yl steht,
und R², R³, A und Z die oben angegebenen Bedeutungen haben.

Ebenfalls bevorzugt sind Verbindungen der Formel (I),
in welcher
- R²: für Wasserstoff oder Methyl steht,
und R¹, R³, A und der Ring B die oben angegebenen Bedeutungen haben.

Besonders bevorzugt sind Verbindungen der Formel (I),
in welcher
- R²: für Wasserstoff steht,
und R¹, R³, A und der Ring B die oben angegebenen Bedeutungen haben.

Ebenfalls bevorzugt sind Verbindungen der Formel (I),
in welcher
- R³: für Wasserstoff, Fluor, Chlor oder Methyl steht,
und R¹, R², A und der Ring B die oben angegebenen Bedeutungen haben.

Besonders bevorzugt sind Verbindungen der Formel (I),
in welcher
- R³: für Wasserstoff oder Methyl steht,
und R¹, R², A und der Ring B die oben angegebenen Bedeutungen haben.

Ganz besonders bevorzugt sind Verbindungen der Formel (I),
in welcher
- R³: für Wasserstoff steht,
und R¹, R², A und der Ring B die oben angegebenen Bedeutungen haben.

Ebenfalls bevorzugt sind Verbindungen der Formel (I),
in welcher
- A: für Schwefel steht,
und R¹, R², R³ und der Ring B die oben angegebenen Bedeutungen haben.

Ebenfalls bevorzugt sind Verbindungen der Formel (I),
in welcher
- A: für Sauerstoff steht,
und R¹, R², R³ und der Ring B die oben angegebenen Bedeutungen haben.

Besonders bevorzugt sind Verbindungen der Formel (Ia),
in welcher
- A: für Schwefel steht,
und R¹, R², R³ und Z die oben angegebenen Bedeutungen haben.

Ebenfalls besonders bevorzugt sind Verbindungen der Formel (Ia),
in welcher
in welcher
- A: für Sauerstoff steht,
und R¹, R², R³ und Z die oben angegebenen Bedeutungen haben.

Ebenfalls bevorzugt sind Verbindungen der Formel (I),
in welcher
- der Ring B: für Benzo, welches gegebenenfalls durch 1 bis 3 Reste ausgewählt aus der Gruppe Wasserstoff, Halogen, Formyl, Carbamoyl, Cyano, Trifluormethyl, Trifluormethoxy, Nitro, Amino, Formamido, Acetamido, C₁-C₄-Alkyl, C₁C₄-Alkoxy, C₁-C₄-Alkylthio, C₁-C₄-Alkylamino, Furylcarbonylamino, Phenylcarbonylamino, Methylsulfonylamino, Di-(phenylsulfonyl)amino, Cyclopropylcarbonylmethyl und Amino(hydroxyimino)methyl substituiert sind,
und R¹, R², R³ und A die oben angegebenen Bedeutungen haben.

Besonders bevorzugt sind Verbindungen der Formel (I),
in welcher
- der Ring B: für Benzo, welches gegebenenfalls durch 1 bis 3 Reste ausgewählt aus der Gruppe Wasserstoff, Halogen, Cyano, Trifluormethyl, Trifluormethoxy, Nitro, Amino, Formamido, Acetamido und (C₁-C₄)-Alkyl substituiert sind,
und R¹, R², R³ und A die oben angegebenen Bedeutungen haben.
in welcher
- der Ring B: für Benzo steht, wobei Benzo gegebenenfalls durch 1 bis 3 Reste ausgewählt aus der Gruppe Wasserstoff, Halogen, Cyano, Trifluormethyl, Trifluormethoxy, Nitro, Amino, Formamido, Acetamido und (C₁-C₄)-Alkyl substituiert ist,
und R¹, R², R³ und A die oben angegebene Bedeutung haben.

Ebenfalls besonders bevorzugt sind Verbindungen der Formel (Ia),
in welcher
- Z: für Wasserstoff, Halogen, Formyl, Carbamoyl, Cyano, Trifluormethyl, Trifluormethoxy, Nitro, Amino, Formamido, Acetamido, Methyl, Ethyl, Methoxy, Ethoxy, C₁-C₄-Alkylamino, Furylcarbonylamino, Phenylcarbonylamino, Methylsulfonylamino, Di-(phenylsulfonyl)amino, Cyclopropylcarbonylmethyl oder Amino(hydroxyimino)methyl steht,
und R¹, R², R³ und A die oben angegebenen Bedeutungen haben.

Ganz besonders bevorzugt sind Kombinationen von zwei oder mehreren der oben genannten Vorzugsbereiche.

Vor allem bevorzugt sind Verbindungen der Formel (I),
in welcher
- R¹: für (3*R*)-1-Aza-bicyclo[2.2.2]oct-3-yl steht,
- R¹: für (3*R*)-1-Aza-bicyclo[2.2.2]oct-3-yl steht,
- R² und R³: für Wasserstoff stehen,
- A: für Schwefel steht,
und
- der Ring B: für Benzo, welches gegebenenfalls durch 1 bis 3 Reste ausgewählt aus der Gruppe Wasserstoff, Halogen, Cyano, Trifluormethyl, Trifluormethoxy, Nitro, Amino, Formamido, Acetamido und (C₁-C₄)-Alkyl substituiert sind.

Die Erfindung betrifft weiterhin Verfahren zur Herstellung der Verbindungen der Formel (I), dadurch gekennzeichnet, dass man Verbindungen der allgemeinen Formel (II)

R¹R²NH (II),

in welcher R¹ und R² die oben genannte Bedeutung haben,
mit einer Verbindung der allgemeinen Formel (III) 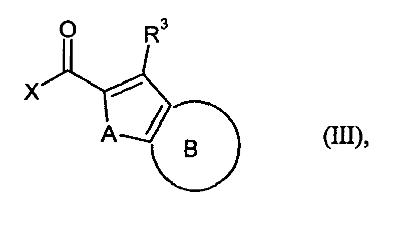 in welcher
R³, A und der Ring B die oben genannte Bedeutung haben, und
in einem inerten Lösungsmittel gegebenenfalls in Gegenwart eines Kondensationsmittels und gegebenenfalls in Gegenwart einer Base umsetzt.

Wenn X eine Abgangsgruppe ist, sind Chlor, Mesyloxy und Isobutyloxycarbonyloxy, besonders Chlor bevorzugt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin, bevorzugt sind Tetrahydrofuran, Dimethylformamid oder Chloroform.

Kondensationsmittel sind beispielsweise Carbodiimide wie z.B. N,N'-Diethyl-, N,N,'-Dipropyl-, N,N'-Diisopropyl-, N,N'-Dicydohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchlorformiat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetramethyluroniumhexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyluroniumhexafluorophosphat (HATU) oder Benzotriazol-1-yloxytris(dimethylamino)-phosphoniumhexafluorophosphat (BOP), oder Mischungen aus diesen.

Gegebenenfalls kann es vorteilhaft sein, diese Kondensationsmittel in Gegenwart eines Hilfsnucleophils wie z.B. 1-Hydroxybenzotriazol (HOBt) zu verwenden.

Basen sind beispielsweise Alkalicarbonate, wie z.B. Natrium- oder Kaliumcarbonat, oder -hydrogencarbonat, oder organische Basen wie Trialkylamine, z.B. Triethylamin, N-Methylmorpholin, N-Methylpiperidin, 4-N,N-Dimethylaminopyridin oder N,N-Diisopropylethylamin.

Bevorzugt ist O-(7-Azabenzotriazol-1-yl)-N,N,N',N'-tetramethyluroniumhexafluorophosphat (HATU) in Gegenwart von N,N-Diisopropylethylamin sowie die Kombination von N-(3-Dimethylaminoisopropyl)-N'-ethylcarbodiimid-Hydrochlorid (EDC) und 1-Hydroxybenzotriazol (HOBt) jeweils in Dimethylformamid.

Vorzugsweise wird das erfindungsgemäße Verfahren in einem Temperaturbereich von Raumtemperatur bis 50°C bei Normaldruck durchgeführt.

Die Verbindungen der allgemeinen Formeln (II) und (III) sind bekannt oder lassen sich nach bekannten Verfahren aus den entsprechenden Edukten synthetisieren (vgl. z.B. ,Comprehensive Heterocyclic Chemistry', Katritzki et al., Hrsg.; Elsevier, 1996).

So können beispielsweise substituierte Benzothiophen-2-carbonsäuren aus entsprechend substituierten 2-Halogenbenzaldehyden durch Reaktion mit Mercaptoessigsäuremethylester (siehe z.B. A.J. Bridges, A. Lee, E.C. Maduakor, Schwartz, *Tetrahedron Lett.* **1992**, *33*, 7499) und anschließende Verseifung des Esters erhalten werden (Syntheseschema 1):

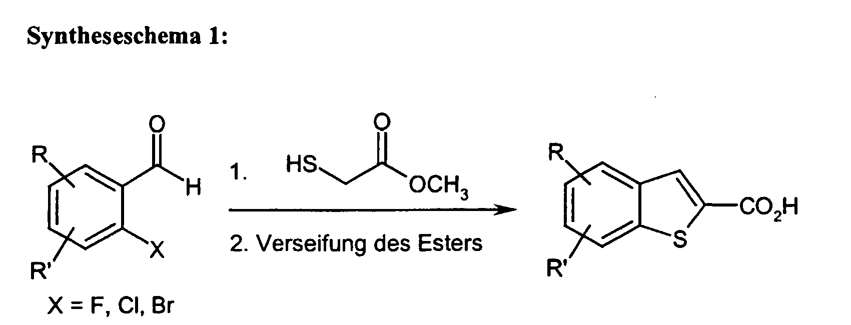

Zur Synthese der entsprechenden Pyrido-Derivate ist ausgehend von 2-Halogenbenzonitrilen eine Reaktion mit Mercaptoessigsäuremethylester zu den 3-Aminobenzothiophen-2-carbonsäureestern möglich (Syntheseschema 2). Die Aminofunktion kann durch Diazotierung entfernt werden. Schließlich wird der Ester zur Zielverbindung verseift:

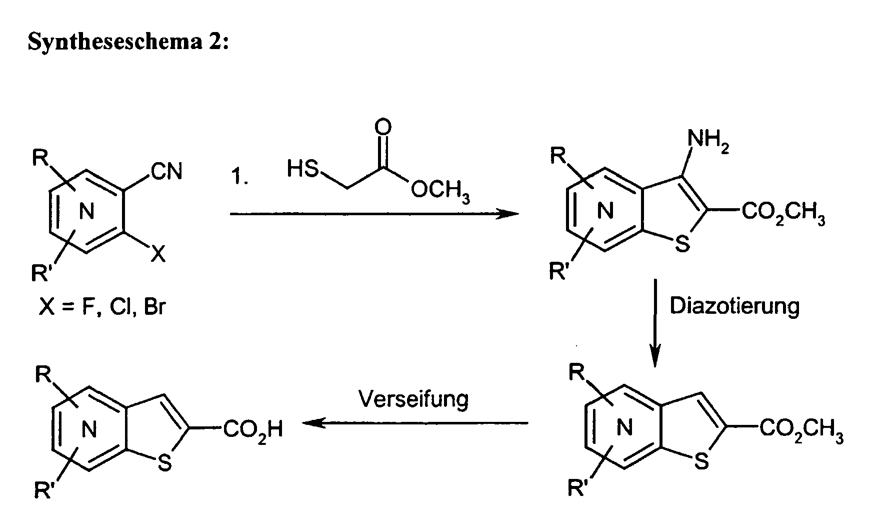

Substituierte Benzofuran-2-carbonsäuren sind z.B. gemäß D. Bogdal, M. Warzala, *Tetrahedron* **2000**, *56,* 8769 zugänglich (Syntheseschema 3):

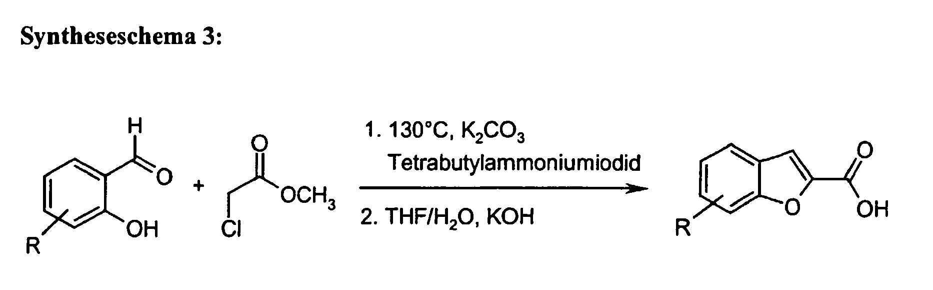

Die erfindungsgemäßen Verbindungen der allgemeinen Formel (I) eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspektrum.

Sie zeichnen sich als Liganden, insbesondere Agonisten am α7-nAChR aus.

Die erfindungsgemäßen Verbindungen können aufgrund ihrer pharmakologischen Eigenschaften allein oder in Kombination mit anderen Arzneimitteln zur Behandlung und/oder Prävention von kognitiven Störungen, insbesondere der Alzheimerschen Krankheit eingesetzt werden. Wegen ihrer selektiven Wirkung als α7-nAChR-Agonisten eignen sich die erfindungsgemäßen Verbindungen besonders zur Verbesserung der Wahrnehmung, Konzentrationsleistung, Lernleistung, oder Gedächtnisleistung insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel-Him-Trauma, allgemeine Konzentrationsstörungen, Konzentrationsstörungen bei Kindern mit Lern- und Gedächtnisproblemen, Attention Deficit Hyperactivity Disorder, Alzheimersche Krankheit, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrophe Lateralsklerose (ALS), Huntingtonsche Krankheit, Multiple Sklerose, Thalamische Degeneration, Creutzfeld-Jacob-Demenz, HIV-Demenz, Schizophrenie, Schizophrenie mit Demenz oder Korsakoff-Psychose.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994), insbesondere zur Behandlung von Krebs-induzierten Schmerzen und chronischen neuropathischen Schmerzen, wie zum Beispiel bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Die *in* vitro-Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhimmembranen

Der [³H]-Methyllyeaconitine-Bindungstest ist eine Modifikation der von Davies et al. (*Neuropharmacol.* **1999,** *38,* 679-690) beschriebenen Methode.

Rattenhirngewebe (Hippocampus oder Gesamthirn) wird in Homogenisierungspuffer (10 % w/v) (0.32 M Sucrose, 1 mM EDTA, 0.1 mM Phenylmethylsulfonylfluorid (PMSF), 0.01 % (w/v) NaN₃, pH 7.4, 4°C) bei 600 rpm in einem Glashomogenisator homogenisiert. Das Homogenat wird zentrifugiert (1000 x g, 4°C, 10 min) und der Überstand wird abgenommen. Das Pellet wird erneut suspendiert (20 % w/v) und zentrifugiert (1000 x g, 4°C, 10 min). Die beiden Überstände werden vereinigt und zentrifugiert (15.000 x g, 4°C, 30 min). Dieses Pellet wird als P2-Fraktion bezeichnet.

Das P2-Pellet wird zweimal mit Bindungspuffer gewaschen (50 mM Tris-HCl, 1 mM MgCl₂, 120 mM NaCl, 5 mM KCl, 2 mM CaCl₂, pH 7.4) und zentrifugiert (15.000 x g, 4°C, 30 min).

Die P2-Membranen werden in Bindungspuffer resuspendiert und in einem Volumen von 250 µl (Membranproteinmenge 0.1 - 0.5 mg) für 2.5 h bei 21°C inkubiert in der Gegenwart von 1-5 nM [³H]-Methyllycaconitine, 0.1 % (w/v) BSA (bovines Serumalbumin) und verschiedenen Konzentrationen der Testsubstanz. Die unspezifische Bindung wird bestimmt durch Inkubation in der Gegenwart von 1 µM α-Bungarotoxin oder 100 µM Nikotin oder 10 µM MLA (Methyllycaconitine).

Die Inkubation wird beendet durch Zugabe von 4 ml PBS (20 mM Na₂HPO₄ 5 mM KH₂PO₄, 150 mM NaCl, pH 7.4, 4°C) und Filtration durch Typ A/E glass fibre filters (Gelman Sciences), die vorher 3 h in 0.3 % (v/v) Polyethyleneimin (PEI) eingelegt waren. Die Filter werden zweimal mit 4 ml PBS (4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50 % des am Rezeptor gebundenen Liganden verdrängt werden), der Dissoziationskonstante K_{D} und der Konzentration L von [³H]Methyllycaconitine wird die Dissoziationskonstante der Testsubstanz Kᵢ bestimmt [Kᵢ = IC₅₀ / (1+L/K_{D})].

Anstelle von [³H]-Methyllycaconitine können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Repräsentative in-vitro-Wirkdaten fiir die erfindungsgemäßen Verbindungen sind in Tabelle A wiedergegeben:

**Tabelle A**

| **Beispiel** | **Kᵢ (nM)** |
|---|---|
| 19 | 42,0 |
| 35 | 3,7 |
| 36 | 63,0 |
| 37 | 80,0 |
| 42 | 37,0 |
| 46 | 58,0 |
| 48 | 75,0 |
| 51 | 3,9 |
| 58 | 3,1 |
| 59 | 20,0 |
| 62 | 50,0 |
| 64 | 20,0 |
| 65 | 55,0 |
| 68 | 2,0 |
| 73 | 2,8 |
| 74 | 22,0 |
| 76 | 6,7 |
| 77 | 20,0 |
| 78 | 80,0 |
| 80 | 31,0 |
| 88 | 28,0 |

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wird wie beschrieben durchgeführt (Blokland et al., *NeuroReport* **1998,** *9,* 4205-4208; Ennaceur, A., Delacour, J., *Behav. Brain Res.* **1988,** *31*, 47-59; Ennaceur, A., Meliani, K., *Psychopharmacology* **1992**, *109*, 321-330; Prickaerts et al., *Eur. J Pharmacol.* **1997,** *337,* 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleich stark inspektieren. Die Gabe einer Substanz mit lern- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskiminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest:

Der Soziale Wiedererkennungstest ist ein Test zur Prüfung der lern- oder gedächtnisverbessernden Wirkung von Testsubstanzen.
Adulte Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die Zeit gemessen, die das adulte Tier das juvenile Tier untersucht (Trial 1). Gemessen werden alle deutlich auf das Jungtier gerichteten Verhaltensweisen, d.h. anogenitale Inspektion, Verfolgen sowie Fellpflege, bei denen das Alttier einen Abstand von höchstens 1 cm zu dem Jungtier hat. Danach wird das juvenile Tier herausgenommen und das adulte in seinem Testkäfig belassen (bei 24 Stunden Retention wird das Tier in seinen Heimkäfig zurückgesetzt). Vor oder nach dem ersten Test wird das adulte Testtier mit Testsubstanz behandelt. Je nach Zeitpunkt der Behandlung kann das Erlernen oder das Speichern der Information über das Jungtier durch die Substanz beeinflusst werden. Nach einem festgelegten Zeitraum (Retention) wird der Test wiederholt (Trial 2). Je größer die Differenz zwischen den in Trials 1 und 2 ermittelten Investigationszeiten, desto besser hat sich das adulte Tier an das Jungtier erinnert.

Die erfindungsgemäßen Verbindungen der allgemeinen Formel (I) eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Verbindungen der Formel (I) bestehen, sowie Verfahren zur Herstellung dieser Zubereitungen.

Die Verbindungen der Formel (I) sollen in diesen Zubereitungen in einer Konzentration von 0.1 bis 99.5 Gew.-%, bevorzugt von 0.5 bis 95 Gew.-% der Gesamtmischung vorhanden sein.

Neben den Verbindungen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0.5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0.001 bis 10 mg/kg, bei oraler Anwendung vorzugsweise etwa 0.005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- BINAP: 2,2'-Bis-(diphenylphosphino)-1,1'-binaphthyl
- DCI: direkte chemische Ionisation (bei MS)
- DMF: *N,N*-Dimethylformamid
- DMSO: Dimethylsulfoxid
- d.Th.: der Theorie (bei Ausbeute)
- EDC: *N'*-(3-Dimethylaminopropyl)-*N*-ethylcarbodiimid x HCl
- EDTA: Ethylendiamintetraessigsäure
- eq.: Äquivalent(e)
- ESI: Elektrospray-Ionisation (bei MS)
- HATU: *O*-(7-Azabenzotriazol-1-yl)-*N,N,N',N'*-tetramethyluronium-Hexafluorphosphat
- HOBt: 1-Hydroxy-1H-benzotriazol x H₂O
- HPLC: Hochdruck-, Hochleistungsflüssigchromatographie
- LC-MS: Flüssigchromatographie-gekoppelte Massenspektroskopie
- MS: Massenspektroskopie
- NMR: Kernresonanzspektroskopie
- Pd₂(dba)₃: Tris-(dibenzylidenaceton)-dipalladium(0)
- RT: Raumtemperatur, 20 °C
- Rₜ: Retentionszeit (bei HPLC)
- TBTU: *O*-(Benzotriazol-1-yl)-*N,N,N',N'*-tetrainethyluronium-Tetrafluoroborat
- TFA: Trifluoressigsäure
- THF: Tetrahydrofuran
- Tris: Tris-(hydroxymethyl)-aminomethan

### LC-MS Methode A:

| | | | | |
|---|---|---|---|---|
| Gerätetyp MS: | Micromass Quattro LCZ | | | |
| | Ionisierung: ESI positiv | | | |
| Gerätetyp HPLC: | HP 1100 | | | |
| | UV-Detektor DAD: 208-400 nm | | | |
| | Ofentemp.: 40°C | | | |
| Säule: | Symmetry C 18 | | | |
| | 50 mm x 2.1 mm; 3.5 µm | | | |
| Gradient: | Zeit (min) | A: % | B: % | Fluss (ml/min) |
| | 0.00 | 10.0 | 90.0 | 0.50 |
| | 4.00 | 90.0 | 10.0 | 0.50 |
| | 6.00 | 90.0 | 10.0 | 0.50 |
| | 6.10 | 10.0 | 90.0 | 1.00 |
| | 7.50 | 10.0 | 90.0 | 0.50 |
| A: | Acetonitril + 0.1% Ameisensäure | | | |
| B: | Wasser + 0.1% Ameisensäure | | | |

### LC-MS Methode B:

| | | | | | |
|---|---|---|---|---|---|
| Gerätetyp MS: | Finnigan MAT 900S | | | | |
| | Ionisierung: ESI positiv | | | | |
| Gerätetyp HPLC: | TSP: P4000, AS3000, UV3000HR | | | | |
| | UV-Detektor 3000HR: 210 nm | | | | |
| | Ofentemp.: 70°C | | | | |
| Säule: | Symmetry C 18 150 mm x 2.1 mm; 5 µm | | | | |
| Lieferfirma: | Waters | | | | |
| Gradient: | Zeit (min) | A: % | B: % | C: % | Fluss (ml/min) |
| | 0 | 2 | 49 | 49 | 0.9 |
| | 2.5 | 95 | 2.5 | 2.5 | 1.2 |
| | 5 | 95 | 2.5 | 2.5 | 1.2 |
| | 5.5 | 2 | 49 | 49 | 1.2 |
| | 6.5 | 2 | 49 | 49 | 1.2 |
| | 7 | 2 | 49 | 49 | 0.9 |
| A: | Acetonitril | | | | |
| B: | Wasser + 0.6 g/l 35%-ige Salzsäure | | | | |
| C: | Wasser | | | | |

### LC-MS Methode C:

| | | | | |
|---|---|---|---|---|
| Gerätetyp MS: | Micromass Platform LCZ | | | |
| | Ionisierung: ESI positiv | | | |
| Gerätetyp HPLC: | HP 1100 | | | |
| | UV-Detektor DAD: 208-400 nm | | | |
| | Ofentemp.: 40°C | | | |
| Säule: | Symmetry C 18 | | | |
| | 50 mm x 2.1 mm; 3.5 µm | | | |
| Gradient: | Zeit (min) | A: % | B: % | Fluss (ml/min) |
| | 0.00 | 10.0 | 90.0 | 0.50 |
| | 4.00 | 90.0 | 10.0 | 0.50 |
| | 6.00 | 90.0 | 10.0 | 0.50 |
| | 6.10 | 10.0 | 90.0 | 1.00 |
| | 7.50 | 10.0 | 90.0 | 0.50 |
| A: | Acetonitril + 0.1% Ameisensäure | | | |
| B: | Wasser + 0.1% Ameisensäure | | | |

### LC-MS Methode D:

Instrument: Micromass Platform LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90% A → 4.0 min 10% A → 6.0 min 10% A; Ofen: 40°C; Fluss: 0.5 ml/min; UV-Detektion: 208-400 nm.

### LC-MS Methode E:

Instrument: Micromass Quattro LCZ, HP1100; Säule: Uptisphere HDO, 50 mm x 2.0 mm, 3 µm; Eluent A: Wasser + 0.05% Ameisensäure, Eluent B: Acetonitril + 0.05% Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### LC-MS Methode F:

Instrument: Micromass Quattro LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90% A → 4.0 min 10% A → 6.0 min 10% A; Ofen: 40°C; Fluss: 0.5 ml/min; UV-Detektion: 208-400 nm.

### LC-MS Methode G:

Gerätetyp MS: Micromass ZQ; Gerätetyp HPLC: Waters Alliance 2790; Säule: Uptisphere C18, 50 mm x 2.0 mm, 3.0 µm; Eluent A: Acetonitril + 0.05% Ameisensäure, Eluent B: Wasser + 0.05% Ameisensäure; Gradient: 0.0 min 5% A → 2.0 min 40% A → 4.5 min 90% A → 5.5 min 90% A; Ofen: 45°C; Fluss: 0.0 min 0.75 ml/min → 4.5 min 0.75 ml/min → 5.5 min 1.25 ml/min; UV-Detektion: 210 nm.

### HPLC-Methode H:

Instrument: HP 1100 mit DAD-Detektion; Säule: Kromasil RP-18, 60 mm x 2 mm, 3.5 µm; Eluent A: 5 ml HClO₄/l H₂O, Eluent B: Acetonitril; Gradient: 0 min 2% B, 0.5 min 2% B, 4.5 min 90% B, 6.5 min 90% B; Fluss: 0.75 ml/min; Temperatur: 30°C; UV-Detektion: 210 nm.

### Ausgangsverbindungen:

Allgemeines Reaktionsschema für die Synthese von 1-Benzothiophen-2-carbonsäuremethylestem:

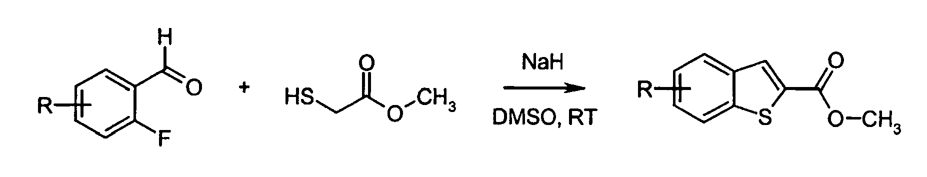

### Allgemeine Arbeitsvorschrift für die Synthese von 1-Benzothiophen-2-carbonsäuremethylestern:

Unter einer Argonatmosphäre werden 1.5 Äquivalente Natriumhydrid (60%-ig) in absolutem DMSO (0.60-1.26 molare Suspension) vorgelegt. Bei Raumtemperatur werden langsam 1.1 Äquivalente Mercaptoessigsäuremethylester zur Reaktionsmischung hinzugetropft und man lässt bis zur Beendigung der Wasserstoffentwicklung (ca. 15 min.) bei Raumtemperatur rühren. 1.0 Äquivalente des entsprechenden Benzaldehydes werden in absolutem DMSO gelöst (1.60-3.36 molare Lösung) und bei Raumtemperatur zur Reaktionsmischung gegeben. Die Reaktions-mischung wird bis zur Beendigung der Reaktion (ca. 5-10 min.) gerührt und anschließend in Eiswasser gegossen. Der entstandene Niederschlag wird abgesaugt, über Nacht im Vakuum bei 40°C getrocknet und als Rohprodukt weiter umgesetzt.

### Allgemeines Reaktionsschema für die Synthese von 1-Benzothiophen-2-carbonsäuren:

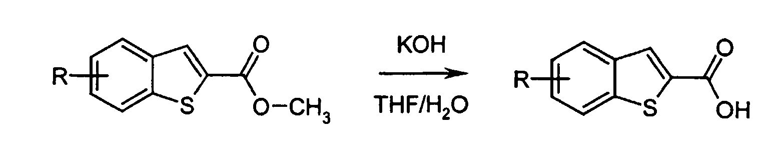

### Allgemeine Arbeitsvorschrift für die Synthese von 1-Benzothiophen-2-carbonsäuren:

Der entsprechende 1-Benzothiophen-2-carbonsäuremethylester wird mit einer Mischung aus gleichen Teilen THF und 2 N Kaliumhydroxid-Lösung versetzt (0.28-0.47 M Lösung). Man lässt die Reaktionsmischung bei Raumtemperatur über Nacht rühren. Im Vakuum wird das THF entfernt und die wässrige Reaktionsmischung mit konzentrierter Salzsäure sauer gestellt. Der entstandene Niederschlag wird abgesaugt und im Vakuum bei 40°C getrocknet.

### Allgemeine Arbeitsvorschrift zur Amid-Knüpfung zwischen 3-Chinuklidinamin und 2-Benzothiophen- bzw. 2-Benzofurancarbonsäuren (Variante A):

1.5 eq. des entsprechenden enantiomeren 3-Chinuklidinamin-Hydrochlorids werden zusammen mit 1 eq. der Carbonsäure und 1.5 eq. HATU bei 0°C in DMF vorgelegt. Nach Zugabe von 1.5 eq. N,N-Diisopropylethylamin wird 30 min. gerührt. Es werden weitere 4 eq. N,N-Diisopropylethylamin zugegeben und bei RT über Nacht nach-gerührt. Die Reinigung erfolgt chromatographisch.

### Allgemeine Arbeitsvorschrift zur Amid-Knüpfung zwischen 3-Chinuklidinamin und 2-Benzothiophen- bzw. 2-Benzofurancarbonsäuren (Variante B):

1.0 eq. des entsprechenden enantiomeren 3-Chinuklidinamin-Dihydrochlorids werden zusammen mit 1 eq. der Carbonsäure und 1.2 eq. HATU bei 0°C in DMF vorgelegt. Nach Zugabe von 1.2 eq. N,N-Diisopropylethylamin wird 30 min. gerührt. Es werden weitere 2.4 eq. N,N-Diisopropylethylamin zugegeben und bei RT über Nacht nachgerührt. Die Reinigung erfolgt chromatographisch.

### Allgemeine Arbeitsvorschrift für die Synthese von 3-Aminothienopyridin-2-carbonsäuremethylestern:

1.0 Äquivalente des entsprechenden Pyridin-Derivats werden in absolutem DMSO (0.93-0.96 M Lösung) gelöst und mit 2 Äquivalenten Triethylamin versetzt. Nach Zugabe von 1 Äquivalent Mercaptoessigsäuremethylester wird die Reaktionsmischung über Nacht bei 60°C gerührt. Die Reaktionsmischung wird in Eiswasser gegossen und darin verrührt. Der ausgefallene Feststoff wird abgesaugt und gegebenenfalls säulenchromatographisch gereinigt (Kieselgel 60, Laufmittel Toluol / Essigsäureethylester 20:1 bis 5:1).

### Allgemeine Arbeitsvorschrift für die Synthese von Thienopyridin-2-carbonsäuremethylestern:

1.0 Äquivalente des entsprechenden 3-Aminothienopyridin-2-carbonsäuremethylesters werden in 75%-iger Schwefelsäure (0.33-0.36 M Lösung) unter Kühlung auf -5°C vorgelegt. Eine Lösung von 3.2 Äquivalenten Natriumnitrit in Wasser (0.92-1.00 M Lösung) wird so langsam zur Reaktionsmischung hinzugetropft, dass die Temperatur der Reaktionsmischung nicht über 0°C steigt. Man lässt 45 min bei 0°C rühren. Danach werden 60-65 Äquivalente eisgekühlter 50%-iger Hypophosphorsäure ebenfalls so zur Reaktionsmischung getropft, dass die Temperatur nicht über 0°C steigt. Man lässt eine Stunde bei -5°C rühren, bevor die Reaktionsmischung über Nacht in den Kühlschrank gestellt wird. Die Reaktionsmischung wird mit Natriumhydrogencarbonat-Lösung und Essigsäureethylester verdünnt und portionsweise bis zur basischen Reaktion mit Natriumhydrogencarbonat versetzt. Die Aufarbeitung wird anhand der nachfolgenden Beispiele beschrieben.

### Allgemeine Arbeitsvorschrift für die Synthese von Thienopyridin-2-carbonsäuren:

Der entsprechende Thienopyridin-2-carbonsäuremethylester wird mit einer Mischung aus gleichen Teilen THF und 2 N Kaliumhydroxid-Lösung versetzt (0.22 M Lösung). Man lässt die Reaktionsmischung bei Raumtemperatur über Nacht rühren. Die Reaktionsmischung wird mit Wasser verdünnt, zweimal mit Essigsäureethylester gewaschen und mit konzentrierter Salzsäure sauer gestellt. Die Aufarbeitung wird anhand der nachfolgenden Beispiele beschrieben.

### Beispiel 1A

### 6-Fluor-1-benzothiophen-2-carbonsäuremethylester

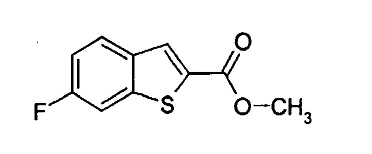

Ausgehend von 2.00 g (14.07 mmol) 2,4-Difluorbenzaldehyd werden mit 0.84 g (21.11 mmol) Natriumhydrid (60%-ig) und 1.64 g (15.48 mmol) Mercaptoessigsäuremethylester 1.99 g des gewünschten Produktes erhalten. Es wird in einer Reinheit erhalten, die eine weitere Umsetzung ermöglicht, und wird ohne weitere Aufreinigung umgesetzt.
MS (EIpos): *m*/*z* = 210 (M)⁺.

### Beispiel 2A

### 5-Brom-1-benzothiophen-2-carbonsäuremethylester

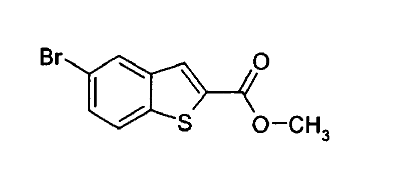

Ausgehend von 1.62 g (8.00 mmol) 5-Brom-2-fluorbenzaldehyd werden mit 0.48 g (12.00 mmol) Natriumhydrid (60%-ig) und 0.93 g (8.80 mmol) Mercaptoessigsäuremethylester 1.53 g (71% d.Th.) des gewünschten Produktes erhalten.
LC-MS (Methode A): Rₜ = 4.80 min.
MS (EIpos): *m*/*z* = 272 (M)⁺
¹H-NMR (200 MHz, CDCl₃): δ = 8.07-7.92 (m, 2H), 7.78-7.66 (m, 1H), 7.59-7.48 (m, 1H), 3.95 (s, 3H).

### Beispiel 3A

### 5-Methyl-1-benzothiophen-2-earbonsäuremethylester

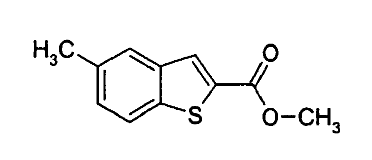

Ausgehend von 2.32 g (16.78 mmol) 2-Fluor-5-methylbenzaldehyd werden mit 1.01 g (25.17 mmol) Natriumhydrid (60%-ig) und 1.96 g (18.46 mmol) Mercaptoessigsäuremethylester 1.96 g (57% d.Th.) des gewünschten Produktes erhalten.
LC-MS (Methode C): Rₜ = 4.68 min.
MS (EIpos): *m*/*z* = 206 (M)⁺
¹H-NMR (200 MHz, CDCl₃): δ = 7.99 (s, 1H), 7.79-7.64 (m, 2H), 7.34-7.23 (m, 1H), 3.94 (s, 3H).

### Beispiel 4A

### 6-Fluor-1-benzothiophen-2-carbonsäure

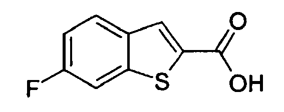

Ausgehend von 1.99 g (9.46 mmol) 6-Fluor-1-benzothiophen-2-carbonsäuremethylester werden 1.43 g des gewünschten Produktes erhalten. Es wird in einer Reinheit erhalten, die eine weitere Umsetzung ermöglicht, und wird ohne weitere Aufreinigung umgesetzt.
MS (EIpos): *m*/*z* = 196 (M)⁺.

### Beispiel 5A

### 5-Brom-1-benzothiophen-2-carbonsäure

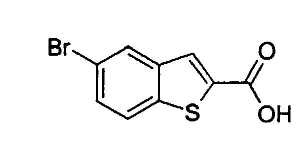

Ausgehend von 1.53 g (5.64 mmol) 5-Brom-1-benzothiophen-2-carbonsäure-methylester werden 1.31 g (90% d.Th.) des gewünschten Produktes erhalten.
HPLC (Methode H): Rₜ = 4.50 min.
MS (ESIneg): *m*/*z* = 255 (M-H)⁻.
¹H-NMR (200 MHz, CDCl₃): δ = 8.01 (d, 1H), 7.94 (s, 1H), 7.74 (d, 1H), 7.53 (dd, 1H).

### Beispiel 6A

### 5-Methyl-1-benzothiophen-2-carbonsäure

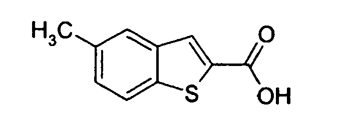

Ausgehend von 1.96 g (9.49 mmol) 5-Methyl-1-benzothiophen-2-carbonsäuremethylester werden 1.46 g (80% d.Th.) des gewünschten Produktes erhalten.
HPLC (Methode H): Rₜ = 4.38 min.
MS (DCI): *m*/*z* = 210 (M+NH₄)⁺
¹H-NMR (200 MHz, CDCl₃): δ = 8.09 (s, 1H), 7.65-7.83 (m, 2H), 7.39-7.29 (m, 1H), 2.49 (s, 3H).

### Beispiel 7A

### 3-Aminothieno[2,3-b]pyridin-2-carbonsäuremethylester

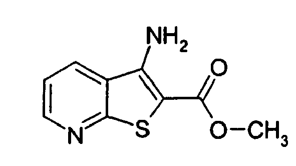

Ausgehend von 0.92 g (6.51 mmol) 2-Chlornicotinsäurenitril werden mit 1.37 g (13.53 mmol) Triethylamin und 0.72 g (6.77 mmol) Mercaptoessigsäuremethylester 0.22 g (16% d.Th.) des gewünschten Produktes erhalten.
HPLC (Methode H): Rₜ = 3.56 min.
MS (ESIpos): m/z = 209 (M+H)⁺
¹H-NMR (300 MHz, CDCl₃): δ = 8.76-8.62 (m, 1H), 7.99-7.87 (m, 1H), 7.37-7.28 (m, 1H), 5.91 (br. s, 2H), 3.91 (s, 3H).

### Beispiel 8A

### 3-Aminothieno[3,2-b]pyridin-2-carbonsäuremethylester

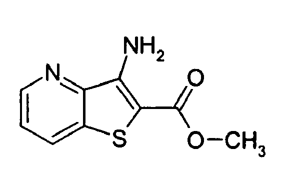

Ausgehend von 2.00 g (14.43 mmol) 2-Chlor-2-pyridincarbonitril werden mit 3.03 g (30.02 mmol) Triethylamin und 1.59 g (15.01 mmol) Mercaptoessigsäuremethylester 2.47 g (81% d.Th.) des gewünschten Produktes erhalten.
LC-MS (Methode A): Rₜ = 3.4 min.
MS (ESIpos): m/z = 209 (M+H)⁺
¹H-NMR (300 MHz, CDCl₃): δ = 8.67-8.58 (m, 1H), 8.10-8.01 (m, 1H), 7.42-7.33 (m, 1H), 6.22 (br. s, 2H), 3.92 (s, 3H).

### Beispiel 9A

### Thieno[2,3-b]pyridin-2-carbonsäuremethylester

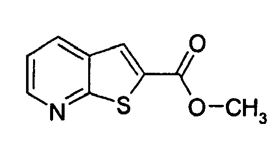

Die Synthese erfolgt nach der allgemeinen Arbeitsvorschrift; zur Aufarbeitung wird der entstandene Niederschlag abgesaugt und zweimal mit Wasser und dreimal mit Essigsäureethylester ausgewaschen. Die organische Phase des erhaltenen Filtrats wird abgetrennt, getrocknet und eingeengt. Ausgehend von 0.22 g (1.07 mmol) 3-Aminothieno[2,3-b]pyridin-2-carbonsäuremethylester werden mit 0.24 g (3.41 mmol) Natriumnitrit 84 mg (41% d.Th.) des gewünschten Produktes erhalten.
LC-MS (Methode A): Rₜ = 3.34 min.
MS (ESIpos): m/z = 194 (M+H)⁺
¹H-NMR (200 MHz, CDCl₃): δ = 8.82-8.66 (m, 1H), 8.53-8.38 (m, 1H), 8.23 (s, 1H), 7.63-7.48 (m, 1H), 3.92 (s, 3H).

### Beispiel 10A

### Thieno[3,2-b]pyridin-2-carbonsäuremethylester

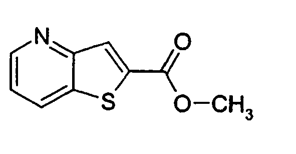

Die Synthese erfolgt nach der allgemeinen Arbeitsvorschrift; zur Aufarbeitung wird der entstandene Niederschlag abgesaugt und mehrmals mit Wasser, Essigsäureethylester und THF ausgewaschen. Die organischen Phasen der erhaltenen Filtrate werden abgetrennt, die wässrige Phase mit Essigsäureethylester gewaschen und die vereinigten organischen Phasen werden getrocknet und eingeengt. Das erhaltene Produkt wird mittels präparativer HPLC gereinigt. Ausgehend von 2.40 g (11.53 mmol) 3-Aminothieno[3,2-b]pyridin-2-carbonsäuremethylester werden mit 2.54 g (36.88 mmol) Natriumnitrit 0.12 g (5% d.Th.) des gewünschten Produktes erhalten.
LC-MS (Methode C): Rₜ = 3.18 min.
MS (ESIpos): m/z = 194 (M+H)⁺
¹H-NMR (400 MHz, CDCl₃): δ = 8.83-8.76 (m, 1H), 8.63-8.56 (m, 1H), 8.21 (s, 1H), 7.58-7.50 (m, 1H), 3.93 (s, 3H).

### Beispiel 11A

### Thieno[2,3-b]pyridin-2-carbonsäure

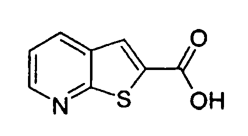

Die Synthese erfolgt nach der allgemeinen Arbeitsvorschrift; zur Aufarbeitung wird die wässrige Phase zweimal mit Essigsäureethylester ausgeschüttelt, die vereinigten organischen Phasen werden mit gesättigter Natriumchlorid-Lösung gewaschen, getrocknet und das Lösungsmittel im Vakuum entfernt. Ausgehend von 84 mg (0.43 mmol) Thieno[2,3-b]pyridin-2-carbonsäuremethylester werden 49 mg (63% d.Th.) des gewünschten Produktes erhalten.
LC-MS (Methode C): Rₜ = 2.46 min.
MS (ESIpos): m/z = 180 (M+H)⁺
¹H-NMR (200 MHz, CDCl₃): δ = 13.76 (br. s, 1H), 8.80-8.63 (m, 1H), 8.50-8.34 (m, 1H), 8.11 (s, 1H), 7.61-7.47 (m, 1H).

### Beispiel 12A

### Thieno[3,2-b]pyridin-2-carbonsäure

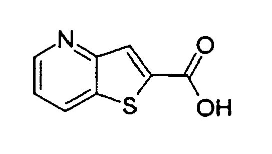

Die Synthese erfolgt nach der allgemeinen Arbeitsvorschrift; zur Aufarbeitung wird der ausgefallene Feststoff abgesaugt, mit Wasser und Acetonitril gewaschen und im Vakuum getrocknet. Ausgehend von 115 mg (0.60 mmol) Thieno[3,2-b]pyridin-2-carbonsäuremethylester werden 77 mg (72% d.Th.) des gewünschten Produktes erhalten.
HPLC (Methode H): Rₜ = 2.08 min.
MS (ESIpos): m/z = 180 (M+H)⁺
¹H-NMR (400 MHz, D₂O): δ = 8.63 (d, 1H), 8.39 (d, 1H), 7.85 (s, 1H), 7.45 (dd, 1H).

### Beispiel 13A

### 7-Brom-1-benzothiophen-2-carbonsäuremethylester

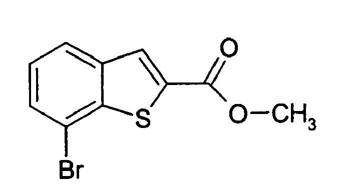

Ausgehend von 27.8 g (137.1 mmol) 3-Brom-2-fluorbenzaldehyd werden mit 8.2 g (205.7 mmol) Natriumhydrid (60%-ig) und 16.0 g (150.9 mmol) Mercaptoessigsäuremethylester 20.57 g eines Gemisches aus der Titelverbindung und der korrespondierenden Säure (ca. 1:1) erhalten.

### Beispiel 14A

### Methyl thieno[2,3-f][1,3]benzodioxol-6-carboxylat

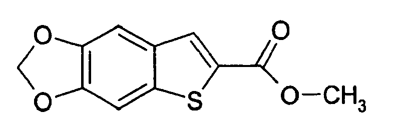

Ausgehend von 3.0 g (13.1 mmol) 6-Brompiperonal werden mit 0.79 g (19.7 mmol) Natriumhydrid (60%-ig) und 1.53 g (14.4 mmol) Mercaptoessigsäuremethylester 732 mg (18.5% d.Th.) der Titelverbindung erhalten.
¹H-NMR (200 MHz, DMSO-d₆): δ = 8.03 (s, 1H), 7.62 (s, 1H), 7.48 (s, 1H), 6.14 (s, 2H), 3.86 (s, 3H) ppm.
HPLC: Rₜ = 4.6 min (Methode H)
MS (ESIpos): *m*/*z* = 237 (M+H)⁺

### Beispiel 15A

### 6-Cyano-1-benzothiophen-2-carbonsäuremethylester

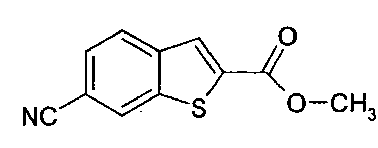

4.08 g (23.2 mmol) 4-Cyano-2-nitrobenzaldehyd, 2.46 g (23.2 mmol) Mercaptoessigsäuremethylester und 6.46 ml (46.4 mmol) Triethylamin werden in 12.3 ml DMSO für 2.5 h auf 80°C erhitzt. Die Reaktionslösung wird auf 400 ml Eiswasser gegeben. Nach der Zugabe von 4 ml Essigsäure wird der entstandene Niederschlag abgesaugt, zweimal mit Wasser gewaschen und über Nacht bei 50°C im Vakuum getrocknet. Man erhält 4.19 g (83.2% d.Th.) der Titelverbindung.
¹H-NMR (200 MHz, DMSO-d₆): δ = 8.73 (d, 1H), 8.32 (s, 1H), 8.21 (d, 1H), 7.85 (dd, 1H), 3.92 (s, 3H) ppm.
HPLC: Rₜ = 4.4 min (Methode H)
MS (ESIpos): *m*/*z* = 218 (M+H)⁺

### Beispiel 16A

### 7-Fluor-1-benzothiophen-2-carbonsäuremethylester

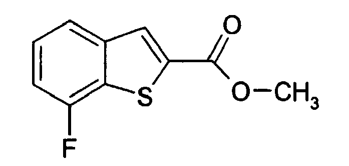

Ausgehend von 5.0 g (35.2 mmol) 2,3-Difluorbenzaldehyd werden mit 2.11 g (52.8 mmol) Natriumhydrid (60%-ig) und 4.11 g (38.7 mmol) Mercaptoessigsäuremethylester 3.30 g (44.6% d.Th.) der Titelverbindung erhalten.
¹H-NMR (400 MHz, DMSO-d₆): δ = 8.33 (d, 1H), 7.92 (d, 1H), 7.55 (m, 1H), 7.46 (dd, 1H), 3.93 (s, 3H) ppm.
HPLC: Rₜ = 4.7 min (Methode H)
MS (ESIpos): *m*/*z* = 228 (M+NH₄)⁺

### Beispiel 17A

### 7-Methoxy-1-benzothiophen-2-carbonsäuremethylester

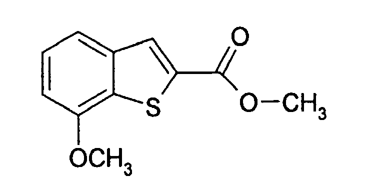

Zu einer Lösung von 600 mg (3.31 mmol) 3-Methoxy-2-nitrobenzaldehyd in 8 ml DMF werden 550 mg (3.97 mmol) Kaliumcarbonat und 350 mg (3.31 mmol) Mercaptoessigsäuremethylester zugefügt. Die Reaktionsmischung wird für 4 h auf 70°C erwärmt. Nach Abkühlen wird Wasser hinzugefügt. Der entstandene Niederschlag wird abfiltriert, mit Wasser gewaschen und im Vakuum getrocknet. Man erhält 387 mg (45.7% d.Th.) der Titelverbindung.
¹H-NMR (200 MHz, DMSO-d₆): δ = 8.21 (s, 1H), 7.63 (d, 1H), 7.46 (dd, 1H), 7.11 (d, 1H), 3.98 (s, 3H), 3.89 (s, 3H) ppm.
MS (ESIpos): *m*/*z* = 240 (M+NH₄)⁺

### Beispiel 18A

### 6-Nitro-1-benzothiophen-2-carbonsäuremethylester

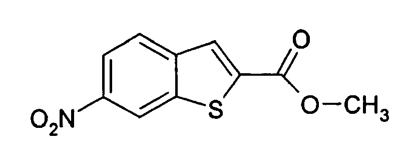

24.8 g (126.7 mmol) 2,4-Dinitrobenzaldehyd, 13.4 g (126.7 mmol) Mercaptoessigsäuremethylester und 35.3 ml (253.3 mmol) Triethylamin werden in 74.5 ml DMSO für 1 h auf 80°C erhitzt. Die Reaktionslösung wird auf 250 ml Eiswasser gegeben. Der entstandene Niederschlag wird abgesaugt, mit Wasser gewaschen und über Nacht bei 50°C im Vakuum getrocknet. Man erhält 16.1 g (53.6% d.Th.) der Titelverbindung.
¹H-NMR (300 MHz, CDCl₃): δ = 8.80 (d, 1H), 8.26 (dd, 1H), 8.12 (s, 1H), 8.00 (d, 1H), 3.99 (s, 3H) ppm.
HPLC: Rₜ = 4.7 min (Methode H)
MS (ESIpos): *m*/*z* = 255 (M+NH₄)⁺

### Beispiel 19A

### 4-Nitro-1-benzothiophen-2-carbonsäuremethylester

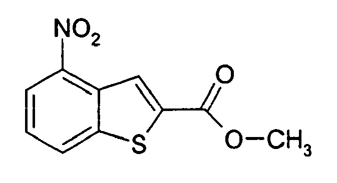

1.0 g (5.10 mmol) 2,6-Dinitrobenzaldehyd, 0.54 g (5.10 mmol) Mercaptoessigsäuremethylester und 1.42 ml (10.20 mmol) Triethylamin werden in 3 ml DMSO für 3.5 h auf 80°C erhitzt. Die Reaktionslösung wird auf 100 ml Eiswasser gegeben. Der entstandene Niederschlag wird abgesaugt, mit Wasser gewaschen und über Nacht bei 50°C im Vakuum getrocknet. Man erhält 1.10 g (88.7% d.Th.) der Titelverbindung.
¹H-NMR (200 MHz, DMSO-d₆): δ = 8.63 (s, 1H), 8.61 (d, 1H), 8.45 (d, 1H), 7.80 (dd, 1H), 3.97 (s, 3H) ppm.
HPLC: Rₜ = 4.6 min (Methode H)
MS (ESIpos): *m*/*z* = 238 (M+NH₄)⁺

### Beispiel 20A

### 6-Brom-1-benzothiophen-2-carbonsäuremethylester

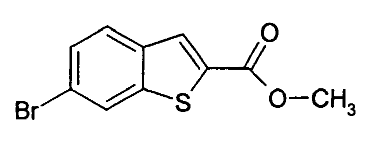

Ausgehend von 6.54 g (32.2 mmol) 4-Brom-2-fluorbenzaldehyd werden mit 1.93 g (48.3 mmol) Natriumhydrid (60%-ig) und 3.76 g (35.5 mmol) Mercaptoessigsäuremethylester 4.06 g (46.4% d.Th.) der Titelverbindung erhalten.
¹H-NMR (200 MHz, DMSO-d₆): δ = 8.42 (d, 1H), 8.22 (s, 1H), 7.98 (d, 1H), 7.65 (dd, 1H), 3.90 (s, 3H) ppm.
HPLC: Rₜ = 5.3 min (Methode H)
MS (ESIpos): *m*/*z* = 270 (M⁺), 288 (M+NH₄)⁺

### Beispiel 21A

### 7-Trifluormethyl-1-benzothiophen-2-carbonsäuremethylester

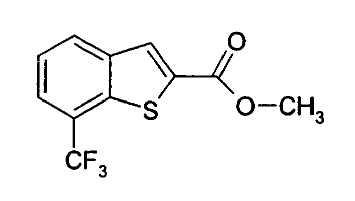

Ausgehend von 5.0 g (26.0 mmol) 2-Fluor-3-trifluormethylbenzaldehyd werden mit 1.56 g (39.0 mmol) Natriumhydrid (60%-ig) und 3.0 g (28.6 mmol) Mercaptoessigsäuremethylester 5.4 g (79.7% d.Th.) der Titelverbindung erhalten.
¹H-NMR (200 MHz, DMSO-d₆): δ = 8.40 (s, 1H), 8.38 (m, 1H), 8.00 (d, 1H), 7.73 (dd, 1H), 3.92 (s, 3H) ppm.
HPLC: Rₜ = 5.1 min (Methode H)
MS (ESIpos): *m*/*z* = 260 (M⁺)

### Beispiel 22A

### 5-Trifluormethyl-1-benzothiophen-2-carbonsäuremethylester

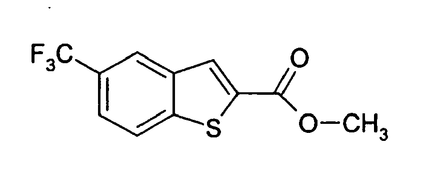

Ausgehend von 4.28 g (22.3 mmol) 2-Fluor-5-trifluormethylbenzaldehyd werden mit 1.34 g (33.4 mmol) Natriumhydrid (60%-ig) und 2.6 g (24.5 mmol) Mercaptoessigsäuremethylester 4.93 g (80.0% d.Th.) der Titelverbindung erhalten.
¹H-NMR (200 MHz, DMSO-d₆): δ = 8.48 (s, 1H), 8.37 (s, 1H), 8.33 (d, 1H), 7.82 (m, 1H), 3.93 (s, 3H) ppm.
MS (ESIpos): *m*/*z* = 260 (M⁺)

### Beispiel 23A

### 7-Chlor-1-benzothiophen-2-carbonsäuremethylester

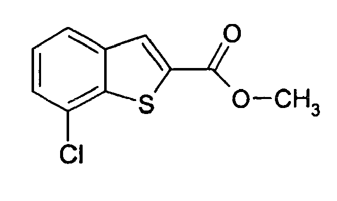

Ausgehend von 1.0 g (6.31 mmol) 2-Fluor-3-chlorbenzaldehyd werden mit 0.38 g (9.46 mmol) Natriumhydrid (60%-ig) und 0.74 g (6.94 mmol) Mercaptoessigsäuremethylester 1.04 g (72.3% d.Th.) der Titelverbindung erhalten.
¹H-NMR (200 MHz, DMSO-d₆): δ = 8.34 (s, 1H), 8.06 (dd, 1H), 7.70 (dd, 1H), 7.56 (dd, 1H), 3.92 (s, 3H) ppm.
HPLC: Rₜ = 4.9 min (Methode H)
MS (ESIpos): *m*/*z* = 227 (M+H)⁺

### Beispiel 24A

### 5-Fluor-1-benzothiophen-2-carbonsäuremethylester

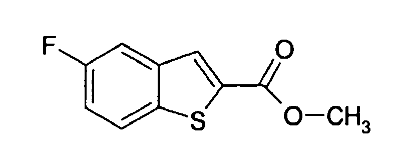

Ausgehend von 5.0 g (35.2 mmol) 2,5-Difluorbenzaldehyd werden mit 2.11 g (52.78 mmol) Natriumhydrid (60%-ig) und 4.11 g (38.7 mmol) Mercaptoessigsäuremethylester 1.1 g (14.3% d.Th.) der Titelverbindung erhalten.
¹H-NMR (200 MHz, CDCl₃): δ = 8.02 (s, 1H), 7.82 (dd, 1H), 7.53 (dd, 1H), 7.23 (ddd, 1H), 3.96 (s, 3H) ppm.
HPLC: Rₜ = 4.8 min (Methode H)
MS (ESIpos): *m*/*z* = 210 (M⁺)

### Beispiel 25A

### 4-Fluor-1-benzothiophen-2-carbonsäuremethylester

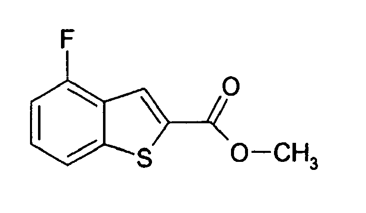

Ausgehend von 5.0 g (35.2 mmol) 2,6-Difluorbenzaldehyd werden mit 2.11 g (52.8 mmol) Natriumhydrid (60%-ig) und 4.11 g (38.7 mmol) Mercaptoessigsäuremethylester 5.61 g (72.8% d.Th.) der Titelverbindung erhalten.
¹H-NMR (200 MHz, DMSO-d₆): δ = 8.16 (s, 1H), 7.95 (d, 1H), 7.59 (ddd, 1H), 7.32 (dd, 1H), 3.91 (s, 3H) ppm.
MS (ESIpos): *m*/*z* = 210 (M⁺)

### Beispiel 26A

### 5,7-Difluor-1-benzothiophen-2-carbonsäuremethylester

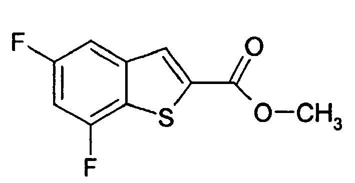

Ausgehend von 3.94 g (24.6 mmol) 2,3,5-Trifluorbenzaldehyd werden mit 1.48 g (36.9 mmol) Natriumhydrid (60%-ig) und 2.88 g (27.1 mmol) Mercaptoessigsäuremethylester 3.58 g (56% d.Th.) der Titelverbindung in einer Reinheit von 89% erhalten. Umkristallisation aus Methanol ergibt das Produkt in einer Reinheit von 97%.
¹H-NMR (200 MHz, DMSO-d₆): δ = 8.28 (d, 1H), 7.81 (dd, 1H), 7.61 (m, 1H), 3.93 (s, 3H) ppm.
MS (ESIpos): *m*/*z* = 228 (M⁺)

### Beispiel 27A

### 6-Methoxy-1-benzothiophen-2-carbonsäuremethylester

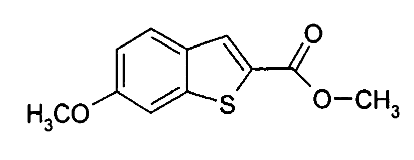

Ausgehend von 2.5 g (16.2 mmol) 2-Fluor-4-methoxybenzaldehyd werden mit 0.97 g (24.3 mmol) Natriumhydrid (60%-ig) und 1.89 g (17.8 mmol) Mercaptoessigsäuremethylester 3.05 g (84.7% d.Th.) der Titelverbindung erhalten.
¹H-NMR (200 MHz, DMSO-d₆): δ = 8.12 (s, 1H), 7.91 (d, 1H), 7.64 (d, 1H), 7.09 (dd, 1H), 3.88 (s, 3H), 3.87 (s, 3H) ppm.
HPLC: Rₜ = 4.7 min (Methode H)
MS (ESIpos): *m*/*z* = 223 (M+H)⁺, 240 (M+NH₄)⁺

### Beispiel 28A

### 6-Cyano-1-benzothiophen-2-carbonsäuremethylester

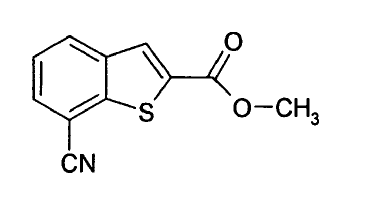

Ausgehend von 2.5 g (15.1 mmol) 2-Chlor-3-cyanobenzaldehyd wird entsprechend der allgemeinen Versuchsvorschrift mit 0.90 g (22.7 mmol) Natriumhydrid (60%-ig) und 1.76 g (16.6 mmol) Mercaptoessigsäuremethylester umgesetzt. Das erhaltene Reaktionsprodukt wird zunächst an Kieselgel säulenchromatographisch gereinigt (Laufmittel: Dichlormethan/Methanol 100:1), dann in 30 ml Pyridin gelöst und mit 650 µl (8.4 mmol) Methansulfonsäurechlorid versetzt. Es wird 2 h bei 80°C gerührt. Nach dem Abkühlen wird mit Essigsäureethylester versetzt, zweimal mit 1 N Salzsäure und einmal mit gesättigter Kochsalz-Lösung gewaschen, über Natriumsulfat getrocknet, eingeengt und im Hochvakuum getrocknet. Man erhält 1.63 g (49.9% d.Th.) der Titelverbindung.
¹H-NMR (400 MHz, DMSO-d₆): δ = 8.40 (d, 1H), 8.39 (s, 1H), 8.16 (d, 1H), 7.70 (dd, 1H), 3.94 (s, 3H) ppm.
HPLC: Rₜ = 4.3 min (Methode H)
MS (ESIpos): *m*/*z* = 218 (M+H⁺)

### Beispiel 29A

### (2-Chlor-3-nitrophenyl)methanol

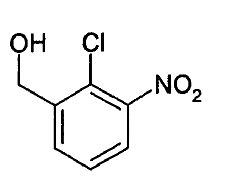

10.0 g (49.61 mmol) 2-Chlor-3-nitrobenzoesäure werden in 50 ml THF vorgelegt. Unter Eiskühlung versetzt man mit 104 ml 1 M Boran-THF-Komplex und läßt über Nacht bei RT nachrühren. Bei 0°C wird vorsichtig mit Wasser hydrolysiert. Nach beendeter Gasentwicklung wird mit 500 ml Wasser verdünnt und die wässrige Phase dreimal mit insgesamt 500 ml Essigsäureethylester extrahiert. Die organische Phase wird mit gesättigter Kochsalz-Lösung gewaschen und über Magnesiumsulfat getrocknet. Schließlich wird das Solvens unter reduziertem Druck am Rotationsverdampfer entfernt. Es werden 9.20 g (98% d.Th.) der Titelverbindung erhalten.
¹H-NMR (300 MHz, DMSO-d₆): δ = 7.89 (m, 2H), 7.62 (t, 1H), 5.70 (t, 1H), 4.67 (d, 2H).
HPLC: Rₜ = 3.53 min (Methode H)
MS (ESIpos): *m*/*z* = 205 (M+NH₄)⁺

### Beispiel 30A

### 2-Chlor-3-nitrobenzaldehyd

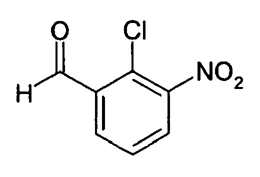

9.2 g (49.05 mmol) (2-Chlor-3-nitrophenyl)methanol und 13.2 g (151.8 mmol) aktiviertes Mangan(IV)oxid werden in 50 ml Chloroform für 20 h unter Rückfluß erhitzt. Es wird über Kieselgur filtriert, eingeengt und im Hochvakuum getrocknet. Die erhaltene Titelverbindung wird direkt weiter umgesetzt.
¹H-NMR (200 MHz, DMSO-d₆): δ = 10.34 (s, 1H), 8.32 (dd, 1H), 8.14 (dd, 1H), 7.79 (t, 1H) ppm.
HPLC: Rₜ = 3.76 min (Methode H)
MS (ESIpos): *m*/*z* = 185 (M)⁺

### Beispiel 31A

### 7-Nitro-1-benzothiophen-2-carbonsäuremethylester

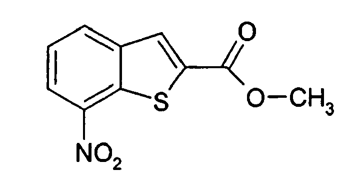

Ausgehend von 3.04 g (16.4 mmol) 2-Chlor-3-nitrobenzaldehyd werden mit 0.98 g (24.6 mmol) Natriumhydrid (60%-ig) und 2.09 g (19.7 mmol) Mercaptoessigsäuremethylester 3.68 g (85% d.Th.) der Titelverbindung erhalten.
¹H-NMR (300 MHz, DMSO-d₆): δ = 8.60 (dd, 1H), 8.53 (dd, 1H), 8.44 (s, 1H), 7.80 (dd, 1H), 3.95 (s, 3H) ppm.
HPLC: Rₜ = 4.6 min (Methode H)
MS (ESIpos): *m*/*z* = 255 (M+NH₄)⁺

### Beispiel 32A

### 3-Brom-2-hydroxybenzaldehyd

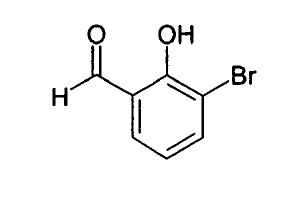

20 g (115.6 mmol) 4-Brom-2-hydroxybenzaldehyd werden zusammen mit 16.84 g (176.9 mmol) Magnesiumchlorid in 500 ml wasserfreiem Acetonitril vorgelegt. Es werden 41.9 ml (300.6 mmol) Triethylamin zugegeben und 3 h unter Rückfluß erhitzt. Es wird auf 0°C gekühlt und mit 300 ml 2 N Salzsäure versetzt. Die wässrige Phase wird dreimal mit insgesamt 600 ml Diethylether extrahiert. Die organische Phase wird mit 200 ml gesättigter Kochsalz-Lösung gewaschen und anschließend über Magnesiumsulfat getrocknet. Das Solvens wird unter reduziertem Druck entfernt. Schließlich werden letzte Lösungsmittelreste im Hochvakuum entfernt. Es werden 24 g (64% d.Th.) der Titelverbindung isoliert und ohne weitere Reinigung weiter umgesetzt.
HPLC: Rₜ = 4.25 min (Methode H)
MS (ESIpos): *m*/*z* = 201 (M)⁺

### Beispiel 33A

### 7-Brom-1-benzofuran-2-carbonsäure

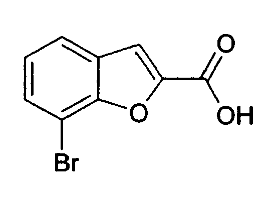

10 g (49.75 mmol) 3-Brom-2-hydroxybenzaldehyd und 1.84 g (4.97 mmol) Tetra-N-butylammoniumiodid werden zusammen mit 27.5 g (199.0 mmol) wasserfreiem Kaliumcarbonat vorgelegt. Es werden 24.3 g (223.86 mmol) Chloressigsäuremethylester zugegeben. Das Reaktionsgemisch wird 4 h auf 130°C erhitzt und anschließend mittels eines Eisbades auf 0°C abgekühlt. Es werden 100 ml THF und 16.75 g (298.5 mmol) Kaliumhydroxid in 100 ml Wasser zugegeben und anschließend 4 h bei 40°C gerührt. Das Solvens wird unter reduziertem Druck entfernt. Es wird mit Wasser verdünnt und mit konz. Salzsäure sauer gestellt. Das Produkt wird über Kieselgel 60 (Laufmittel: Toluol/Essigsäureethylester/Essigsäure 40:10:1) gereinigt. Das Solvens wird unter reduziertem Druck entfernt. Schließlich werden letzte Lösungsmittelreste im Hochvakuum entfernt. Eine Feinreinigung erfolgt durch präparative HPLC. Es werden 303 mg (2.5% d.Th.) der Titelverbindung isoliert.
HPLC: Rₜ = 4.16 min (Methode H)
MS (ESIpos): *m*/*z* = 258 (M+NH₄)⁺

### Beispiel 34A

### 6-Brom-1-benzofuran-2-carbonsäure

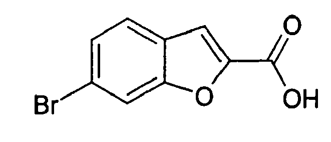

8.0 g (39.8 mmol) 4-Brom-2-hydroxybenzaldehyd und 1.47 g (3.98 mmol) Tetra-N-butylammoniumiodid werden zusammen mit 22 g (159.19 mmol) wasserfreiem Kaliumcarbonat vorgelegt. Es werden 9.07 g (83.57 mmol) Chloressigsäuremethylester zugegeben. Das Reaktionsgemisch wird 4 h auf 130°C erhitzt und anschließend mittels eines Eisbades auf 0°C abgekühlt. Es werden 100 ml THF und 13.4 g (238.8 mmol) Kaliumhydroxid in 50 ml Wasser zugegeben und anschließend über Nacht bei RT gerührt. Das Solvens wird unter reduziertem Druck entfernt. Es wird mit Wasser verdünnt und mit konz. Salzsäure sauer gestellt. Das Produkt wird abfiltriert und im Hochvakuum getrocknet. Zur Feinreinigung wird über Kieselgel 60 (Laufmittel: Toluol → Toluol/Essigsäure 50:1 → Toluol/Essigsäureethylester/Essigsäure 35:5:1) gereinigt. Das Solvens wird unter reduziertem Druck entfernt. Es werden 3.8 g (40% d.Th.) der Titelverbindung isoliert.
¹H-NMR (400 MHz, Methanol-d₄): δ = 7.91 (m, 1H), 7.61-7.51 (m, 3H) ppm.
HPLC: Rₜ = 4.1 min (Methode H)
MS (ESIpos): *m*/*z* = 258 (M+NH₄)⁺

### Beispiel 35A

### 5,7-Difluor-1-benzofuran-2-carbonsäure

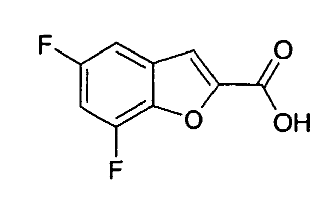

### Stufe a):

1.0 g (7.69 mmol) 2,4-Difluorophenol wird in 6 ml TFA vorgelegt. 2.16 g (15.37 mmol) Hexamethylentetramin werden portionsweise innerhalb von 30 min. zugegeben. Anschließend wird 20 h unter Rückfluß erhitzt. Bei RT wird mit 9 ml Wasser und 4.5 ml 50%-iger Schwefelsäure versetzt. Nach 2 h bei RT wird das Reaktionsgemisch zweimal mit je 30 ml Essigsäureethylester extrahiert. Die vereinigten organischen Phasen werden viermal mit 1 N Salzsäure und einmal mit Wasser gewaschen und anschließend über Magnesiumsulfat getrocknet. Das Rohprodukt wird ohne weitere Aufreinigung weiter umgesetzt.
LC-MS (Methode D): Rₜ = 3.5 min.
MS (ESIneg): *m*/*z* = 157 (M-H)⁻

### Stufe b):

Das Rohprodukt aus der vorigen Umsetzung und 0.28 g (0.77 mmol) Tetra-N-butylammoniumiodid werden zusammen mit 4.25 g (30.76 mmol) wasserfreiem Kaliumcarbonat vorgelegt. Es werden 1.75 g (16.15 mmol) Chloressigsäuremethylester zugegeben. Das Reaktionsgemisch wird 4 h auf 130°C erhitzt und anschließend mittels eines Eisbades auf 0°C abgekühlt. Es werden 27 ml THF, 2.59 g (46.14 mmol) Kaliumhydroxid und 27 ml Wasser zugegeben. Es wird über Nacht bei RT gerührt. Das Solvens wird unter reduziertem Druck entfernt. Es wird mit Wasser verdünnt und mit konz. Salzsäure sauer gestellt. Es wird zweimal mit Essigsäureethylester extrahiert. Die vereinigten organischen Phasen werden über Magnesiumsulfat getrocknet und das Solvens unter reduziertem Druck am Rotationsverdampfer entfernt. Es wird über Kieselgel 60 (Laufmittel: Toluol/Essigsäureethylester/Essigsäure 35:5:1) gereinigt. Das Solvens wird unter reduziertem Druck entfernt. Schließlich werden letzte Lösungsmittelreste im Hochvakuum entfernt. Es werden 235 mg (15% d.Th. über beide Stufen) der Titelverbindung isoliert.
¹H-NMR (400 MHz, Methanol-d₄): δ = 7.57 (d, 1H), 7.25 (dd, 1H), 7.08 (dt, 1H) ppm.
HPLC: Rₜ = 3.91 min (Methode H)
MS (ESIpos): *m*/*z* = 216 (M+NH₄)⁺

### Beispiel 36A

### 3-Brom-5-fluor-2-hydroxybenzaldehyd

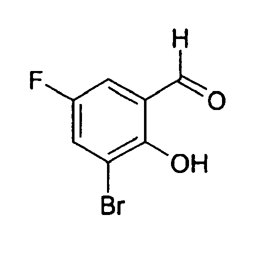

1.0 g (5.24 mmol) 2-Brom-4-fluorphenol werden in 4 ml TFA vorgelegt. 1.47 g (10.47 mmol) Hexamethylentetramin werden portionsweise innerhalb von 20 min. zugegeben. Anschließend wird 28 h unter Rückfluß erhitzt. Bei RT wird mit 6 ml Wasser und 3 ml 50%-iger Schwefelsäure versetzt. Nach 2 h bei RT wird das Reaktionsgemisch zweimal mit je 30 ml Essigsäureethylester extrahiert. Die vereinigten organischen Phasen werden viermal mit 1 N Salzsäure und einmal mit Wasser gewaschen und anschließend über Magnesiumsulfat getrocknet. Das Rohprodukt wird ohne weitere Aufreinigung weiter umgesetzt.
¹H-NMR (200 MHz, CDCl₃): δ = 11.32 (s, 1H, br), 7.66-7.45 (m, 1H), 7.32-7.21 (m, 1H) ppm.
LC-MS (Methode D): Rₜ = 4.2 min.
MS (ESIneg): *m*/*z* = 217 (M-H)⁻

### Beispiel 37A

### 7-Brom-5-fluor-1-benzofuran-2-carbonsäure

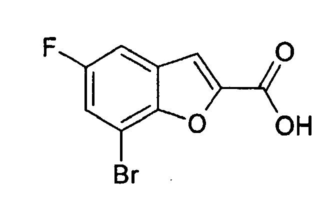

### Stufe a):

1.0 g (5.24 mmol) 2-Brom-4-fluorphenol werden in 4.0 ml Trifluoressigsäure vorgelegt. Es werden portionsweise innerhalb von 20 min. 1.47 g (10.47 mmol) Hexamethylentetraamin zugegeben. Anschließend wird 28 h unter Rückfluß erhitzt. Bei RT werden 6 ml Wasser und 3 ml 50%-ige Schwefelsäure zugegeben. Nach 2 h wird zweimal mit insgesamt 60 ml Essigsäureethylester extrahiert. Die vereinigten organischen Phasen werden viermal mit 1 N Salzsäure und einmal mit Wasser gewaschen. Es wird über Magnesiumsulfat getrocknet und das Solvens unter reduziertem Druck entfernt. Schließlich werden letzte Lösungsmittelreste im Hochvakuum entfernt. Das Rohprodukt wird ohne weitere Aufreinigung weiter umgesetzt.

### Stufe b):

Das Rohprodukt aus der vorigen Umsetzung und 0.19 g (0.52 mmol) Tetra-N-butylammoniumiodid werden zusammen mit 2.9 g (20.96 mmol) wasserfreiem Kaliumcarbonat vorgelegt. Es werden 1.19 g (11.0 mmol) Chloressigsäuremethylester zugegeben. Das Reaktionsgemisch wird 4 h auf 130°C erhitzt und anschließend mittels eines Eisbades auf 0°C abgekühlt. Es werden 18 ml THF, 1.76 g (31.44 mmol) Kaliumhydroxid und 18 ml Wasser zugegeben. Es wird über Nacht bei RT gerührt. Das Solvens wird unter reduziertem Druck entfernt. Es wird mit Wasser verdünnt und mit konz. Salzsäure sauer gestellt. Es wird zweimal mit Essigsäureethylester extrahiert. Die vereinigten organischen Phasen werden über Magnesiumsulfat getrocknet und das Solvens unter reduziertem Druck am Rotationsverdampfer entfernt. Es wird über Kieselgel 60 (Laufmittel: Toluol/Essigsäure 40:1) gereinigt. Das Solvens wird unter reduziertem Druck entfernt. Schließlich werden letzte Lösungsmittelreste im Hochvakuum entfernt. Es werden 257 mg (19% d.Th. über beide Stufen) der Titelverbindung isoliert.
¹H-NMR (400 MHz, Methanol-d₄): δ = 7.60 (m, 1H), 7.48-7.35 (m, H).
HPLC: Rₜ = 4.1 min (Methode H)
MS (ESIpos): *m*/*z* = 276 (M+NH₄)⁺

### Beispiel 38A

### 2-(Hydroxymethyl)-5-nitrophenol

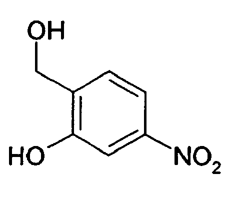

10.0 g (54.6 mmol) 4-Nitrosalicylsäure werden in 100 ml THF vorgelegt. Unter Eiskühlung versetzt man mit 109 ml 1 M Boran-THF-Komplex und läßt über Nacht bei RT nachrühren. Es wird eingeengt und der Niederschlag abgesaugt. Der Feststoff wird in Essigsäureethylester gelöst und über Magnesiumsulfat getrocknet. Nach Einengen und Trocknen im Hochvakuum wird die Titelverbindung direkt weiter umgesetzt.
MS (ESIpos): *m*/*z* = 169 (M⁺)

### Beispiel 39A

### 2-Hydroxy-4-nitrobenzaldehyd

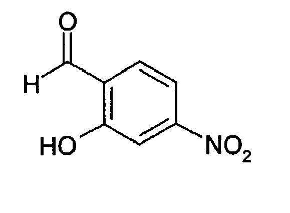

6.0 g (35.5 mmol) 2-(Hydroxymethyl)-5-nitrophenol und 3.1 g (35.5 mmol) aktiviertes Mangan(IV)oxid werden in 100 ml Chloroform für 20 h unter Rückfluß erhitzt. Es wird über Kieselgur filtriert, eingeengt und im Hochvakuum getrocknet. Die Titelverbindung wird direkt weiter umgesetzt.
MS (ESIpos): *m*/*z* = 167 (M⁺)

### Beispiel 40A

### 6-Nitro-1-benzofuran-2-carbonsäure

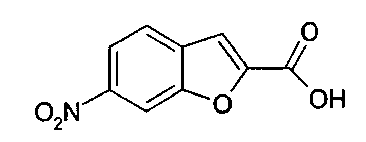

5.8 g (34.7 mmol) 2-Hydroxy-4-nitrobenzaldehyd, 1.28 g (3.5 mmol) Tetra-N-butylammoniumiodid und 19.2 g (138.8 mmol) Kaliumcarbonat werden vermischt, mit 7.9 g (72.9 mmol) Chloressigsäuremethylester versetzt und für 12 h auf 130°C erhitzt. Es werden 100 ml THF hinzugefügt und unter Eiskühlung 11.7 g (208.2 mmol) Kaliumhydroxid zugegeben. Nach Zugabe von 100 ml Wasser wird 20 h bei RT gerührt. Der pH-Wert wird mittels konz. Salzsäure auf pH 0 eingestellt. Es wird mit Essigsäureethylester extrahiert. Die organische Phase wird mit Wasser gewaschen und über Natriumsulfat getrocknet. Nach Zugabe von Kieselgel wird eingeengt und an Kieselgel chromatographiert (Laufmittelgradient: Toluol bis Toluol/Methanol/Eisessig 35:5:1). Nach Einengen der Produktfraktionen und Trocknen im Vakuum erhält man 1.31 g (18.2% d.Th.) der Titelverbindung.
HPLC: Rₜ = 3.8 min (Methode H)
MS (ESIpos): *m*/*z* = 225 (M+NH₄)⁺

### Beispiel 41A

### 7-Nitro-1-benzofuran-2-carbonsäure

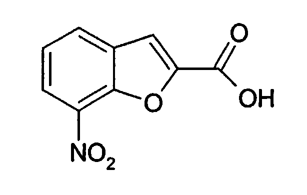

3.0 g (17.9 mmol) 2-Hydroxy-5-nitrobenzaldehyd, 0.66 g (1.80 mmol) Tetra-N-butylammoniumiodid und 9.92 g (71.81 mmol) Kaliumcarbonat werden vermischt, mit 4.09 g (37.7 mmol) Chloressigsäuremethylester versetzt und für 4 h auf 130°C erhitzt. Es werden 35 ml THF hinzugefügt und unter Eiskühlung 6.04 g (107.71 mmol) Kaliumhydroxid zugegeben. Nach Zugabe von 50 ml Wasser wird 20 h bei RT gerührt. Der pH-Wert wird mittels konz. Salzsäure auf pH 0 eingestellt. Es wird mit Essigsäureethylester extrahiert. Die organische Phase wird mit Wasser gewaschen und über Natriumsulfat getrocknet. Nach Zugabe von Kieselgel wird eingeengt und an Kieselgel chromatographiert (Laufmittelgradient: Toluol bis Toluol/Methanol/Eisessig 35:5:1). Nach Einengen der Produktfraktionen und Trocknen im Vakuum erhält man 2.00 g (54% d.Th.) der Titelverbindung.
¹H-NMR (300 MHz, DMSO-d₆): δ = 14.0 (s, 1H, br), 8.36 (d, 1H), 8.25 (d, 1H), 7.88 (s, 1H), 7.59 (t, 1H) ppm.
HPLC: Rₜ = 3.7 min (Methode H)
LC-MS (Methode G): Rₜ = 2.52 min., *m*/*z* = 208 (M+H)⁺
MS (ESIpos): *m*/*z* = 208 (M+H)⁺

### Beispiel 42A

### 6-[(tert.-Butoxycarbonyl)amino]-1-benzothiophen-2-carbonsäure

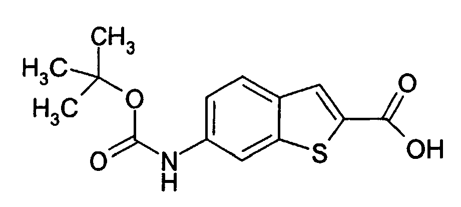

Zu 2.5 ml (2.59 mmol) einer auf 0°C gekühlten 1 M Natriumhexamethyldisilazan-Lösung in Tetrahydrofuran werden 200 mg (1.04 mmol) 6-Amino-1-benzothiophen-2-carbonsäure gegeben. Bei RT wird 15 Minuten nachgerührt. 271.1 mg (1.24 mmol) Pyrokohlensäure-di-tert.-butylester (Boc₂O) werden hinzugefügt. Man läßt für weitere 2 h bei RT rühren, engt den Kolbeninhalt im Vakuum ein, verteilt den Rückstand zwischen 10%-iger Zitronensäurelösung und Essigsäureethylester und extrahiert mit Essigsäureethylester. Die vereinigten organischen Phasen werden über Natriumsulfat getrocknet und eingeengt. Der Rückstand wird mittels präparativer HPLC gereinigt. Man erhält 15 mg (5% d.Th.) der Titelverbindung.
¹H-NMR (200 MHz, DMSO-d₆): δ = 13.33 (s, 1H, br), 9.70 (s, 1H), 8.20 (m, 1H), 7.96 (s, 1H), 7.85 (d, 1H), 7.42 (dd, 1H) ppm.
HPLC: Rₜ = 4.4 min (Methode H)
MS (ESIpos): *m*/*z* = 311 (M+NH₄)⁺

### Beispiel 43A

### 7-Cyano-1-benzothiophen-2-carbonsäure

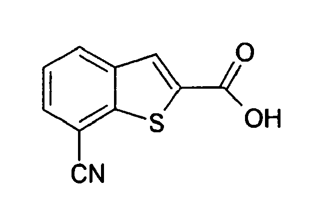

Ausgehend von 1.6 g (7.36 mmol) 7-Cyano-1-benzothiophen-2-carbonsäuremethylester werden 1.5 g eines Edukt/Produkt-Gemisches erhalten, welches ohne weitere Reinigung weiter verwendet wird.

### Beispiel 44A

### 6-Cyano-1-benzothiophen-2-carbonsäure

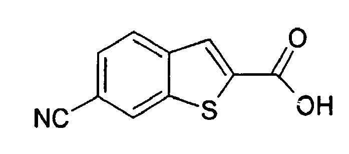

Ausgehend von 0.6 g (2.76 mmol) 6-Cyano-1-benzothiophen-2-carbonsäuremethylester werden 0.49 g (61.6% d.Th.) des gewünschten Produkts erhalten.
HPLC: Rₜ = 3.9 min (Methode H)
MS (ESIpos): *m*/*z* = 222 (M+H)⁺

### Beispiel 45A

### Thieno[2,3-f][1,3]benzodioxol-6-carbonsäure

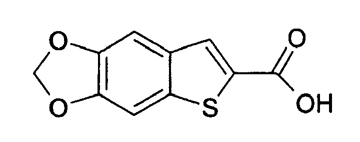

Ausgehend von 700 mg (2.3 mmol) Methyl thieno[2,3-f][1,3]benzodioxol-6-carboxylat werden 513 mg (50% d.Th.) eines Edukt/Produkt-Gemisches erhalten, welches ohne weitere Reinigung weiter verwendet wird.
HPLC: Rₜ = 3.9 min (Methode H)

### Beispiel 46A

### 6-Brom-1-benzothiophen-2-carbonsäure

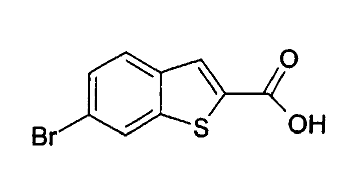

Ausgehend von 4.0 g (14.8 mmol) 6-Brom-1-benzothiophen-2-carbonsäuremethylester werden 3.55 g (93.5% d.Th.) des gewünschten Produkts erhalten.
¹H-NMR (400 MHz, DMSO-d₆): δ = 13.48 (s, 1H, br), 8.38 (s, 1H), 8.22 (s, 1H), 7.96 (d, 1H), 7.63 (m, 1H) ppm.
HPLC: Rₜ = 4.5 min (Methode H)

### Beispiel 47A

### 5-Nitro-1-benzothiophen-2-carbonsäure

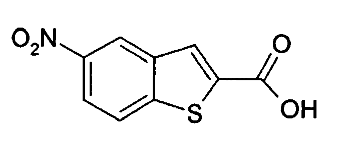

Ausgehend von 1.10 g (4.39 mmol) 5-Nitro-1-benzothiophen-2-carbonsäuremethylester werden 0.93 g (94.8% d.Th.) des gewünschten Produkts erhalten.
¹H-NMR (400 MHz, DMSO-d₆): δ = 13.88 (s, 1H, br), 8.98 (s, 1H), 8.38-8.25 (m, 3H) ppm.
HPLC: Rₜ = 3.9 min (Methode H)
MS (ESIpos): *m*/*z* = 241 (M+NH₄)⁺

### Beispiel 48A

### 7-Brom-1-benzothiophen-2-carbonsäure

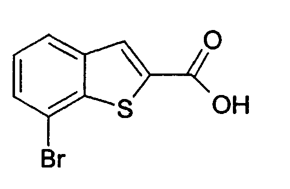

Ausgehend von 10.0 g (36.9 mmol) 7-Brom-1-benzothiophen-2-carbonsäuremethylester werden 8.99 g (91.0% d.Th.) des gewünschten Produkts erhalten.
¹H-NMR (200 MHz, DMSO-d₆): δ = 13.76 (s, 1H, br), 8.28 (s, 1H), 8.07 (d, 1H), 7.78 (d, 1H), 7.46 (dd, 1H) ppm.
HPLC: Rₜ = 4.4 min (Methode H)

### Beispiel 49A

### 6-Nitro-1-benzothiophen-2-carbonsäure

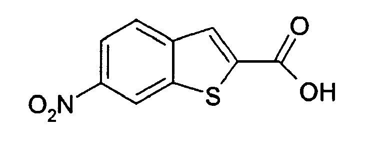

Ausgehend von 16 g (67.4 mmol) 6-Nitro-1-benzothiophen-2-carbonsäuremethylester werden 15.0 g (99.9% d.Th.) des gewünschten Produkts erhalten.
¹H-NMR (200 MHz, DMSO-d₆): δ = 8.91 (d, 1H), 8.15 (dd, 1H), 8.02 (d, 1H), 7.69 (s, 1H) ppm.
HPLC: Rₜ = 4.1 min (Methode H)
MS (ESIpos): *m*/*z* = 241 (M+NH₄)⁺

### Beispiel 50A

### 7-Trifluormethyl-1-benzothiophen-2-carbonsäure

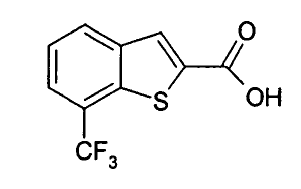

Ausgehend von 4.2 g (16.2 mmol) 7-Trifluormethyl-1-benzothiophen-2-carbonsäuremethylester werden 3.89 g (97.8% d.Th.) des gewünschten Produkts erhalten.
¹H-NMR (500 MHz, DMSO-d₆): δ = 13.88 (s, 1H, br), 8.35 (d, 1H), 8.28 (s, 1H), 7.97 (d, 1H), 7.70 (dd, 1H) ppm.
HPLC: Rₜ = 4.4 min (Methode H)
MS (ESIpos): *m*/*z* = 247 (M+H)⁺

### Beispiel 51A

### 5-Trifluormethyl-1-benzothiophen-2-carbonsäure

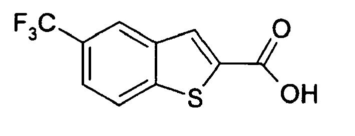

Ausgehend von 4.28 g (18.5 mmol) 5-Trifluormethyl-1-benzothiophen-2-carbonsäuremethylester werden 3.62 g (79.4% d.Th.) des gewünschten Produkts erhalten.
¹H-NMR (400 MHz, DMSO-d₆): δ = 13.78 (s, 1H, br), 8.47 (s, 1H), 8.32 (d, 1H), 8.27 (s, 1H), 7.81 (d, 1H) ppm.
HPLC: Rₜ = 4.5 min (Methode H)
MS (ESIpos): *m*/*z* = 246 (M⁺)

### Beispiel 52A

### 7-Chlor-1-benzothiophen-2-carbonsäure

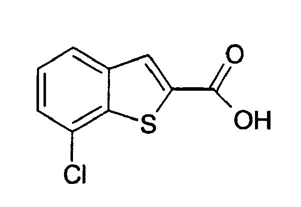

Ausgehend von 500 mg (2.21 mmol) 7-Chlor-1-benzothiophen-2-carbonsäuremethylester werden 391.6 mg (82.7% d.Th.) des gewünschten Produkts erhalten.
¹H-NMR (200 MHz, DMSO-d₆): δ = 13.78 (s, 1H, br), 8.20 (s, 1H), 8.03 (d, 1H), 7.66 (d, 1H), 7.53 (dd, 1H) ppm.
HPLC: Rₜ = 4.2 min (Methode H)
MS (ESIpos): *m*/*z* = 212 (M⁺)

### Beispiel 53A

### 7-Fluor-1-benzothiophen-2-carbonsäure

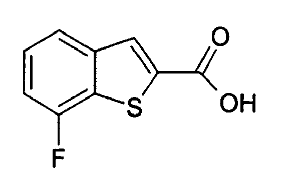

Ausgehend von 3.27 g (15.6 mmol) 7-Fluor-1-benzothiophen-2-carbonsäuremethylester werden 3.05 g (100% d.Th.) des gewünschten Produkts erhalten.
¹H-NMR (200 MHz, CDCl₃): δ = 8.05 (d, 1H), 7.68 (d, 1H), 7.38 (m, 1H), 7.15 (m, 1H) ppm.
HPLC: Rₜ = 4.1 min (Methode H)
MS (ESIpos): *m*/*z* = 214 (M+NH₄)⁺

### Beispiel 54A

### 7-Methoxy-1-benzothiophen-2-carbonsäure

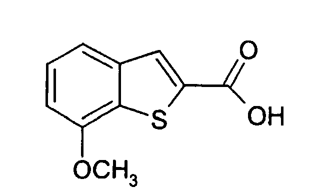

Ausgehend von 300 mg (1.35 mmol) 7-Methoxy-1-benzothiophen-2-carbonsäuremethylester werden 257.8 mg (85.3% d.Th.) des gewünschten Produkts erhalten.
¹H-NMR (200 MHz, DMSO-d₆): δ = 13.52 (s, 1H, br), 8.10 (s, 1H), 7.59 (d, 1H), 7.43 (dd, 1H), 7.08 (dd, 1H) ppm.
HPLC: Rₜ = 4.1 min (Methode H)
MS (ESIpos): *m*/*z* = 226 (M+NH₄)⁺

### Beispiel 55A

### 5-Fluor-1-benzothiophen-2-carbonsäure

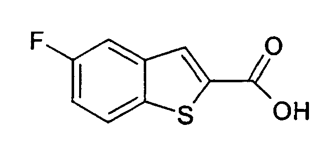

Ausgehend von 1.0 g (4.76 mmol) 5-Fluor-1-benzothiophen-2-carbonsäuremethylester werden 865 mg (92.7% d.Th.) des gewünschten Produkts erhalten.
¹H-NMR (200 MHz, DMSO-d₆): δ = 7.98 (dd, 1H), 7.77 (s, 1H), 7.72 (dd, 1H), 7.29 (ddd, 1H) ppm.
HPLC: Rₜ = 4.1 min (Methode H)
MS (ESIpos): *m*/*z* = 196 (M⁺)

### Beispiel 56A

### 4-Fluor-1-benzothiophen-2-carbonsäure

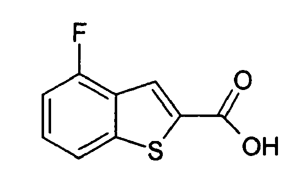

Ausgehend von 3.0 g (14.3 mmol) 4-Fluor-1-benzothiophen-2-carbonsäuremethylester werden 2.66 g (91.1% d.Th.) des gewünschten Produkts erhalten.
¹H-NMR (200 MHz, DMSO-d₆): δ = 13.78 (s, 1H, br), 8.05 (s, 1H), 7.92 (d, 1H), 7.55 (ddd, 1H), 7.29 (dd, 1H) ppm.
HPLC: Rₜ = 4.1 min (Methode H)
MS (ESIpos): *m*/*z* = 196 (M⁺)

### Beispiel 57A

### 5,7-Difluor-1-benzothiophen-2-carbonsäure

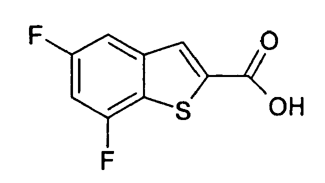

Ausgehend von 2.4 g (10.5 mmol) 5,7-Difluor-1-benzothiophen-2-carbonsäuremethylester werden 2.1g (93.1 % d.Th.) des gewünschten Produkts erhalten.
¹H-NMR (200 MHz, DMSO-d₆): δ = 13.88 (s, 1H, br), 8.17 (d, 1H), 7.77 (dd, 1H), 7.56 (m, 1H) ppm.
HPLC: Rₜ = 3.9 min (Methode H)
MS (ESIpos): *m*/*z* = 214 (M⁺)

### Beispiel 58A

### 5,6-Dimethoxy-1-benzothiophen-2-carbonsäure

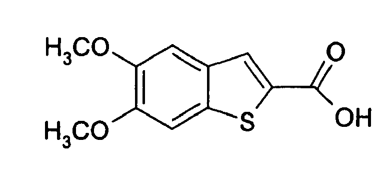

Ausgehend von 1.0 g (2.97 mmol) 5,6-Dimethoxy-1-benzothiophen-2-carbonsäuremethylester werden 0.77 g (99% d.Th.) des gewünschten Produkts erhalten.
¹H-NMR (200 MHz, DMSO-d₆): δ = 13.17 (s, 1H, br), 7.93 (s, 1H), 7.58 (s, 1H), 7.48 (s, 1H), 3.85 (s, 3H), 3.82 (s, 3H) ppm.
HPLC: Rₜ = 3.7 min (Methode H)
MS (ESIpos): *m*/*z* = 239 (M⁺), 256 (M+NH₄)⁺

### Beispiel 59A

### 6-Methoxy-1-benzothiophen-2-carbonsäure

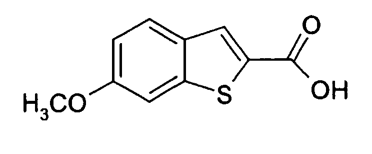

Ausgehend von 2.52 g (11.34 mmol) 6-Methoxy-1-benzothiophen-2-carbonsäuremethylester werden 2.26 g (95.7% d.Th.) des gewünschten Produkts erhalten.
¹H-NMR (200 MHz, DMSO-d₆): δ = 13.29 (s, 1H, br), 8.01 (s, 1H), 7.88 (d, 1H), 7.60 (d, 1H), 7.07 (dd, 1H), 3.86 (s, 3H) ppm.
HPLC: Rₜ = 3.9 min (Methode H)
MS (ESIpos): *m*/*z* = 209 (M+H⁺), 226 (M+NH₄)⁺

### Beispiel 60A

### 4-Nitro-1-benzothiophen-2-carbonsäure

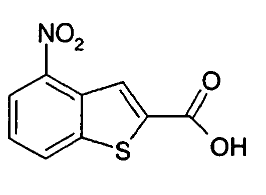

Ausgehend von 1.0 g (4.22 mmol) 4-Nitro-1-benzothiophen-2-carbonsäuremethylester werden 0.89 g (94.1% d.Th.) des gewünschten Produkts erhalten.
¹H-NMR (200 MHz, DMSO-d₆): δ = 14.08 (m, 1H, br), 8.58 (d, 1H), 8.57 (s, 1H), 8.42 (d, 1H), 7.77 (dd, 1H) ppm.
HPLC: Rₜ = 4.0 min (Methode H)
MS (ESIpos): *m*/*z* = 241 (M+NH₄)⁺

### Beispiel 61A

### 7-Nitro-1-benzothiophen-2-carbonsäure

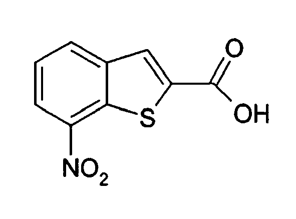

Ausgehend von 3.6 g (13.7 mmol) 7-Nitro-1-benzothiophen-2-carbonsäuremethylester werden 3.08 g (99.5% d.Th.) des gewünschten Produkts erhalten.
¹H-NMR (300 MHz, DMSO-d₆): δ = 13.79 (s, 1H, br), 8.58 (d, 1H), 8.51 (d, 1H), 8.33 (s, 1H), 7.78 (dd, 1H) ppm.
HPLC: Rₜ = 4.0 min (Methode H)
MS (ESIpos): *mlz* = 241 (M+NH₄)⁺

### Beispiel 62A

### N-[(3R)-1-Azabicyclo[2.2.2]oct-3-yl]-6-nitro-1-benzothiophen-2-carboxamid-Hydrochlorid

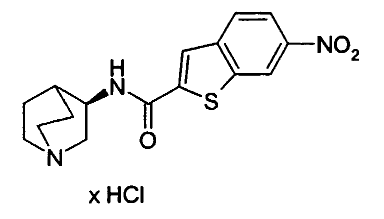

290 mg (1.30 mmol) 6-Nitro-1-benzothiophen-2-carbonsäure, 258.7 mg (1.30 mmol) *R*-3-Aminochinuklidin-Dihydrochlorid, 592.8 mg (1.56 mmol) HATU, 604.5 mg (4.68 mmol) N,N-Diisopropylethylamin und 2.0 ml DMF werden gemäß der allgemeinen Arbeitsvorschrift umgesetzt. Das Reaktionsgemisch wird durch präparative HPLC gereinigt. Die Produktfraktionen werden vereinigt, mit 1 N Salzsäure versetzt, anschließend eingeengt und im Hochvakuum getrocknet. Man erhält 297 mg (62.1% d.Th.) der Titelverbindung.
¹H-NMR (400 MHz, Methanol-d₄): δ = 8.95 (s, 1H), 8.29 (dd, 1H), 8.20 (s, 1H), 8.11 (d, 1H), 4.47 (m, 1H), 3.82 (m, 1H), 3.52-3.22 (m, 5H), 2.40 (m, 1H), 2.28 (m, 1H), 2.11 (m, 2H), 1.97 (m, 1H) ppm.
HPLC: Rₜ = 3.8 min (Methode H)
MS (ESIpos): *mlz* = 332 (M+H)⁺ (freie Base).

### Beispiel 63A

### N-[(3R)-1-Azabicyclo[2.2.2]oct-3-yl]-3-chlor-6-nitro-1-benzothiophen-2-carboxamid

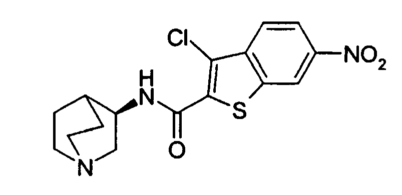

427 mg (1.66 mmol) 3-Chlor-6-nitro-1-benzothiophen-2-carbonsäure, 300 mg (1.51 mmol) *R*-3-Aminochinuklidin-Dihydrochlorid, 687.4 mg (1.81 mmol) HATU, 701 mg (5.43 mmol) N,N-Diisopropylethylamin und 4.0 ml DMF werden gemäß der allgemeinen Arbeitsvorschrift umgesetzt. Es werden 20 ml Methanol und 4 g Dowex WX2-200 zugesetzt und 1 h bei RT gerührt. Man filtriert und wäscht sukzessive mit Wasser, Methanol, Methanol/Trifluoressigsäure 9:1 und Methanol/Trifluoressigsäure 1:1. Nach Eindampfen des Filtrats erhält man 270 mg (49% d.Th.) der Titelverbindung.
¹H-NMR (200 MHz, DMSO-d₆): δ = 9.24 (d, 1H), 8.77 (d, 1H), 8.38 (dd, 1H), 8.11 (d, 1H), 3.97 (m, 1H), 3.70-3.22 (m, 2H), 3.14 (m, 1H), 2.97-2.44 (m, 4H), 1.93 (m, 1H), 1.83 (m, 1H), 1.60 (m, 2H), 1.39 (m, 1H) ppm.
HPLC: Rₜ = 3.9 min (Methode H)
MS (ESIpos): *m*/*z* = 366 (M+H)⁺ (freie Base).

### Ausführungsbeispiele:

### Beispiel 1

### N-[(3R)-1-Azabicyclo[2.2.2]oct-3-yl]-5-brom-1-benzothiophen-2-carboxamid-Hydrochlorid

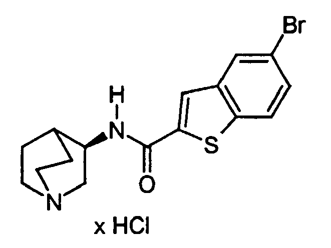

133.7 mg (0.52 mmol) 5-Brom-1-benzothiophen-2-carbonsäure, 155.4 mg (0.78 mmol) (*R*)-3-Aminochinuklidin-Dihydrochlorid, 296.7 mg (0.78 mmol) HATU, 369.8 mg (2.86 mmol) N,N-Diisopropylethylamin und 1.5 ml DMF werden gemäß der allgemeinen Arbeitsvorschrift (Variante A) umgesetzt. Das Reaktionsgemisch wird durch präparative HPLC gereinigt. Das Produkt wird in Acetonitril gelöst und mit einem Überschuß an 1 N Salusäure versetzt. Schließlich wird das Solvens entfernt. Es werden 175 mg (84% d.Th.) der Titelverbindung isoliert.
¹H-NMR (200.1 MHz, DMSO-d₆): δ = 9.44 (br. s, 1H), 8.95 (d, 1H), 8.30-8.10 (m, 2H), 8.03 (d, 1H), 7.60 (m, 1H), 4.38-4.20 (m, 1H), 3.80-3.55 (m, 1H), 3.42-3.05 (m, 5H), 2.25-2.00 (m, 2H), 1.98-1.62 (m, 3H) ppm.
LC-MS (Methode A): Rₜ = 2.63 min., MS (ESIpos): *m*/*z* = 365 (M+H)⁺ (freie Base).

### Beispiel 2

### N-[(3S)-1-Azabicyclo[2.2.2]oct-3-yl]-5-brom-1-benzothiophen-2-carboxamid-Hydrochlorid

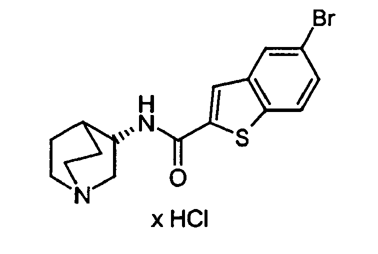

Die Versuchsdurchführung erfolgt in gleicher Weise und Dimension, wie in Beispiel 1 beschrieben, mit *S*-3-Aminochinuklidin-Dihydrochlorid. Es werden 174 mg (83% d.Th.) der Titelverbindung isoliert. Die analytischen Daten stimmen mit denen der enantiomeren Verbindung aus Beispiel 1 überein.

### Beispiel 3

### N-[(3R)-1-Azabicyclo[2.2.2]oct-3-yl]-6-fluor-1-benzothiophen-2-carboxamid-Hydrochlorid

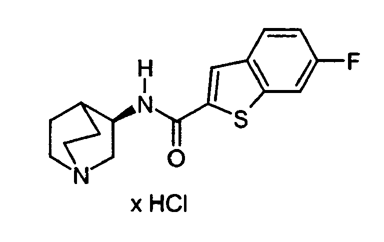

102.1 mg (0.52 mmol) 6-Fluor-1-benzothiophen-2-carbonsäure, 155.4 mg (0.78 mmol) *R*-3-Aminochinuklidin-Dihydrochlorid, 296.7 mg (0.78 mmol) HATU, 369.8 mg (2.86 mmol) N,N-Diisopropylethylamin und 1.5 ml DMF werden gemäß der allgemeinen Arbeitsvorschrift (Variante A) umgesetzt. Das Reaktionsgemisch wird durch präparative HPLC gereinigt. Das Produkt wird in Acetonitril gelöst und mit einem Überschuss an 1 N Salzsäure versetzt. Schließlich wird das Solvens entfernt. Es werden 22 mg (14% d.Th.) der Titelverbindung isoliert.
LC-MS (Methode B): Rₜ =1.22 min., MS (ESIpos): *m*/*z* = 305 (M+H)⁺ (freie Base).

### Beispiel 4

### N-[(3R)-1-Azabicyclo[2.2.2]oct-3-yl]-5-methyl-1-benzothiophen-2-carboxamid-Hydrochlorid

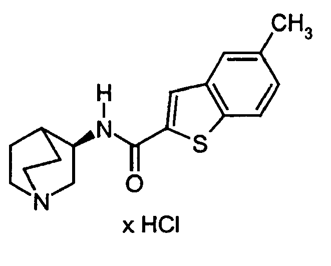

100.0 mg (0.52 mmol) 5-Methyl-1-benzothiophen-2-carbonsäure, 155.4 mg (0.78 mmol) *R*-3-Aminochinuklidin-Dihydrochlorid, 296.7 mg (0.78 mmol) HATU, 369.8 mg (2.86 mmol) N,N-Diisopropylethylamin und 1.5 ml DMF werden gemäß der allgemeinen Arbeitsvorschrift (Variante A) umgesetzt. Das Reaktionsgemisch wird durch präparative HPLC gereinigt. Das Produkt wird in THF gelöst und mit einem Überschuss an 1 N Salzsäure versetzt. Schließlich wird das Solvens entfernt. Es werden 57 mg (36% d.Th.) der Titelverbindung isoliert.
MS (ESIpos): *m*/*z* = 301 (M+H)⁺ (freie Base)
LC-MS (Methode A): Rₜ = 2.50 min., MS (ESIpos): *m*/*z* = 301 (M+H)⁺ (freie Base).

### Beispiel 5

### N-[(3S)-1-Azabicyclo[2.2.2]oct-3-yl]-5-methyl-1-benzothiophen-2-carboxamid-Hydrochlorid

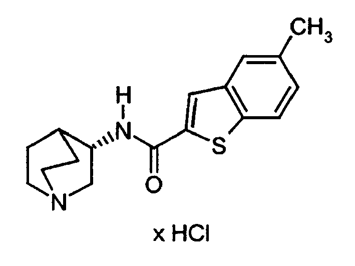

Die Versuchsdurchführung erfolgt in gleicher Weise und Dimension, wie in Beispiel 4 beschrieben, mit S-3-Aminochinuklidin-Dihydrochlorid. Es werden 117 mg (75% d.Th.) der Titelverbindung isoliert. Die analytischen Daten stimmen mit denen der enantiomeren Verbindung aus Beispiel 4 überein.

### Beispiel 6

### N-[(3R)-1-Azabicyclo[2.2.2]oct-3-yl]thieno[3,2-b]pyridin-2-carboxamid

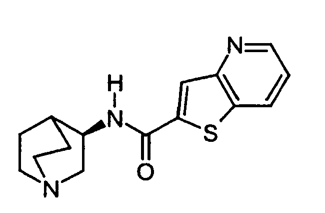

35 mg (0.20 mmol) Thieno[3,2-b]pyridin-2-carbonsäure, 58.3 mg (0.29 mmol) R-3-Aminochinuklidin-Dihydrochlorid, 111.4 mg (0.29 mmol) HATU, 138.8 mg (1.07 mmol) N,N-Diisopropylethylamin und 1.5 ml DMF werden gemäß der allgemeinen Arbeitsvorschrift (Variante A) umgesetzt. Das Reaktionsgemisch wird durch präparative HPLC gereinigt und anschließend über Kieselgel 60 (Laufmittel: Dichlormethan/Methanol/Ammoniak 90:9:1) feingereinigt. Es werden 44 mg (78% d.Th.) der Titelverbindung isoliert.
¹H-NMR (400.1 MHz, DMSO-d₆): δ = 8.74 (d, 1H), 8.69 (d, 1H), 8.51 (d, 1H), 8.41 (s, 1H), 7.45 (dd, 1H), 4.02-3.92 (m, 1H), 3.55-3.38 (m, 1H), 2.95-2.85 (m, 1H), 2.78-2.63 (m, 4H), 1.95-1.80 (m, 2H), 1.68-1.52 (m, 2H), 1.40-1.30 (m, 1H) ppm.
MS (ESIpos): *m*/*z* = 288 (M+H)⁺ (freie Base)
LC-MS (Methode A): Rₜ = 0.39 min., MS (ESIpos): *m*/*z* = 288 (M+H)⁺ (freie Base).

### Beispiel 7

### N-[(3S)-1-Azabicyclo[2.2.2]oct-3-yl]thieno[3,2-b]pyridin-2-carboxamid

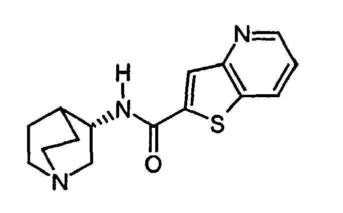

Die Versuchsdurchführung erfolgt in gleicher Weise und Dimension, wie in Beispiel 6 beschrieben, mit *S*-3-Aminochinuklidin-Dihydrochlorid. Es werden 38 mg (68% d.Th.) der Titelverbindung isoliert. Die analytischen Daten stimmen mit denen der enantiomeren Verbindung aus Beispiel 6 überein.

### Beispiel 8

### N-[(3R)-1-Azabicyclo[2.2.2]oct-3-yl]thieno[2,3-b]pyridin-2-carboxamid-Hydrochlorid

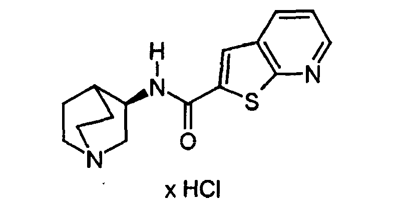

45.0 mg (0.25 mmol) Thieno[2,3-b]pyridin-2-carbonsäure, 75.0 mg (0.38 mmol) *R-*3-Aminochinuklidin-Dihydrochlorid, 143.2 mg (0.38 mmol) HATU, 178.5 mg (1.38 mmol) N,N-Diisopropylethylamin und 1.5 ml DMF werden gemäß der allgemeinen Arbeitsvorschrift (Variante A) umgesetzt. Das Reaktionsgemisch wird durch präparative HPLC gereinigt. Es werden 37 mg (51% d.Th.) der Titelverbindung isoliert.
¹H-NMR (200.1 MHz, DMSO-d₆): δ = 9.49 (br. s, 1H), 8.93 (d, 1H), 8.67 (dd, 1H), 8.42 (dd, 1H), 8.19 (s, 1H), 7.52 (dd, 1H), 4.38-4.20 (m, 1H), 3.80-3.55 (m, 1H), 3.42-3.05 (m, 5H), 2.25-2.00 (m, 2H), 1.98-1.62 (m, 3H) ppm.
MS (ESIpos): *m*/*z* = 288 (M+H)⁺ (freie Base)
LC-MS (Methode A): Rₜ = 0.37 min., MS (ESIpos): *m*/*z* = 288 (M+H)⁺ (freie Base).

### Beispiel 9

### rac-N-(1-Azabicyclo[2.2.2]oct-3-yl)-5-nitro-1-benzothiophen-2-carboxamid-Hydrochlorid

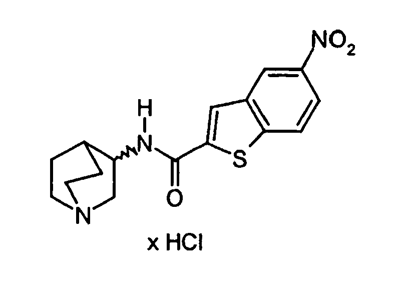

Zu einer Lösung von 180 mg (0.81 mmol) 5-Nitrothiophen-2-carbonsäure und Diisopropylethylamin (0.8 ml) in 4 ml DMF werden bei RT zunächst 244 mg (0.76 mmol) TBTU und 104 mg (0.78 mmol) HOBt gegeben, anschließend werden 153 mg (0.77 mmol) 3-Aminochinuclidin-Dihydrochlorid zugegeben. Das Gemisch wird 4 h bei RT gerührt. Zur Aufarbeitung wird eingeengt und in einer Mischung aus Chloroform und überschüssiger wässriger Natronlauge aufgenommen. Die Phasen werden getrennt und die wässrige Phase mehrmals mit Chloroform nachextrahiert. Die vereinigten organischen Phasen werden über Natriumsulfat getrocknet, eingeengt und das Rohprodukt an Kieselgel chromatographiert (Laufmittel: Chloroform/Methanol/konz. Ammoniak 100:5:0.5 → 100:20:2). Das erhaltene Produkt wird in THF auf-genommen, mit überschüssigem HCl in Diethylether versetzt, eingeengt und im Hochvakuum getrocknet. Man erhält 136 mg (47% d.Th.) des Hydrochlorids. ¹H-NMR (200.1 MHz, DMSO-d₆): δ = 10.20 (br. s, 1H), 9.32 (d, 1H), 8.90 (d, 1H), 8.55 (s, 1H), 8.40-8.20 (m, 2H), 4.40-4.35 (m, 1H), 3.75-3.60 (m, 1H), 3.42-3.15 (m, 4H), 2.28-2.05 (m, 2H), 2.00-1.65 (m, 3H) ppm.
MS (ESIpos): *m*/*z* = 332 (M+H)⁺ (freie Base).

### Beispiel 10

### rac-N-(1-Azabicyclo[2.2.2]oct-3-yl)-5-amino-1-benzothiophen-2-carboxamid-Dihydrochlorid

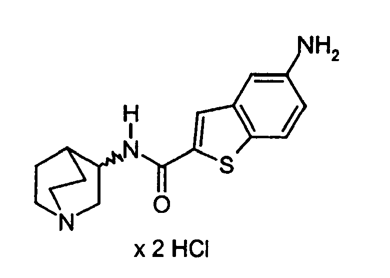

129 mg (0.35 mmol) *rac-N*-(1-Azabicyclo[2.2.2]oct-3-yl)-S-nitro-1-benzothiophen-2-carboxamid-Hydrochlorid werden in einer Mischung aus 5 ml Essigsäure und 3 ml Wasser gelöst. Es werden 114.7 mg (1.75 mmol) Zink zugegeben und 5 h bei RT nachgerührt. Das Reaktionsgemisch wird über Kieselgur filtriert, vom Lösungsmittel befreit und in Dichlormethan aufgenommen. Die resultierende Lösung wird mit 1 N Natronlauge gewaschen. Die organische Phase wird über Natriumsulfat getrocknet und anschließend im Vakuum vom Solvens befreit. Der resultierende Festsoff wird in wenig THF gelöst und mit einem Überschuss an 1 N HCl in Diethylether versetzt. Schließlich wird das THF abdestilliert. Es werden 103 mg (80% d.Th.) der Titelverbindung isoliert.
LC-MS (Methode A): Rₜ = 0.36 min., MS (ESIpos): *m*/*z* = 302 (M+H)⁺ (freie Base).

### Beispiel 11

### rac-N-(1-Azabicyclo[2.2.2]oct-3-yl)-5-acetylamino-1-benzothiophen-2-carboxamid-Hydrochlorid

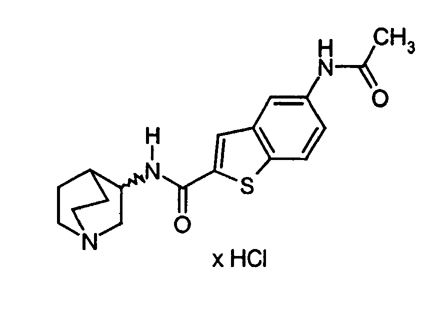

41.0 mg (0.12 mmolo) *rac*-*N*-(1-Azabicyclo[2.2.2]oct-3-yl)-5-amino-1-benzothiophen-2-carboxamid-Dihydrochlorid werden zusammen mit 36.8 mg (0.36 mmol) Triethylamin in 1.5 ml DMF gelöst. Bei 0°C werden 14.3 mg (0.18 mmol) Acetylchlorid zugegeben und über Nacht bei RT nachgerührt. Das Reaktionsgemisch wird durch präparative HPLC gereinigt. Es werden 29 mg (63% d.Th.) der Titelverbindung isoliert.
¹H-NMR (400.1 MHz, DMSO-d₆): δ = 10.55 (br. s, 1H), 10.25 (s, 1H), 9.20 (d, 1H), 8.40 (s, 1H), 8.30 (s, 1H), 7.90 (d, 1H), 7.56 (m, 1H), 4.38-4.25 (m, 1H), 3.65-3.55 (m, 1H), 3.50-3.35 (m, 2H), 3.25-3.10 (m, 3H), 2.22-2.10 (m, 2H), 2.07 (s, 3H), 1.95-1.80 (m, 2H), 1.80-1.70 (m, 1H) ppm.
LC-MS (Methode A): Rₜ = 1.22 min., MS (ESIpos): *m*/*z* = 344 (M+H)⁺ (freie Base).

### Beispiel 12

### N-(1-Azabicyclo[2.2.2]oct-3-yl)thieno[2,3-b]chinolin-2-carboxamid-Hydrochlorid

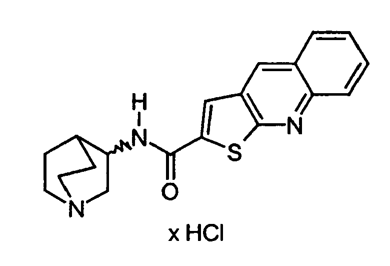

Analog der Vorschrift für die Verbindung aus Beispiel 9 werden ausgehend von 242 mg (1.05 mmol) Thieno[2,3-b]chinolin-2-carbonsäure [Lit.: B. Bhat et al., *Synthesis,* 673ff. (1984)] insgesamt 314 mg (83% d.Th.) der Titelverbindung als farbloser Feststoff erhalten.
¹H-NMR (300.1 MHz, DMSO-d₆): δ = 10.50 (br. s, 1H), 9.45 (d, 1H), 9.02 (s, 1H), 8.58 (s, 1H), 8.22 (d, 1H), 8.10 (d, 1 H), 7.86 (m_{c}, 1H), 7.66 (m_{c}, 1H), 3.72-3.58 (m, 2H), 3.50-3.40 (m, 2H), 3.30-3.15 (m, 3H), 2.28-2.15 (m, 2H), 1.98-1.86 (m, 2H), 1.82-1.70 (m, 1H), 1.80-1.70 (m, 1H) ppm.
MS (ESIpos): *m*/*z* = 338 (M+H)⁺ (freie Base).

### Beispiel 13

### N-(1-Azabicyclo[2.2.2]oct-3-yl)benzothiophen-2-carboxamid-Hydrochlorid

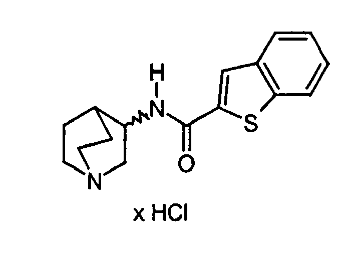

Analog der Vorschrift für die Verbindung aus Beispiel 9 werden ausgehend von 326 mg (1.83 mmol) Benzothiophen-2-carbonsäure insgesamt 332 mg (56% d.Th.) der Titelverbindung als farbloser Feststoff erhalten.
¹H-NMR (300 MHz, CD₃OD): δ = 8.1 (s, 1H), 7.90 (m, 2H), 7.40 (m, 2H), 4.45 (m, 1H), 3.80 (m, 1H), 3.55-3.20 (m, 5H), 2.4-2.20 (m, 2H), 2.10 (m, 2H), 1.95 (m, 1H) ppm.
MS (ESIpos): *m*/*z* = 287 (M+H)⁺ (freie Base).

### Beispiel 14

### N-(1-Azabicyclo[2.2.2]oct-3-yl)-3-chlor-1-benzothiophen-2-carboxamid-Hydrochlorid

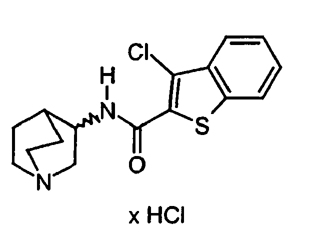

Analog der Vorschrift für die Verbindung aus Beispiel 9 werden ausgehend von 122 mg (0.57 mmol) 3-Chlorbenzothiophen-2-carbonsäure insgesamt 26 mg (13% d.Th.) der Titelverbindung als farbloser Feststoff erhalten.
¹H-NMR (300 MHz, CD₃OD): δ = 8.00 (m, 2H), 7.60 (m, 2H), 4.55 (m, 1H), 3.95 (m, 1H), 3.55-3.20 (m, 5H), 2.50 (m, 1H), 2.35 (m, 1H), 2.15 (m, 2H), 2.05 (m, 1H) ppm.
MS (ESIpos): *m*/*z* = 321 (M+H)⁺ (freie Base).

### Beispiel 15

### N-(1-Azabicyclo[2.2.2]oct-3-yl)-7-brom-1-benzothiophen-2-carboxamid-Hydrochlorid

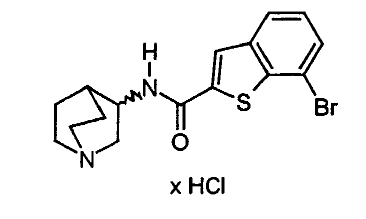

Analog der Vorschrift für die Verbindung aus Beispiel 9 werden ausgehend von 271 mg (1.05 mmol) 3-Chlorbenzothiophen-2-carbonsäure insgesamt 121 mg (29% d.Th.) der Titelverbindung als farbloser Feststoff erhalten.
¹H-NMR (300 MHz, CD₃OD): δ = 8.25 (s, 1H), 7.90 (d, 1H), 7.65 (d, 1H), 7.40 (dd, 1H), 4.45 (m, 1H), 3.85 (m, 1H), 3.55-3.30 (m, 5H), 2.40 (m, 1H), 2.35 (m, 1H), 2.10 (m, 2H), 2.00 (m, 1H) ppm.
MS (ESIpos): *m*/*z* = 365 (M+H)⁺ (freie Base).

### Beispiel 16

### N-(1-Azabicyclo [2.2.2]oct-3-yl)benzofuran-2-carboxamid-Hydrochlorid

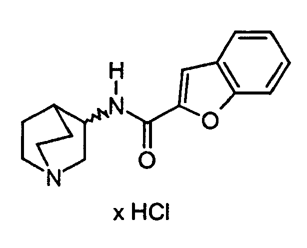

Analog der Vorschrift für die Verbindung aus Beispiel 9 werden ausgehend von 165 mg (0.91 mmol) Benzofuran-2-carbonsäure insgesamt 218 mg (78% d.Th.) der Titelverbindung als farbloser Feststoff erhalten.
¹H-NMR (300 MHz, CDCl₃): δ = 11.50 (s, 1H), 8.6 (d, 1H), 7.80 (s, 1H), 7.60 (d, 1H), 7.55 (d, 1H), 7.35 (dd, 1H), 7.20 (dd, 1H), 4.65 (m, 1H), 4.25 (m, 1H), 4.05 (m, 1H), 3.55 (m, 1H), 3.40-3.20 (m, 3H), 2.40 (m, 1H), 2.25-2.00 (m, 2H), 1.95-1.75 (m, 2H) ppm.
MS (ESIpos): *m*/*z* = 271 (M+H)⁺ (freie Base).

### Beispiel 17

### N-[(3R)-1-Azabicyclo[2.2.2]oct-3-yl]benzofuran-2-carboxamid-Hydrochlorid

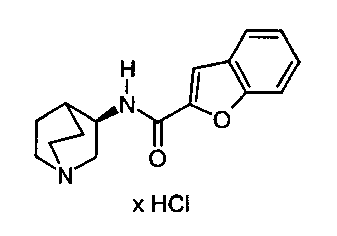

Analog der Vorschrift für die Verbindung aus Beispiel 16 wird durch entsprechende Umsetzung mit 3R-Aminochinuclidin-Dihydrochlorid das entsprechende enantiomerenreine Produkt erhalten. Die analytischen Daten entsprechen denjenigen von Beispiel 16.

### Beispiel 18

### N-[(3R)-1-Azabicyclo[2.2.2]oct-3-yl]-8-brombenzothiophen-2-carboxamid-Hydrochlorid

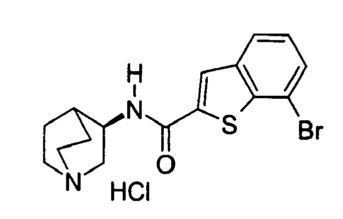

Analog der Vorschrift für die Verbindung aus Beispiel 15 wird durch entsprechende Umsetzung mit 3R-Aminochinuclidin-Dihydrochlorid das entsprechende enantiomerenreine Produkt erhalten. Die analytischen Daten entsprechen denjenigen von Beispiel 15.

### Beispiel 19

### N-[(3R)-1-Azabicyclo[2.2.2]oct-3-yl]-7-methoxy-1-benzofuran-2-carboxamid-Hydrochlorid

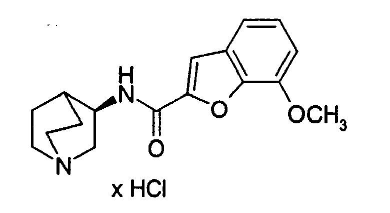

190 mg (0.98 mmol) 7-Methoxybenzofuran-2-carbonsäure, 200 mg (0.98 mmol) *R*-3-Aminochinuklidin-Dihydrochlorid, 450 mg (1.18 mmol) HATU, 461 mg (3.54 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Anschließend wird das Produkt mit einem Überschuß 1 N Salzsäure versetzt. Das Solvens wird unter reduziertem Druck entfernt. Es werden 202 mg (61% d.Th.) der Titelverbindung isoliert.
¹H-NMR (200 MHz, DMSO-d₆): δ = 9.55 (s, 1H, br), 8.91 (d, 1H), 7.62 (s, 1H), 7.36-7.22 (m, 2H), 7.11-7.07 (m, 1H), 4.43-4.29 (m, 1 H), 3.95 (s, 3H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 301 (M+H)⁺ (freie Base)
LC-MS (Methode D): Rₜ = 2.80 min., *m*/*z* = 301 (M+H)⁺ (freie Base).

### Beispiel 20

### N-[(3S)-1-Azabicyclo[2.2.2]oct-3-yl]-7-methoxy-1-benzofuran-2-carboxamid-Hydrochlorid

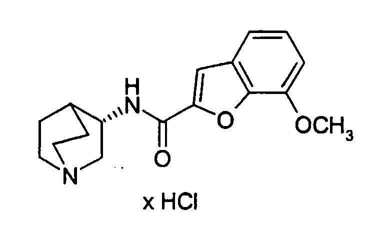

Die Versuchsdurchführung erfolgt in gleicher Weise und Dimension wie in Beispiel 19, jedoch unter Verwendung von *S*-3-Aminochinuklidin-Dihydrochlorid. Es werden 180 mg (54% d.Th.) der Titelverbindung isoliert. Die analytischen Daten stimmen mit denen der enantiomeren Verbindung aus Beispiel 19 überein.

### Beispiel 21

### N-[(3R)-1-Azablcyclo[2.2.2]oct-3-yl]-5,7-dichlor-1-benzefuran-2-carboxamid-Hydrochlorid

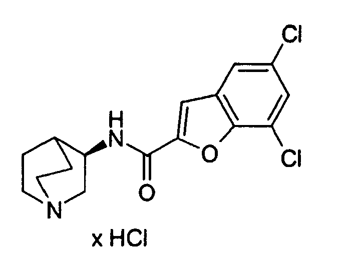

227 mg (0.98 mmol) 5,7-Dichlorbenzofuran-2-carbonsäure, 200 mg (0.98 mmol) *R-*3-Aminochinuklidin-Dihydrochlorid, 449 mg (1.18 mmol) HATU, 458 mg (3.54 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Anschließend wird das Produkt mit einem Überschuß 1 N Salzsäure versetzt. Das Solvens wird unter reduziertem Druck entfernt. Es werden 169 mg (46% d.Th.) der Titelverbindung isoliert.
¹H-NMR (200 MHz, DMSO-d₆): δ = 10.01 (s, 1H, br), 9.11 (d, 1H), 7.94 (d, 1H), 7.82 (s, 1H), 7.65 (d, 1H), 4.43-4.29 (m, 1 H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 339 (M+H)⁺ (freie Base)
LC-MS (Methode D): Rₜ = 3.21 min., *m*/*z* = 339 (M+H)⁺ (freie Base).

### Beispiel 22

### N-[(3S)-1-Azabicyclo[2.2.2]oct-3-yl]-5,7-dichlor-1-benzofuran-2-carboxarnid-Hydrochlorid

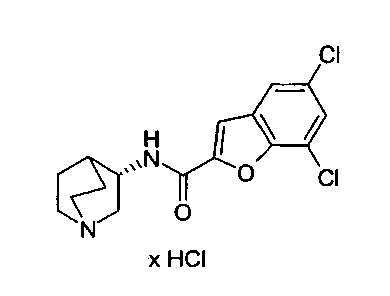

Die Versuchsdurchführung erfolgt in gleicher Weise und Dimension wie für die Verbindung aus Beispiel 21, jedoch unter Verwendung von *S*-3-Aminochinuklidin-Dihydrochlorid. Es werden 216 mg (58% d.Th.) der Titelverbindung isoliert. Die analytischen Daten stimmen mit denen der enantiomeren Verbindung aus Beispiel 21 überein.

### Beispiel 23

### N-[(3R)-1-Azabicyclo[2.2.2]oct-3-yl]-5-brom-1-benzofuran-2-carboxamid-Hydrochlorid

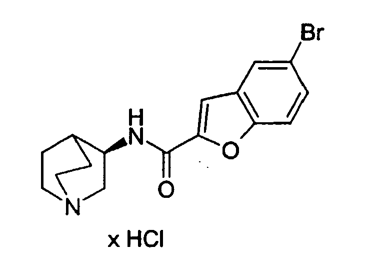

240 mg (0.98 mmol) 5-Brombenzofuran-2-carbonsäure, 200 mg (0.98 mmol) *R*-3-Aminochinuklidin-Dihydrochlorid, 450 mg (1.18 mmol) HATU, 460 mg (3.54 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Anschließend wird das Produkt mit einem Überschuß 1 N Salzsäure versetzt. Das Solvens wird unter reduziertem Druck entfernt. Es werden 202 mg (53% d.Th.) der Titelverbindung isoliert.
¹H-NMR (200 MHz, DMSO-d₆): δ = 9.38 (s, 1H, br), 8.88 (d, 1H), 7.60 (s, 1H), 7.38-7.20 (m, 2H), 7.09 (dd, 1H), 4.43-4.29 (m, 1 H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 349 (M+H)⁺ (freie Base)
LC-MS (Methode D): Rₜ = 2.71 min., *m*/*z* = 349 (M+H)⁺ (freie Base).

### Beispiel 24

### N-[(3S)-1-Azabicyclo[2.2.2]oct-3-yl]-5-brom-1-benzofuran-2-carboxamid-Hydrochlorid

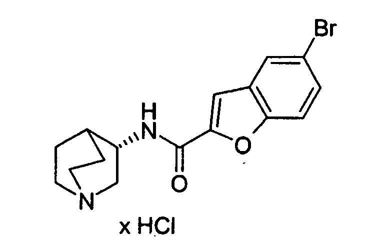

Die Versuchsdurchführung erfolgt in gleicher Weise und Dimension wie für die Verbindung aus Beispiel 23, jedoch unter Verwendung von *S*-3-Aminochinuklidin-Dihydrochlorid. Es werden 277 mg (73% d.Th.) der Titelverbindung isoliert. Die analytischen Daten stimmen mit denen der enantiomeren Verbindung aus Beispiel 23 überein.

### Beispiel 25

### N-[(3R)-1-Azabicyclo[2.2.2]oct-3-yl]-5-chlor-1-benzofuran-2-carboxamid-Hydrochlorid

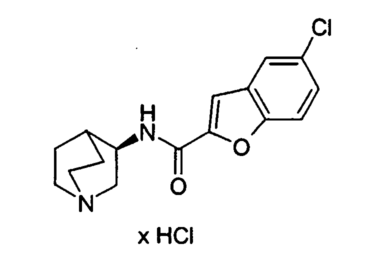

190 mg (0.98 mmol) 5-Chlorbenzofuran-2-carbonsäure, 200 mg (0.98 mmol) *R*-3-Aminochinuklidin-Dihydrochlorid, 450 mg (1.18 mmol) HATU, 460 mg (3.54 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Anschließend wird das Produkt mit einem Überschuß 1 N Salzsäure versetzt. Das Solvens wird unter reduziertem Druck entfernt. Es werden 145 mg (43% d.Th.) der Titelverbindung isoliert.
¹H-NMR (200 MHz, DMSO-d₆): δ = 9.78 (s, 1H, br), 9.07 (d, 1H), 7.91 (d, 1H), 7.72 (d, 1H), 7.64 (s, 1H), 7.51 (dd, 1H), 4.43-4.29 (m, 1 H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 305 (M+H)⁺
LC-MS (Methode D): Rₜ = 2.96 min., *m*/*z* = 305 (M+H)⁺ (freie Base).

### Beispiel 26

### N-[(3S)-1-Azabicyclo[2.2.2]oct-3-yl]-5-chlor-1-benzofuran-2-carboxamid-Hydrochlorid

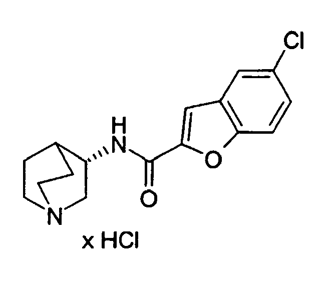

Die Versuchsdurchführung erfolgt in gleicher Weise und Dimension wie für die Verbindung aus Beispiel 25, jedoch unter Verwendung von S-3-Aminochinuklidin-Dihydrochlorid. Es werden 100 mg (30% d.Th.) der Titelverbindung isoliert. Die analytischen Daten stimmen mit denen der enantiomeren Verbindung aus Beispiel 25 überein.

### Beispiel 27

### N-[(3R)-1-Azabieyclo[2.2.2]oct-3-yl]-7-methoxy-5-nitro-1-benzofuran-2-carboxamid-Hydrochlorid

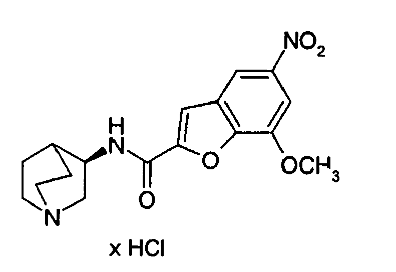

230 mg (0.98 mmol) 7-Methoxy-5-nitro-1-benzofuran-2-carbonsäure, 200 mg (0.98 mmol) *R*-3-Aminochinuklidin-Dihydrochlorid, 450 mg (1.18 mmol) HATU, 460 mg (3.54 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Anschließend wird das Produkt mit einem Überschuß 1 N Salzsäure versetzt. Das Solvens wird unter reduziertem Druck entfernt. Es werden 127 mg (34% d.Th.) der Titelverbindung isoliert.
¹H-NMR (200 MHz, DMSO-d₆): δ = 9.56 (s, 1H, br), 9.10 (d, 1H), 8.42 (d, 1H), 7.88 (d, 1H), 7.84 (s, 1H), 4.43-4.29 (m, 1 H), 4.10 (s, 3H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 346 (M+H)⁺ (freie Base)
LC-MS (Methode D): Rₜ = 3.16 min., *m*/*z* = 346 (M+H)⁺ (freie Base).

### Beispiel 28

### N-[(3S)-1-Azabicyclo[2.2.2]oct-3-yl]-7-methoxy-5-nitro-1-benzofuran-2-carboxamid-Hydrochlorid

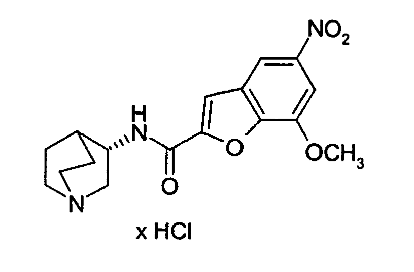

Die Versuchsdurchführung erfolgt in gleicher Weise und Dimension wie für die Verbindung aus Beispiel 27, jedoch unter Verwendung von S-3-Aminochinuklidin-Dihydrochlorid. Es werden 83 mg (20% d.Th.) der Titelverbindung isoliert. Die analytischen Daten stimmen mit denen der enantiomeren Verbindung aus Beispiel 27 überein.

### Beispiel 29

### 5-Amino-N-[(3R)-1-azabicyclo[2.2.2]oct-3-yl]-7-methoxy-1-benzofuran-2-carboxamid-Dihydrochlorid

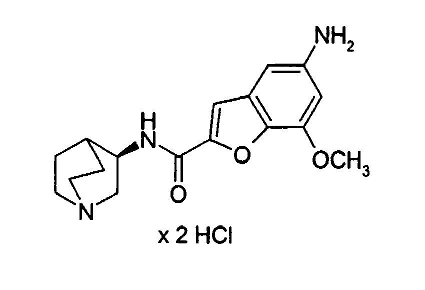

Zunächst wird N-[(3R)-1-Azabicyclo[2.2.2]oct-3-yl]-7-methoxy-5-nitro-1-benzofuran-2-carboxamid-Hydrochlorid (120 mg, 0.31 mmol) in die freie Base überführt. Dazu wird es in Essigsäureethylester aufgenommen und mit 30%-iger wässriger Natronlauge versetzt. Die wässrige Phase wird mehrfach mit Essigsäureethylester extrahiert. Es wird über Magnesiumsulfat getrocknet und das Solvens unter reduziertem Druck entfernt. Die freie Base wird in 3 ml Methanol gelöst. Es werden 120 mg (1.84 mmol) Ammoniumformiat zugegeben und 4 h unter Rückfluß erhitzt. Das Reaktionsgemisch wird über Celite filtriert und mit Ethanol und Essigsäureethylester nachgewaschen. Das Rohprodukt wird über Kieselgel 60 (Laufmittel: Dichlormethan → Dichlormethan/Methanol 10:1 → Dichlormethan/Methanol/Ammoniak 80:20:2) feingereinigt. Die freie Base wird mit einem Überschuß an 4 N HCl in Dioxan versetzt. Schließlich wird das Solvens unter reduziertem Druck entfernt. Es werden 25 mg (17% d.Th.) der Titelverbindung isoliert.
¹H-NMR (400 MHz, D₂O): δ = 7.62 (s, 1H), 7.40 (d, 1H), 7.07 (d, 1H), 4.56-4.50 (m, 1H), 4.09 (s, 3H), 3.90-3.80 (m, 1H), 3.55-3.30 (m, 5H), 2.50-2.40 (m, 1H), 2.35-2.21 (m, 1H), 2.20-2.05 (m, 2H), 2.06-1.93 (m, 1H) ppm.
MS (ESIpos): *m*/*z* = 316 (M+H)⁺ (freie Base)
LC-MS (Methode D): Rₜ = 0.43 min., *m*/*z* = 316 (M+H)⁺ (freie Base).

### Beispiel 30

### N-[(3R)-1-Azabicyclo[2.2.2]oct-3-yl]-5-nitro-1-benzofuran-2-carboxamid

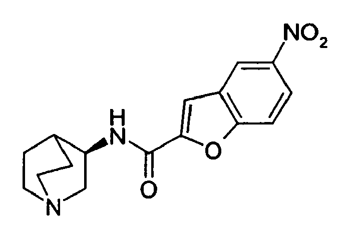

1248 mg (6.03 mmol) 5-Nitrobenzofuran-2-carbonsäure, 1000 mg (5.02 mmol) (R)-3-Aminochinuklidin-Dihydrochlorid, 2291 mg (6.03 mmol) HATU, 2250 mg (18.08 mmol) N,N-Diisopropylethylamin und 15 ml DMF werden gemäß der allgemeinen Arbeitsvorschrift (Variante B) umgesetzt. DMF wird unter reduziertem Druck entfernt. Das Reaktionsgemisch wird in Methanol aufgenommen und zusammen mit saurem Ionentauscherharz (Dowex WX2-200) etwa 40 min. lang gerührt. Der beladene Ionentauscher wird sechsmal mit je 100 ml Methanol gewaschen. Anschließend wird mit Methanol/Triethylamin 99:1 bis 90:10 eluiert. Das Solvens wird unter reduziertem Druck entfernt. Das Produkt wird in 1 N wässriger Natronlauge aufgenommen und dreimal mit Essigsäureethylester extrahiert. Es wird über Magnesiumsulfat getrocknet. Es werden 1100 mg (69% d.Th.) der Titelverbindung isoliert.
¹H-NMR (400 MHz, Methanol-d₄): δ = 8.67 (d, 1H), 8.36 (dd, 3H), 7.75 (d, 1H), 7.67 (s, 1H), 4.24-4.18 (m, 1H), 3.34-3.29 (m, 1H), 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H) ppm.
MS (ESIpos): *m*/*z* = 316 (M+H)⁺ (freie Base)
LC-MS (Methode D): Rₜ = 2.28 min., *m*/*z* = 316 (M+H)⁺ (freie Base).

### Beispiel 31

### N-[(3S)-1-Azabicyclo[2.2.2]oct-3-yl]-5-nitro-1-benzofuran-2-carboxamid

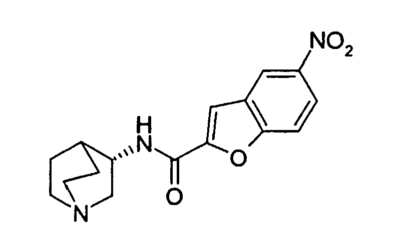

187 mg (0.90 mmol) 5-Nitrobenzofuran-2-carbonsäure, 150 mg (0.75 mmol) (S)-3-Aminochinuklidin-Dihydrochlorid, 343.7 mg (0.90 mmol) HATU, 350.5 mg (3.71 mmol) N,N-Diisopropylethylamin und 5 ml DMF werden gemäß der allgemeinen Arbeitsvorschrift (Variante B) umgesetzt. Es werden 169.1 mg (70% d.Th.) der Titelverbindung erhalten. Die analytischen Daten stimmen mit denen der enantiomeren Verbindung aus Beispiel 30 überein.

### Beispiel 32

### N-[(3R)-1-Azabicyclo[2.2.2]oct-3-yl]-5-amino-1-benzofuran-2-carboxamid

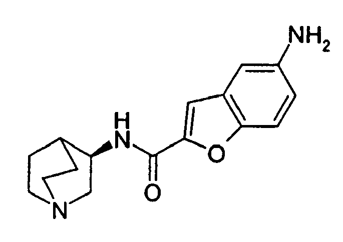

N-[(3R)-1-Azabicyclo[2.2.2]oct-3-yl]-5-nitro-1-benzofuran-2-carboxamid (100 mg, 0.32 mmol) wird mit 2.0 ml (4 mmol) einer 2 M Zinn(II)chlorid-Lösung in DMF versetzt. Es wird über Nacht gerührt. Das Reaktionsgemisch wird auf Wasser gegeben und mit 1 N wässriger Natronlauge basisch gestellt. Die wässrige Phase wird sechsmal mit Essigsäureethylester extrahiert. Die vereinigten organischen Phasen werden über Magnesiumsulfat getrocknet und das Solvens unter reduziertem Druck am Rotationsverdampfer enfernt. Das Rohprodukt wird in Methanol aufgenommen und zusammen mit saurem Ionentauscherharz (Dowex WX2-200) etwa 20 min. lang geschüttelt. Der beladene Ionentauscher wird dreimal mit je 30 ml Methanol, dann mit Wasser/ Methanol 8:2, wieder mit Methanol, mit Dichlormethan und schließlich wieder mit Methanol gewaschen. Das Produkt wird mit Methanol/Triethylamin 95:5 eluiert. Das Solvens wird unter reduziertem Druck am Rotationsverdampfer entfernt. Es werden 52 mg (58% d.Th.) der Titelverbindung isoliert.
¹H-NMR (400 MHz, Methanol-d₄): δ = 7.35 (d, 1H), 7.32 (s, 1H), 6.97 (d, 1H), 6.89 (dd, 3H), 4.24-4.18 (m, 1H), 3.34-3.29 (m, 1H), 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H) ppm.
MS (ESIpos): *m*/*z* = 286 (M+H)⁺ (freie Base)
LC-MS (Methode D): Rₜ = 2.02 min., *m*/*z* = 286 (M+H)⁺ (freie Base).

### Beispiel 33

### N-[(3S)-1-Azabicyclo[2.2.2]oct-3-yl]-5-amino-1-benzofuran-2-carboxamid

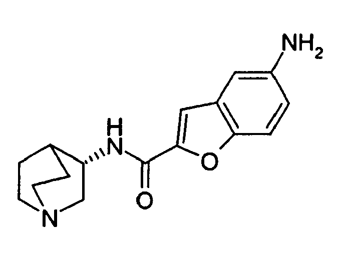

N-[(3S)-1-Azabicyclo[2.2.2]oct-3-yl]-5-nitro-1-benzofuran-2-carboxamid (135 mg, 0.32 mmol) wird mit 2.0 ml (4 mmol) einer 2 M Zinn(II)chlorid-Lösung in DMF versetzt und 18 h bei RT gerührt. Das Rohprodukt wird in Methanol aufgenommen und zusammen mit saurem Ionentauscherharz (Dowex WX2-200) etwa 20 min. lang geschüttelt. Der beladene Ionentauscher wird dreimal mit je 30 ml Methanol, dann mit Wasser/Methanol 8:2, wieder mit Methanol, mit Dichlormethan und schließlich wieder mit Methanol gewaschen. Das Produkt wird mit Methanol/Triethylamin 95:5 eluiert. Das Solvens wird unter reduziertem Druck am Rotationsverdampfer entfernt und das Rohgemisch mit 20 ml 1 N wässriger Natronlauge versetzt. Die wässrige Phase wird sechsmal mit je 20 ml Essigsäureethylester extrahiert. Die vereinigten organischen Phasen werden über Magnesiumsulfat getrocknet und unter reduziertem Druck am Rotationsverdampfer vom Solvens befreit. Es werden 100.7 mg (82% d.Th.) der Titelverbindung isoliert. Die analytischen Daten stimmen mit denen der enantiomeren Verbindung aus Beispiel 32 überein.

### Beispiel 34

### N-[(3R)-1-Azabicyclo[2.2.2]oct-3-yl]-6-nitro-1-benzofuran-2-carboxamid

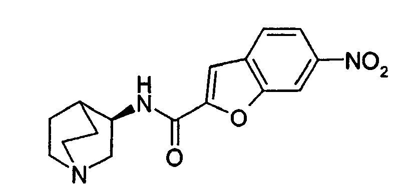

1040 mg (5.02 mmol) 6-Nitrobenzofuran-2-carbonsäure, 1000 mg (5.02 mmol) (R)-3-Aminochinuklidin-Dihydrochlorid, 2290 mg (6.03 mmol) HATU, 2250 mg (18.08 mmol) N,N-Diisopropylethylamin und 15 ml DMF werden gemäß der allgemeinen Arbeitsvorschrift (Variante B) umgesetzt. DMF wird unter reduziertem Druck entfernt. Das Produkt wird in 100 ml 1 N wässriger Natronlauge aufgenommen und dreimal mit je 50 ml Essigsäureethylester extrahiert. Die vereinigten organischen Phasen werden über Magnesiumsulfat getrocknet und unter reduziertem Druck am Rotationsverdampfer vom Solvens befreit. Das Reaktionsgemisch wird in Methanol aufgenommen und zusammen mit saurem Ionentauscherharz (Dowex WX2-200) etwa 40 min. lang gerührt. Der beladene Ionentauscher wird sechsmal mit je 100 ml Methanol gewaschen. Anschließend wird mit Methanol/Triethylamin 99:1 bis 90:10 eluiert. Das Solvens wird unter reduziertem Druck entfernt. Es wird über Magnesiumsulfat getrocknet. Es werden 1.75 g (99% d.Th.) der Titelverbindung isoliert.
¹H-NMR (200 MHz, CDCl₃): δ = 8.45 (s, 1H), 8.25 (dd, 1H), 7.80 (d, 1H), 7.56 (s, 1H), 7.05 (d, 1H), 4.35-4.18 (m, 1H), 3.1-2.82 (m, 5H), 2.20-2.10 (m, 1H), 1.98-1.53 (m, 5H) ppm.
MS (ESIpos): *m*/*z* = 316 (M+H)⁺ (freie Base)
HPLC: Rₜ = 3.6 min (Methode H)
LC-MS (Methode D): Rₜ = 2.62 min., *m*/*z* = 316 (M+H)⁺ (freie Base).

### Beispiel 35

### N-[(3R)-1-Azabicyclo[2.2.2]oct-3-yl]-6-amino-1-benzofuran-2-carboxamid

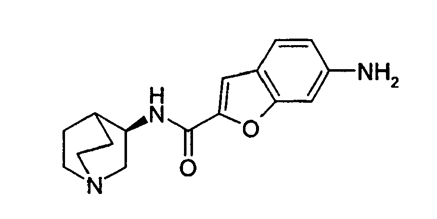

N-[(3R)-1-Azabicyclo[2.2.2]oct-3-yl]-6-nitro-1-benzofüran-2-carboxamid (1550 mg, 4.92 mmol) wird mit 15.0 ml (30 mmol) einer 2 M Zinn(II)chlorid-Lösung in DMF versetzt. Es wird über Nacht gerührt. Das Solvens wird unter reduziertem Druck am Rotationsverdampfer entfernt. Das Rohprodukt wird in Methanol aufgenommen und zusammen mit saurem Ionentauscherharz (Dowex WX2-200) etwa 1 h lang geschüttelt. Der beladene Ionentauscher wird mit Methanol, dann mit Wasser, DMF, wieder mit Methanol, mit Dichlormethan und schließlich wieder mit Methanol gewaschen. Das Produkt wird mit Methanol/Triethylamin 95:5 eluiert. Das Solvens wird unter reduziertem Druck am Rotationsverdampfer entfernt. Das Rohprodukt wird über Kieselgel 60 (Laufmittel: Dichlormethan/Triethylamin 100:1 → Dichlormethan/Methanol/Triethylamin 100:1:1 → Dichlormethan/Methanol/Triethylamin 90:10:1) gereinigt. Es werden 643 mg (46% d.Th.) der Titelverbindung isoliert.
MS (ESIpos): *m*/*z* = 286 (M+H)⁺ (freie Base)
HPLC: Rₜ = 2.6 min (Methode H)
LC-MS (Methode G): Rₜ = 1.62 min., *m*/*z* = 286 (M+H)⁺ (freie Base).

### Beispiel 36

### N-[(3R)-1-Azabicyclo[2.2.2]oct-3-yl]-7-nitro-1-benzofuran-2-carboxamid

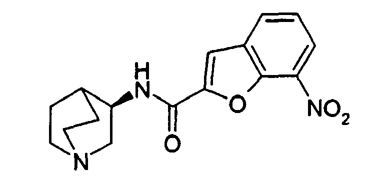

1040 mg (5.02 mmol) 6-Nitrobenzofuran-2-carbonsäure, 1000 mg (5.02 mmol) (R)-3-Aminochinuklidin-Dihydrochlorid, 2290 mg (6.03 mmol) HATU, 2250 mg (18.08 mmol) N,N-Diisopropylethylamin und 9 ml DMF werden gemäß der allgemeinen Arbeitsvorschrift (Variante B) umgesetzt. DMF wird unter reduziertem Druck entfernt. Das Produkt wird in 100 ml 1 N wässriger Natronlauge aufgenommen und dreimal mit je 50 ml Essigsäureethylester extrahiert. Die vereinigten organischen Phasen werden achtmal mit 1 N Natronlauge und einmal mit gesättiger Kochsalz-Lösung gewaschen, anschließend über Magnesiumsulfat getrocknet und unter reduziertem Druck am Rotationsverdampfer vom Solvens befreit. Es werden 1.34 g (79% d.Th.) der Titelverbindung erhalten.
¹H-NMR (400 MHz, Methanol-d₄): δ = 8.35 (d, 1H), 8.18 (d, 1H), 7.71 (s, 1H), 7.55 (dd, 1H), 4.25-4.18 (m, 1H), 3.40-3.31 (m, 1H), 3.10-2.97 (m, 1H), 2.93-2.77 (m, 4H), 2.13-2.05 (m, 1H), 2.03-1.92 (m, 1H), 1.84-1.75 (m, 2H), 1.63-1.53 (m, 1H) ppm.
MS (ESIpos): *m*/*z* = 316 (M+H)⁺ (freie Base)
HPLC: Rₜ = 3.55 min (Methode H)
LC-MS (Methode E): Rₜ = 3.15 min., *m*/*z* = 316 (M+H)⁺ (freie Base).

### Beispiel 37

### N-[(3R)-1-Azabicyclo[2.2.2]oct-3-yl]-7-amino-1-benzofuran-2-carboxamid

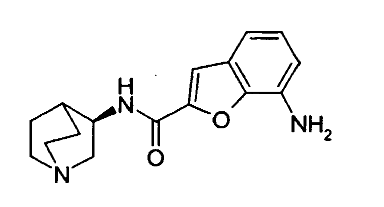

N-[(3R)-1-Azabicyclo[2.2.2]oct-3-yl]-7-nitro-1-benzofuran-2-carboxamid (1340 mg, 4.25 mmol) wird mit 6.0 ml (12 mmol) einer 2 M Zinn(II)chlorid-Lösung in DMF versetzt. Es wird über Nacht gerührt. Das Solvens wird unter reduziertem Druck am Rotationsverdampfer enfemt. Das Rohprodukt wird in Methanol aufgenommen und zusammen mit saurem Ionentauscherharz (Dowex WX2-200) etwa 1 h lang geschüttelt. Der beladene Ionentauscher wird mit Methanol, dann mit Wasser, wieder mit Methanol, mit DMF, wieder mit Methanol, mit THF, wieder mit Methanol, mit Dichlormethan und schließlich nochmals mit Methanol gewaschen. Das Produkt wird mit Methanol/Triethylamin 95:5 eluiert. Das Solvens wird unter reduziertem Druck am Rotationsverdampfer entfernt. Es werden 1200 mg (98% d.Th.) der Titelverbindung erhalten.
¹H-NMR (300 MHz, DMSO-d₆): δ = 8.18 (m, 1H), 7.40 (s, 1H), 6.98 (d, 1H), 6.87 (d, 1H), 6.65 (d, 1H), 5.45 (br. s, 1H), 4.06-3.94 (m, 1H), 3.29-3.15 (m, 1H), 3.04-2.88 (m, 1H), 2.85-2.65 (m, 4H), 1.98-1.77 (m, 2H), 1.72-1.54 (m, 2H), 1.48-1.32 (m, 1H) ppm.
MS (ESIpos): *m*/*z* = 286 (M+H)⁺ (freie Base)
HPLC: Rₜ = 2.95 min (Methode H)
LC-MS (Methode E): Rₜ = 3.03 min., *m*/*z* = 286 (M+H)⁺ (freie Base).

### Beispiel 38

### N-[(3R)-1-Azabicyclo[2.2.2]oct-3-yl]-5,7-difluor-1-benzofuran-2-carboxamid

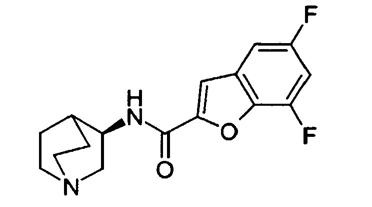

55 mg (0.28 mmol) 5,7-Difluor-1-benzofuran-2-carbonsäure, 50 mg (0.25 mmol) (*R*)-3-Aminochinuklidin-Dihydrochlorid, 114.6 mg (0.3 mmol) HATU, 117 mg (0.9 mmol) N,N-Diisopropylethylamin und 2.0 ml DMF werden gemäß der allgemeinen Arbeitsvorschrift (Variante B) umgesetzt. DMF wird unter reduziertem Druck entfernt und das Rohprodukt in 1 N wässriger Natronlauge gelöst. Die wässrige Phase wird mit Essigsäureethylester extrahiert. Die vereinigten organischen Phasen werden über Magnesiumsulfat getrocknet und das Solvens unter reduziertem Druck am Rotationsverdampfer entfernt. Das Rohprodukt wird in Methanol aufgenommen und zusammen mit saurem Ionentauscherharz (Dowex WX2-200) etwa 20 min. lang geschüttelt. Der beladene Ionentauscher wird dreimal mit je 30 ml Methanol, dann mit Wasser, wieder mit Methanol, mit Dichlormethan und schließlich wieder mit Methanol gewaschen. Das Produkt wird mit Methanol/Triethylamin 95:5 eluiert. Das Solvens wird unter reduziertem Druck am Rotationsverdampfer entfernt. Es werden 40 mg (52% d.Th.) der Titelverbindung isoliert.
¹H-NMR (400 MHz, Methanol-d₄): δ = 7.58-7.55 (d, 1H), 7.34-7.29 (m, 1H), 7.20-7.13 (m, 1H), 4.24-4.18 (m, 1H), 3.34-3.29 (m, 1H), 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H) ppm.
MS (ESIpos): *m*/*z* = 307 (M+H)⁺ (freie Base)
LC-MS (Methode D): Rₜ = 2.70 min., *m*/*z* = 307 (M+H)⁺ (freie Base).

### Beispiel 39

### N-[(3S)-1-Azabicyclo[2.2.2]oct-3-yl]-5,7-difluor-1-benzofuran-2-carboxamid

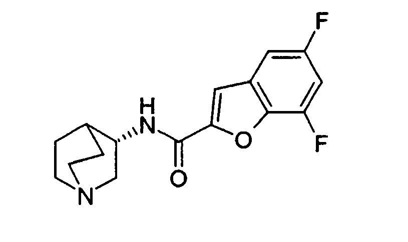

Die Versuchsdurchführung erfolgt in gleicher Weise und Dimension wie für die Verbindung aus Beispiel 38, jedoch unter Verwendung von *S*-3-Aminochinuklidin-Dihydrochlorid. Es werden 63 mg (82% d.Th.) der Titelverbindung isoliert. Die analytischen Daten stimmen mit denen der enantiomeren Verbindung aus Beispiel 38 überein.

### Beispiel 40

### N-[(3R)-1-Azabicyclo[2.2.2]oct-3-yl]-5-fluor-7-brom-1-benzofuran-2-carboxamid

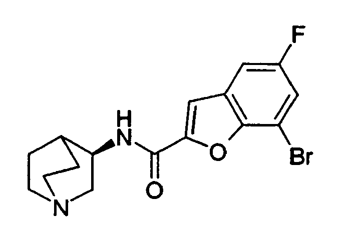

143 mg (0.55 mmol) 5-Fluor-7-brom-1-benzofuran-2-carbonsäure, 100 mg (0.50 mmol) (*R*)-3-Aminochinuklidin-Dihydrochlorid, 229.14 mg (0.6 mmol) HATU, 234 mg (1.81 mmol) N,N-Diisopropylethylamin und 2.0 ml DMF werden gemäß der allgemeinen Arbeitsvorschrift (Variante B) umgesetzt. DMF wird unter reduziertem Druck entfernt und das Rohprodukt in 1 N wässrige Natronlauge gelöst. Die wässrige Phase wird mit Essigsäureethylester extrahiert und mit gesättigter Kochsalz-Lösung gewaschen. Die vereinigten organischen Phasen werden über Magnesiumsulfat getrocknet und das Solvens unter reduziertem Druck am Rotationsverdampfer entfernt. Das Rohprodukt wird in Methanol aufgenommen und zusammen mit saurem Ionentauscherharz (Dowex WX2-200) etwa 20 min. lang geschüttelt. Der beladene Ionentauscher wird dreimal mit je 30 ml Methanol, dann mit Wasser, wieder mit Methanol, mit Dichlormethan und schließlich wieder mit Methanol gewaschen. Das Produkt wird mit Methanol/Triethylamin 95:5 eluiert. Das Solvens wird unter reduziertem Druck am Rotationsverdampfer entfernt. Es werden 181 mg (98% d.Th.) der Titelverbindung isoliert.
¹H-NMR (400 MHz, Methanol-d₄): δ = 7.59 (d, 1H), 7.53-7.46 (m, 2H), 4.24-4.18 (m, 1H), 3.34-3.29 (m, 1H), 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H) ppm.
MS (ESIpos): *m*/*z* = 367 (M+H)⁺ (freie Base)
LC-MS (Methode D): Rₜ = 2.92 min., *m*/*z* = 367 (M+H)⁺ (freie Base).

### Beispiel 41

### N-[(3S)-1-Azabicyclo[2.2.2]oct-3-yl]-5-fluor-7-brom-1-benzofuran-2-carboxamid

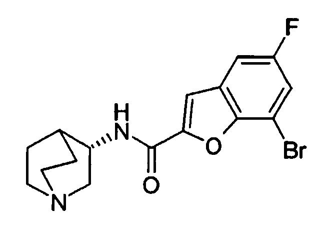

Die Versuchsdurchführung erfolgt in gleicher Weise und mit der halben Menge an Edukt und Reagenzien wie für die Verbindung aus Beispiel 40, jedoch unter Verwendung von *S*-3-Aminochinuklidin-Dihydrochlorid. Es werden 115 mg (47% d.Th.) der Titelverbindung isoliert. Die analytischen Daten stimmen mit denen der enantiomeren Verbindung aus Beispiel 40 überein.

### Beispiel 42

### N-[(3R)-1-Azabicyclo[2.2.2]oct-3-yl]-5-fluor-1-benzofuran-2-carboxamid-Hydrochlorid

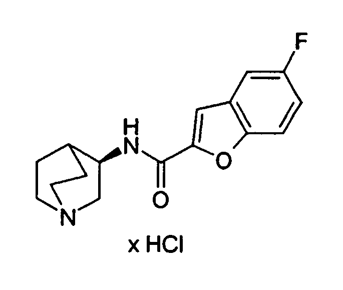

180 mg (1.00 mmol) 5-Fluor-1-benzofuran-2-carbonsäure, 200 mg (1.0 mmol) *R*-3-Aminochinuklidin-Dihydrochlorid, 460 mg (1.2 mmol) HATU, 470 mg (3.62 mmol) N,N-Diisopropylethylamin und 2.0 ml DMF werden gemäß der allgemeinen Arbeitsvorschrift (Variante B) umgesetzt. Das Reaktionsgemisch wird präparative HPLC gereinigt. Das so gereinigte Produkt wird mit 1 N wässriger Natronlauge versetzt und mit Essigsäureethylester ausgeschüttelt. Die vereinigten organischen Phasen werden über Natriumsulfat getrocknet und das Solvens unter reduziertem Druck entfernt. Das Produkt wird in Methanol gelöst und mit einem Überschuß an 4 N HCl in Dioxan in das Hydrochlorid überführt. Es werden 256 mg (79% d.Th.) der Titelverbindung isoliert.
¹H-NMR (200 MHz, DMSO-d₆): δ = 10.23 (s, 1H, br), 9.12 (d, 1H), 7.76-7.59 (m, 3H), 7.40-7.29 (m, 1H), 4.42-4.27 (m, 1H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 289 (M+H)⁺ (freie Base)
LC-MS (Methode D): Rₜ = 1.4 min., *m*/*z* = 289 (M+H)⁺ (freie Base).

### Beispiel 43

### N-[(3S)-1-Azabicyclo[2.2.2]oct-3-yl]-5-fluor-1-benzofuran-2-carboxamid-Hydrochlorid

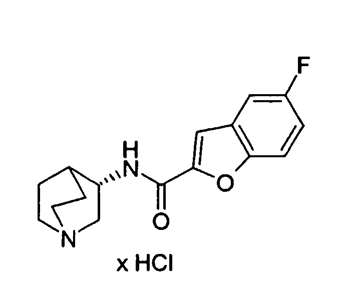

Die Versuchsdurchführung erfolgt in gleicher Weise und Dimension wie für die Verbindung aus Beispiel 42, jedoch unter Verwendung von *S*-3-Aminochinuklidin-Dihydrochlorid. Es werden 224 mg (69% d.Th.) der Titelverbindung isoliert. Die analytischen Daten stimmen mit denen der enantiomeren Verbindung aus Beispiel 42 überein.

### Beispiel 44

### N-[(3R)-1-Azabicyclo[2.2.2]oct-3-yl]-7-fluor-1-benzofuran-2-carboxamid-Hydrochlorid

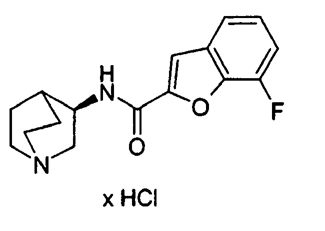

130 mg (0.73 mmol) 7-Fluorbenzofuran-2-carbonsäure, 150 mg (0.73 mmol) *R*-3-Aminochinuklidin-Dihydrochlorid, 330 mg (0.87 mmol) HATU, 340 mg (2.62 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Anschließend wird das Produkt mit einem Überschuß an 4 N HCl in Dioxan versetzt. Das Solvens wird unter reduziertem Druck entfernt. Es werden 116 mg (49% d.Th.) der Titelverbindung isoliert.
¹H-NMR (400 MHz, Methanol-d₄): δ = 7.67-7.53 (m, 1H), 7.40-7.22 (m, 2H), 4.54-4.46 (m, 1H), 3.92-3.79 (m, 1H), 3.53-3.29 (m, 1H), 3.53-3.29 (m, 4H), 2.46-2.39 (m, 1H), 2.31-2.20 (m, 1H), 2.17-2.07 (m, 2H), 2.06-1.92 (m, 1H) ppm.
MS (ESIpos): *m*/*z* = 289 (M+H)⁺ (freie Base)
LC-MS (Methode D): Rₜ = 2.50 min., *m*/*z* = 289 (M+H)⁺ (freie Base).

### Beispiel 45

### N-[(3S)-1-Azabicyclo[2.2.2]oct-3-yl]-7-fluor-1-benzofuran-2-carboxamid-Hydrochlorid

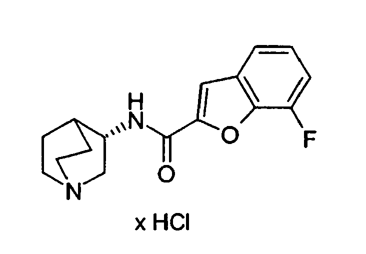

Die Versuchsdurchführung erfolgt in gleicher Weise und Dimension wie für die Verbindung aus Beispiel 44, jedoch unter Verwendung von *S*-3-Aminochinuklidin-Dihydrochlorid. Es werden 115 mg (47% d.Th.) der Titelverbindung isoliert. Die analytischen Daten stimmen mit denen der enantiomeren Verbindung aus Beispiel 44 überein.

### Beispiel 46

### N-[(3R)-1-Azabicyclo[2.2.2]oct-3-yl]-7-brom-1-benzofuran-2-carboxamid

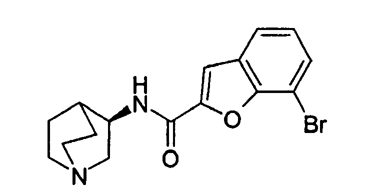

300 mg (1.25 mmol) 7-Brombenzofuran-2-carbonsäure, 248 mg (1.25 mmol) *R*-3-Aminochinuklidin-Dihydrochlorid, 568 mg (1.494 mmol) HATU, 579 mg (4.48 mmol) N,N-Diisopropylethylamin und 5 ml DMF werden gemäß der allgemeinen Arbeitsvorschrift (Variante B) umgesetzt. Das Rohprodukt wird in Methanol aufgenommen und zusammen mit saurem Ionentauscherharz (Dowex WX2-200) etwa 30 min. lang geschüttelt. Der beladene Ionentauscher wird mit DMF, Wasser, Methanol, Dichlormethan, Wasser, DMF und nochmals mit Methanol gewaschen. Das Produkt wird mit Methanol/Triethylamin 90:10 eluiert. Das Solvens wird unter reduziertem Druck am Rotationsverdampfer entfernt. Das vorgereinigte Produkt wird über präparative HPLC gereinigt. Es werden 320 mg (74% d.Th.) der Titelverbindung isoliert.
¹H-NMR (400 MHz, Methanol-d₄): 8.41 (s, 1H), 7.70 (d, 1H), 7.68-7.60 (m, 2H), 7.25 (t, 1H), 4.54-4.46 (m, 1H), 3.78-3.68 (m, 1H), 3.52-3.38 (m, 1H), 3.38-3.22 (m, 4H), 2.46-2.38 (m, 1H), 2.30-2.18 (m, 1H), 2.18-2.04 (m, 2H), 1.98-1.86 (m, 1H) ppm.
HPLC: Rₜ = 3.80 min (Methode H)
MS (ESIpos): *m*/*z* = 349 (M+H)⁺ (freie Base)
LC-MS (Methode G): Rₜ = 2.67 min., *m*/*z* = 349 (M+H)⁺ (freie Base).

### Beispiel 47

### N-[(3R)-1-Azabicyclo[2.2.2]oct-3-yl]-6-brom-1-benzofuran-2-carboxamid-Hydrochlorid

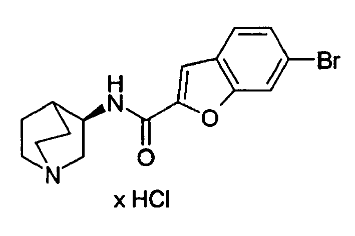

3.8 g (15.77 mmol) 6-Brombenzofuran-2-carbonsäure, 3.14 g (15.77 mmol) *R*-3-Aminochinuklidin-Dihydrochlorid, 7.19 g (18.92 mmol) HATU, 7.34 g (56.76 mmol) N,N-Diisopropylethylamin und 50 ml DMF werden gemäß der allgemeinen Arbeitsvorschrift (Variante B) umgesetzt. Das Rohprodukt wird in Methanol aufgenommen und zusammen mit saurem Ionentauscherharz (Dowex WX2-200) etwa 20 min. lang geschüttelt. Der beladene Ionentauscher wird sukzessive mit Methanol, Dichlormethan und erneut mit Methanol gewaschen. Das Produkt wird mit Methanol/ Triethylamin 90:10 eluiert. Das Solvens wird unter reduziertem Druck am Rotationsverdampfer entfernt. Schließlich werden letzte Lösungsmittelreste im Hochvakuum entfernt. Es werden 5.14 g (85% d.Th.) der Titelverbindung isoliert. Für die Analytik wird eine kleine Menge des Produkts mittels 4 N HCl in Dioxan in das Hydrochlorid überführt.
¹H-NMR (200 MHz, DMSO-d₆): δ = 10.55 (s, 1H, br), 9.22 (d, 1H), 8.05 (s, 1H), 7.75-7.55 (m, 3H), 4.43-4.29 (m, 1H), 3.70-3.55 (m, 1H), 3.45-3.10 (m, 5H), 2.25-2.00 (m, 2H), 1.98-1.82 (m, 2H), 1.80-1.60 (m, 1H) ppm.
HPLC: Rₜ = 3.9 min (Methode H)
MS (ESIpos): *m*/*z* = 349 (M+H)⁺ (freie Base)
LC-MS (Methode G): Rₜ = 1.49 min., *mlz* = 349 (M+H)⁺ (freie Base).

### Beispiel 48

### N-[(3R)-1-Azabicyclo[2.2.2]oct-3-yl]naphtho[1,2-b]furan-2-carboxamid-Hydrochlorid

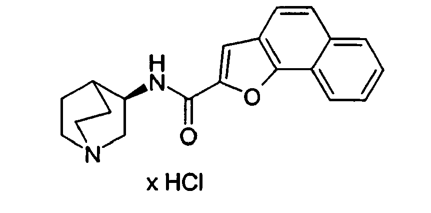

210 mg (0.98 mmol) Naphtho[1,2-b]furan-2-carbonsäure, 200 mg (0.98 mmol) *R*-3-Aminochinuklidin-Dihydrochlorid, 450 mg (1.18 mmol) HATU, 460 mg (3.54 mmol) N,N-Diisopropylethylamin und 2.0 ml DMF werden gemäß der allgemeinen Arbeitsvorschrift (Variante B) umgesetzt. Das Reaktionsgemisch durch präparative HPLC gereinigt. Anschließend wird das Produkt mit einem Überschuss an 1 N wässriger Salzsäure versetzt. Das Solvens wird unter reduziertem Druck entfernt. Es werden 74 mg (21% d.Th.) der Titelverbindung isoliert.
¹H-NMR (200 MHz, DMSO-d₆): δ = 9.60 (s, 1H, br), 9.04 (d, 1H), 8.38 (d, 1H), 8.31 (s, 1H), 8.13-8.00 (m, 2H), 7.84 (d, 1H), 7.74-7.56 (m, 2H), 4.43-4.29 (m, 1H), 3.70-3.55 (m, 1H), 3.45-3.10 (m, 5H), 2.25-2.00 (m, 2H), 1.98-1.82 (m, 2H), 1.80-1.60 (m, 1H) ppm.
MS (ESIpos): *m*/*z* = 321 (M+H)⁺ (freie Base)
LC-MS (Methode D): Rₜ = 3.10 min., *m*/*z* = 321 (M+H)⁺ (freie Base).

### Beispiel 49

### N-[(3S)-1-Azabicyclo[2.2.2]oct-3-yl]naphtho[1,2-b]furan-2-carboxamid-Hydrochlorid

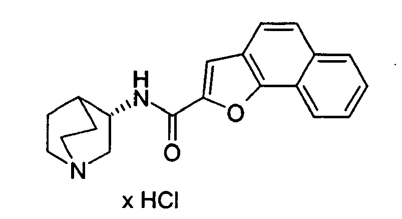

Die Versuchsdurchführung erfolgt in gleicher Weise und Dimension wie für die Verbindung aus Beispiel 48, jedoch unter Verwendung von *S*-3-Aminochinuklidin-Dihydrochlorid. Es werden 300 mg (85% d.Th.) der Titelverbindung isoliert. Die analytischen Daten stimmen mit denen der enantiomeren Verbindung aus Beispiel 48 überein.

### Beispiel 50

### N-[(3R)-1-Azabicyclo[2.2.2]oct-3-yl]-6-(2-cyclopropyl-2-oxoethyl)-1-benzofuran-2-carboxamid

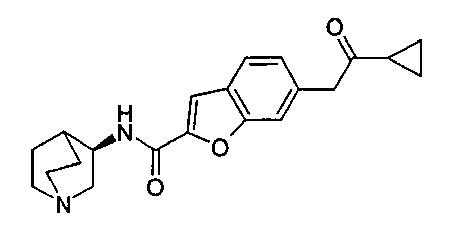

150 mg (0.43 mmol) N-[(3R)-1-Azabicyclo[2.2.2]oct-3-yl]-6-brom-1-benzofuran-2-carboxamid, 27 mg (0.04 mmol) rac-BINAP, 45 mg (0.47 mmol) Natrium-tert.-butylat und 20 mg (0.02 mmol) Tris(benzylidenaceton)dipalladium werden im Hochvakuum 1 h lang getrocknet. Unter Argonatmosphäre werden 2 ml Dioxan zugegeben. Es werden 72 mg (0.86 mmol) Methylcyclopropylketon zugetropft und über Nacht auf 85°C erhitzt. Das Rohprodukt wird durch präparative HPLC gereinigt. Das Solvens wird unter reduziertem Druck entfernt. Schließlich werden letzte Lösungsmittelreste im Hochvakuum entfernt. Es werden 75 mg (50% d.Th.) der Titelverbindung isoliert.
¹H-NMR (400 MHz, Methanol-d₄): δ = 7.62-7.49 (m, 3H), 7.35-7.30 (m, 1H), 4.54-4.45 (m, 1H), 4.04 (s, 2H), 3.87-3.77 (m, 1H), 3.53-3.24 (m, 5H), 2.42-2.31 (m, 1H), 2.30-2.19 (m, 1H), 2.20-2.04 (m, 3H), 1.98-1.88 (m, 1H), 1.01-0.87 (m, 4H) ppm.
HPLC: Rₜ = 3.7 min (Methode H)
MS (ESIpos): m/z = 353 (M+H)⁺.

### Beispiel 51

### N-[(3R)-1-Azabicyclo[2.2.2]oct-3-yl]-1-benzothiophen-2-carboxamid-Hydrochlorid

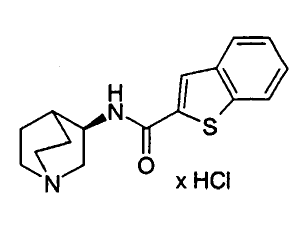

322.2 mg (1.81 mmol) Benzothiophen-2-carbonsäure, 300.0 mg (1.51 mmol) *R*-3-Aminochinuklidin-Dihydrochlorid, 687.4 mg (1.81 mmol) HATU, 701.1 mg (5.43 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem 1:1-Gemisch aus Methanol und 1 N wässriger Salzsäure gelöst und anschließend eingeengt. Man erhält 67 mg (13.8% d.Th.) der Titelverbindung. Die analytischen Daten stimmen mit denen des Racemats (Beispiel 13) überein.

### Beispiel 52

### N-[(3R)-1-Azabicyclo[2.2.2]oct-3-yl]-6-brom-1-benzothiophen-2-carboxamid-Hydrochlorid

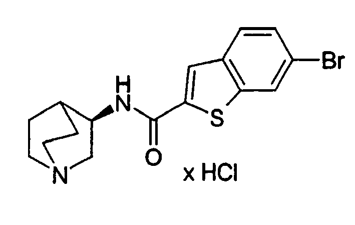

900.0 mg (3.50 mmol) 4-Brom-1-benzothiophen-2-carbonsäure, 697.0 mg (3.50 mmol) *R*-3-Aminochinuklidin-Dihydrochlorid, 1597.1 mg (4.20 mmol) HATU, 1628.7 mg (12.60 mmol) N,N-Diisopropylethylamin und 8.0 ml DMF werden gemäß der allgemeinen Arbeitsvorschrift (Variante B) umgesetzt. Das Reaktionsgemisch wird durch präparative HPLC gereinigt. Das Produkt wird in einem 1:1-Gemisch aus 4 M HCl in Dioxan und 1 N wässriger Salzsäure gelöst und anschließend eingeengt. Die Umkristallisation aus Methanol/Ethanol (1:10) ergibt 594 mg (42.1% d.Th.) der Titelverbindung.
¹H-NMR (300 MHz, DMSO-d₆): δ = 9.81 (s, 1H, br), 8.76 (m, 1H), 8.33 (s, 1H), 8.22 (s, 1H), 7.91 (d, 1H), 7.59 (dd, 1H), 4.15 (m, 1H), 3.51-2.93 (m, 6H), 2.12-1.92 (m, 2H), 1.79 (m, 2H), 1.58 (m, 1H) ppm.
HPLC: Rₜ = 4.1 min (Methode H)
MS (ESIpos): *m*/*z* = 366 (M, ⁸¹Br)⁺, 364 (M, ⁷⁹Br)⁺ (freie Base).

### Beispiel 53

### N-[(3R)-1-Azabicymo[2.2.2]oct-3-yl]thieno[2,3-f][1,3]benzodioxol-6-carboxamid-Hydrochlorid

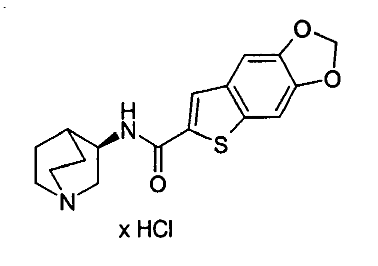

122.8 mg (ca. 0.55 mmol) eines 1:1-Gemisches aus Methyl thieno[2,3-f][1,3]benzodioxol-6-carboxylat und Thieno[2,3-f][1,3]benzodioxol-6-carbonsäure, 100 mg (0.50 mmol) *R*-3-Aminochinuklidin-Dihydrochlorid, 229.1 mg (0.60 mmol) HATU, 233.7 mg (1.81 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend eingeengt und im Hochvakuum getrocknet. Man erhält 29 mg (15.8% d.Th.) der Titelverbindung.
¹H-NMR (300 MHz, DMSO-d₆): δ = 10.18 (s, 1H, br), 8.87 (d, 1H), 8.13 (s, 1H), 7.56 (s, 1H), 7.42 (s, 1H), 6.12 (s, 2H), 4.29 (m, 1H), 3.90-3.55 (m, 2H), 3.43-3.12 (m, 5H), 2.20 (m, 1H), 2.12 (m, 1H), 1.91 (m, 2H), 1.75 (m, 1H) ppm.
HPLC: Rₜ = 3.7 min (Methode H)
MS (ESIpos): *m*/*z* = 331 (M+H)⁺ (freie Base).

### Beispiel 54

### N-[(3S)-1-Azabieyclo[2.2.2]oct-3-yl]thieno[2,3-f][1,3]benzodioxol-6-carboxamid-Hydrochlorid

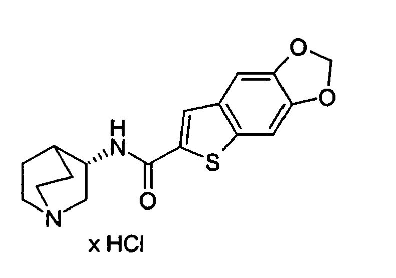

122.8 mg (ca. 0.55 mmol) eines 1:1-Gemisches aus Methyl thieno[2,3-f][1,3]benzodioxol-6-carboxylat und Thieno[2,3-f][1,3]benzodioxol-6-carbonsäure, 100 mg (0.50 mmol) *S*-3-Aminochinuklidin-Dihydrochlorid, 229.1 mg (0.60 mmol) HATU, 233.7 mg (1.81 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend eingeengt und im Hochvakuum getrocknet. Man erhält 46 mg (25% d.Th.) der Titelverbindung. Die analytischen Daten stimmen mit denen der enantiomeren Verbindung aus Beispiel 53 überein.

### Beispiel 55

### N-[(3R)-1-Azabicyclo[2.2.2]oct-3-yl]-5-nitro-1-benzothiophen-2-carboxamid-Hydrochlorid

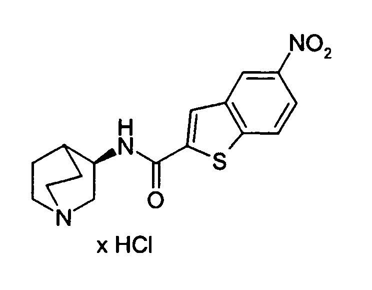

420 mg (1.88 mmol) 5-Nitro-1-benzothiophen-2-carbonsäure, 374.7 mg (1.88 mmol) *R*-3-Aminochinuklidin-Dihydrochlorid, 858.6 mg (2.26 mmol) HATU, 875.5 mg (6.78 mmol) N,N-Diisopropylethylamin und 2.0 ml DMF werden gemäß der allgemeinen Arbeitsvorschrift (Variante B) umgesetzt. Der entstandene Niederschlag wird in 1 N HCl in Diethylether suspendiert, abfiltriert, zweimal mit Dichlormethan gewaschen und im Hochvakuum getrocknet. Man erhält 523 mg (75.6% d.Th.) der Titelverbindung.
¹H-NMR (200 MHz, DMSO-d₆): δ = 10.45 (s, 1H, br), 9.43 (d, 1H), 8.37 (m, 1H), 8.60 (s, 1H), 8.30 (m, 2H), 4.35 (m, 1H), 3.65 (m, 1H), 3.40 (m, 2H), 3.23 (m, 3H), 2.24 (m, 1H), 2.17 (m, 1H), 1.93 (m, 2H), 1.76 (m, 1H) ppm.
MS (ESIpos): *m*/*z* = 332 (M+H)⁺ (freie Base).

### Beispiel 56

### N-[(3R)-1-Azabicyclo[2.2.2]oct-3-yl]-3-chlor-6-fluor-1-benzothiophen-2-carboxamid-Hydrochlorid

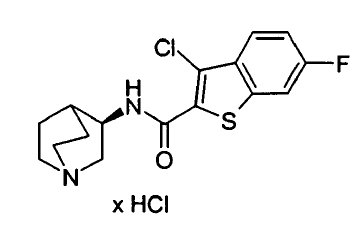

191.1 mg (0.83 mmol) 3-Chlor-6-fluor-1-benzothiophen-2-carbonsäure, 150.0 mg (0.75 mmol) *R*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in 4 M HCl in Dioxan gelöst, anschließend wieder eingeengt und im Hochvakuum getrocknet. Man erhält 223.3 mg (77.4% d.Th.) der Titelverbindung.
¹H-NMR (200 MHz, DMSO-d₆): δ = 9.67 (m, 1H), 8.82 (d, 1H), 8.11 (dd, 1H), 7.95 (dd, 1H), 7.50 (ddd, 1H), 4.33 (m, 1H), 3.80-3.08 (m, 6H), 2.27 (m, 1H), 2.13 (m, 1H), 2.00-1.68 (m, 3H) ppm.
HPLC: Rₜ = 3.9 min (Methode H)
MS (ESIpos): *m*/*z* = 339 (M+H)⁺ (freie Base).

### Beispiel 57

### N-[(3S)-1-Azabicyclo[2.2.2]oct-3-yl]-3-chlor-6-fluor-1-benzothiophen-2-carboxamid-Hydrochlorid

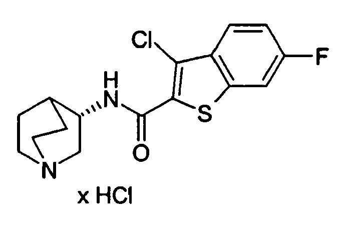

158 mg (0.69 mmol) 3-Chlor-6-fluor-1-benzothiophen-2-carbonsäure, 124 mg (0.62 mmol) *S*-3-Aminochinuklidin-Dihydrochlorid, 284.2 mg (0.75 mmol) HATU, 289.8 mg (2.24 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus 4 M HCl in Dioxan und Methanol gelöst und anschließend wieder eingeengt. Man erhält 190.5 mg (81.5% d.Th.) der Titelverbindung. Die analytischen Daten stimmen mit denen der enantiomeren Verbindung aus Beispiel 56 überein.

### Beispiel 58

### N-[(3R)-1-Azabicyclo[2.2.2]oct-3-yl]-3-fluor-1-benzothiophen-2-carboxamid-Hydrochlorid

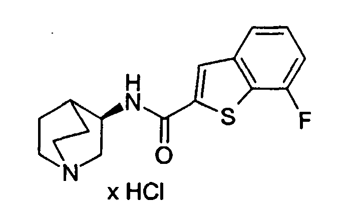

162.6 mg (0.83 mmol) 3-Fluor-l-benzothiophen-2-carbonsäure, 150.0 mg (0.75 mmol) *R*-3-Aminochinuklidin-Dihydrochlorid, 343.7 mg (0.90 mmol) HATU, 350.5 mg (2.71 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in 4 M HCl in Dioxan gelöst, anschließend wieder eingeengt und im Hochvakuum getrocknet. Man erhält 126.9 mg (48% d.Th.) der Titelverbindung.
¹H-NMR (200 MHz, DMSO-d₆): δ = 9.84 (s, 1H, br), 9.11 (d, 1H), 8.39 (d, 1H), 7.85 (d, 1H), 7.51 (m, 1H), 7.38 (m, 1H), 4.32 (m, 1H), 3.78-3.14 (m, 6H), 2.28-2.03 (m, 2H), 1.99-1.66 (m, 3H) ppm.
HPLC: Rₜ = 3.9 min (Methode H)
MS (ESIpos): *m*/*z* = 305 (M+H)⁺ (freie Base).

### Beispiel 59

### N-[(3S)-1-Azabicyclo[2.2.2]oct-3-yl]-7-fluor-1-benzothiophen-2-carboxamid-Hydrochlorid

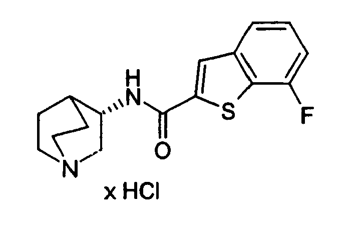

162.6 mg (0.83 mmol) 3-Fluor-1-benzothiophen-2-carbonsäure, 150.0 mg (0.75 mmol) *S*-3-Aminochinuklidin-Dihydrochlorid, 343.7 mg (0.90 mmol) HATU, 350.5 mg (2.71 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 162.8 mg (63% d.Th.) der Titelverbindung. Die analytischen Daten stimmen mit denen der enantiomeren Verbindung aus Beispiel 58 überein.

### Beispiel 60

### N-[(3R)-1-Azabicyclo[2.2.2]oct-3-yl]-3-chlor-1-benzothiophen-2-carboxamid-Hydrochlorid

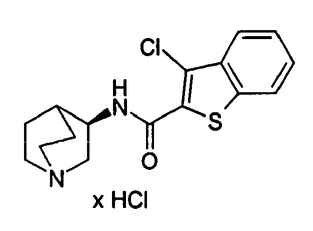

356.7 mg (1.68 mmol) 3-Chlor-1-benzothiophen-2-carbonsäure, 334 mg (1.68 mmol) *R*-3-Aminochinuklidin-Dihydrochlorid, 765.3 mg (2.01 mmol) HATU, 780.5 mg (6.04 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in 4 M HCl in Dioxan gelöst und anschließend wieder eingeengt. Man erhält 265 mg (44.2% d.Th.) der Titelverbindung. Die analytischen Daten stimmen mit denen des Racemats (Beispiel 14) überein.

### Beispiel 61

### N-[(3S)-1-Azabicyclo[2.2.2]oct-3-yl]-3-chlor-l-benzothiophen-2-carboxamid-Hydrochlorid

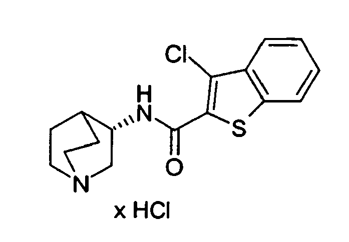

254.2 mg (1.20 mmol) 3-Chlor-1-benzothiophen-2-carbonsäure, 238 mg (1.20 mmol) *S*-3-Aminochinuklidin-Dihydrochlorid, 545.4 mg (1.43 mmol) HATU, 556.1 mg (4.30 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in 4 M HCl in Dioxan gelöst und anschließend wieder eingeengt. Man erhält 213 mg (49.9% d.Th.) der Titelverbindung. Die analytischen Daten stimmen mit denen des Racemats (Beispiel 14) überein.

### Beispiel 62

### N-[(3R)-1-Azabicyclo[2.2.2]oct-3-yl]-5-amino-1-benzothiophen-2-carboxamid-Dihydrochlorid

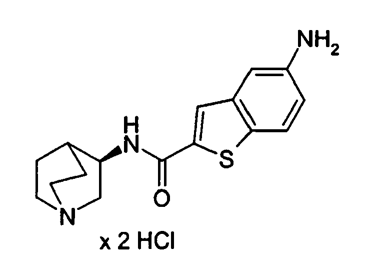

385.0 mg (1.05 mmol) N-[(3R)-1-Azabicyclo[2.2.2]oct-3-yl]-5-nitro-1-benzothiophen-2-carboxamid-Hydrochlorid werden in 10 ml eines 1:1-Gemisches aus Essigsäure und Wasser suspendiert. Nach der Zugabe von 300.0 mg (4.59 mmol) Zink wird 1 h bei RT gerührt. Das Reaktionsgemisch wird über Kieselgur filtriert und mit Methanol nachgewaschen. Das Filtrat wird im Vakuum eingeengt und der Rückstand mittels präparativer HPLC gereinigt. Die Produktfraktion wird in 4 M HCl in Dioxan gelöst, im Vakuum eingeengt und aus Acetonitril umkristallisiert. Man erhält 233 mg (59.5% d.Th.) der Titelverbindung.
¹H-NMR (400 MHz, D₂O): δ = 7.99 (d, 1H), 7.96 (s, 1H), 7.88 (d, 1H), 7.38 (dd, 1H), 4.36 (m, 1H), 3.76 (ddd, 1H), 3.43-3.19 (m, 5H), 2,32 (m, 1H), 2.16 (m, 1H), 2.01 (m, 2H), 1.90 (m, 1H) ppm.
HPLC: Rₜ = 2.9 min (Methode H)
MS (ESIpos): *m*/*z* = 302 (M+H)⁺ (freie Base).

### Beispiel 63

### N-[(3S)-1-Azabicyclo[2.2.2]oct-3-yl]-5-amino-1-benzothiophen-2-carboxamid-Dihydrochlorid

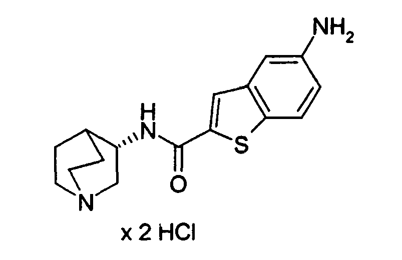

422 mg (1.15 mmol) N-[(3S)-1-Azabicyclo[2.2.2]oct-3-yl]-5-nitro-1-benzothiophen-2-carboxamid-Hydrochlorid werden in 10 ml eines 1:1-Gemisches aus Essigsäure und Wasser suspendiert. Nach der Zugabe von 300.0 mg (4.59 mmol) Zink wird 1 h bei RT gerührt. Das Reaktionsgemisch wird über Kieselgur filtriert und mit Methanol nachgewaschen. Das Filtrat wird im Vakuum eingeengt und der Rückstand mittels präparativer HPLC gereinigt. Die Produktfraktion wird in 4 M HCl in Dioxan gelöst, im Vakuum eingeengt und aus Acetonitril umkristallisiert. Man erhält 203 mg (47.3% d.Th.) der Titelverbindung. Die analytischen Daten stimmen mit denen der enantiomeren Verbindung aus Beispiel 62 überein.

### Beispiel 64

### N-[(3R)-1-Azabicyclo[2.2.2]oct-3-yl]-7-trifluormethyl-1-benzothiophen-2-carboxamid-Hydrochlorid

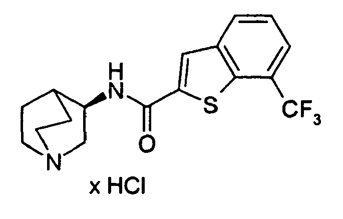

204.0 mg (0.83 mmol) 7-Trifluormethyl-1-benzothiophen-2-carbonsäure, 150.0 mg (0.75 mmol) *S*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift umgesetzt. Das Reaktionsgemisch wird durch präparative HPLC gereinigt. Das Produkt wird in 4 M HCl in Dioxan gelöst, anschließend wieder eingeengt und im Hochvakuum getrocknet. Man erhält 218.8 mg (74.3% d.Th.) der Titelverbindung.
¹H-NMR (400 MHz, DMSO-d₆): δ = 10.50 (s, 1H, br), 9.41 (d, 1H), 8.60 (s, 1H), 8.28 (d, 1H), 7.92 (d, 1H), 7.68 (dd, 1H), 4.36 (m, 1H), 3.64 (m, 1H), 3.43 (m, 2H), 3.20 (m, 3H), 2.24 (m, 1H), 2.17 (m, 1H), 1.92 (m, 2H), 1.74 (m, 1H) ppm.
HPLC: Rₜ = 4.1 min (Methode H)
MS (ESIpos): *m*/*z* = 355 (M+H)⁺ (freie Base).

### Beispiel 65

### N-[(3S)-1-Azabicyclo[2.2.2]oct-3-yl]-7-trifluormethyl-1-benzothiophen-2-carboxamid-Hydrochlorid

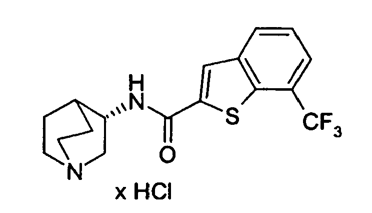

204.0 mg (0.83 mmol) 7-Trifluormethyl-1-benzothiophen-2-carbonsäure, 150.0 mg (0.75 mmol) *S*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in 4 M HCl in Dioxan gelöst, anschließend wieder eingeengt und im Hochvakuum getrocknet. Man erhält 158.6 mg (53.9% d.Th.) der Titelverbindung. Die analytischen Daten stimmen mit denen der enantiomeren Verbindung aus Beispiel 64 überein.

### Beispiel 66

### N-[(3R)-1-Azabicyclo[2.2.2]oct-3-yl]-5-trifluormethyl-1-benzothiophen-2-carboxamid-Hydrochlorid

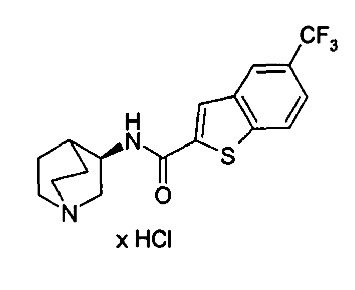

204.0 mg (0.83 mmol) 5-Trifluormethyl-1-benzothiophen-2-carbonsäure, 150.0 mg (0.75 mmol) *R*-3-Aminochinuklidin-Dihydrochlorid, 343.7 mg (0.90 mmol) HATU, 350.5 mg (2.71 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in 4 M HCl in Dioxan gelöst, anschließend wieder eingeengt und im Hochvakuum getrocknet. Man erhält 89 mg (29% d.Th.) der Titelverbindung.
¹H-NMR (400 MHz, D₂O + DMSO-d₆): δ = 8.36 (s, 1H), 8.19 (m, 2H), 7.80 (d, 1H), 4.46 (m, 1H), 3.85 (m, 1H), 3.76-3.52 (m, 1H), 3.50-3.25 (m, 4H), 2.40 (m, 1H), 2.22 (m, 1H), 2.10 (m, 2H), 1.96 (m, 1H) ppm.
HPLC: Rₜ = 4.1 min (Methode H)
MS (ESIpos): *m*/*z* = 355 (M+H)⁺ (freie Base).

### Beispiel 67

### 5-Amino-N-[(3R)-1-azabicyclo[2.2.2]oct-3-yl]-3-methyl-1-benzothiophen-2-carboxamid-Dihydrochlorid

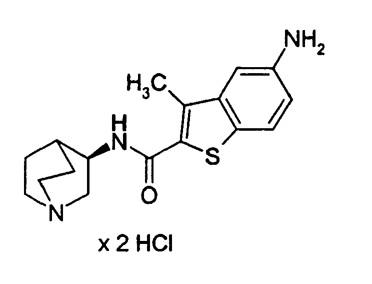

317 mg (0.83 mmol) N-[(3R)-1-Azabicyclo[2.2.2]oct-3-yl]-3-methyl-5-nitro-1-benzothiophen-2-carboxamid-Hydrochlorid werden in 2 ml Essigsäure suspendiert. Nach der Zugabe von 300.0 mg (4.59 mmol) Zink wird 1 h bei RT gerührt. Das Reaktionsgemisch wird über Kieselgur filtriert und mit Methanol nachgewaschen. Das Filtrat wird im Vakuum eingeengt und der Rückstand mittels präparativer HPLC gereinigt. Die Produktfraktion wird in 4 M HCl in Dioxan gelöst, im Vakuum eingeengt und aus Acetonitril umkristallisiert. Man erhält 154 mg (47.8% d.Th.) der Titelverbindung.
¹H-NMR (400 MHz, D₂O): δ = 8.10 (d, 1H), 7.91 (d, 1H), 7.53 (dd, 1H), 4.52 (m, 1H), 3.92 (m, 1H), 3.52-3.31 (m, 5H), 2.62 (s, 3H), 2,48 (m, 1H), 2.25 (m, 1H), 2.17 (m, 2H), 2.05 (m, 1H) ppm.
MS (ESIpos): *m*/*z* = 316 (M+H)⁺ (freie Base).

### Beispiel 68

### N-[(3R)-1-Azabicyclo[2.2.2]oct-3-yl]-7-amino-1-benzothiophen-2-carboxamid-Dihydrochlorid

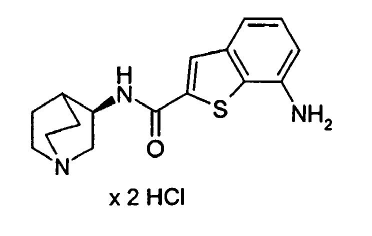

87 mg (0.22 mmol) N-[(3R)-1-Azabicyclo[2.2.2]oct-3-yl]-3-brom-1-benzothiophen-2-carboxamid-Hydrochlorid, 47.1 mg (0.26 mmol) Benzophenonimin, 12.1 mg (0.02 mmol) *rac*-BINAP, 45.8 mg (0.48 mmol) Natrium-tert.-butylat und 6.0 mg (0.01 mmol) Pd₂(dba)₃ werden unter Argon in einen ausgeheizten Kolben gegeben. 1.5 ml Toluol werden hinzugefügt und die Reaktionsmischung auf 80°C erhitzt. Nach 30 min. werden 0.5 ml THF und nach 6 h weitere 6.0 mg (0.01 mmol) Pd₂(dba)₃ hinzugefügt. Nach weiteren 6 h erfolgt eine Filtration (0.45 µm-Filter) und anschließende Reinigung mittels präparativer HPLC. Das entstandene Benzophenonimin-Addukt wird in einem 1:1-Gemisch aus THF und Methanol unter Zugabe von 20 Vol.-% 1 N Salzsäure gelöst. Nach 1 h bei RT wird das Reaktionsgemisch eingeengt. Der entstandene Feststoff wird mit Acetonitril verrührt und abfiltriert. Nach Trocknung im Hochvakuum werden 17 mg (21% d.Th.) der Titelverbindung erhalten.
¹H-NMR (400.1 MHz, D₂O): δ = 8.11 (s, 1H), 7.89 (d, 1H), 7.53 (dd, 1H), 7.37 (d, 1H), 4.48 (m, 1H), 3.87 (m, 1H), 3.52-3.30 (m, 5H), 2.44 (m, 1H), 2.27 (m, 1H), 2.12 (m, 2H), 2.00 (m, 1H) ppm.
HPLC: Rₜ = 2.9 min (Methode H)
MS (ESIpos): *m*/*z* = 302 (M+H)⁺ (freie Base).

### Beispiel 69

### N-[(3R)-1-Azabicyclo[2.2.2]oct-3-yl]-7-chlor-1-benzothiophen-2-carboxamid-Hydrochlorid

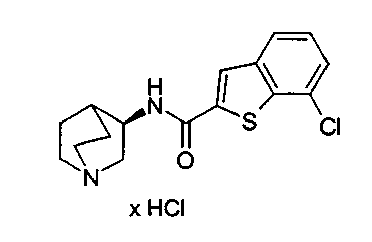

176.2 mg (0.83 mmol) 7-Chlor-1-benzothiophen-2-carbonsäure, 150 mg (0.75 mmol) *R*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus 4 M HCl in Dioxan und Methanol gelöst und anschließend eingeengt. Man erhält 175.2 mg (65.1% d.Th.) der Titelverbindung.
¹H-NMR (200 MHz, DMSO-d₆): δ = 10.03 (s, 1H, br), 9.17 (d, 1H), 8.43 (s, 1H), 7.98 (m, 1H), 7.63 (m, 1H), 7.52 (dd, 1H), 4.33 (m, 1H), 3.77-3.10 (m, 6H), 2.28-2.02 (m, 2H), 1.92 (m, 2H), 1.75 (m, 1H) ppm.
HPLC: Rₜ = 4.0 min (Methode H)
MS (ESIpos): *m*/*z* = 321 (M+H)⁺ (freie Base).

### Beispiel 70

### N-[(3S)-1-Azabicyclo[2.2.2]oct-3-yl]-7-chlor-1-benzothiophen-2-carboxamid-Hydrochlorid

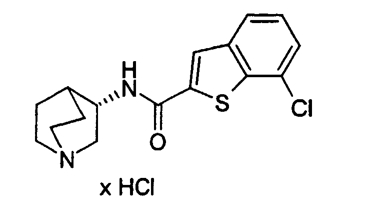

176.2 mg (0.83 mmol) 7-Chlor-1-benzothiophen-2-carbonsäure, 150 mg (0.75 mmol) *S*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus 4 M HCl in Dioxan und Methanol gelöst und anschließend eingeengt. Man erhält 231.9 mg (85.7% d.Th.) der Titelverbindung. Die analytischen Daten stimmen mit denen der enantiomeren Verbindung aus Beispiel 69 überein.

### Beispiel 71

### N-[(3R)-1-Azabicyclo[2.2.2]oct-3-yl]-5-(2-furoylamino)-1-benzothiophen-2-carboxamid-Hydrochlorid

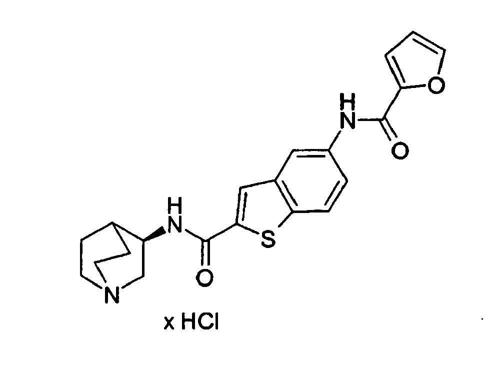

30 mg (0.08 mmol) N-[(3R)-1-A zabicyclo[2.2.2]oct-3-yl]-5-amino-1-benzothiophen-2-carboxamid-Dihydrochlorid werden in 1 ml DMF gelöst und mit 33.5 µl (0.24 mmol) Triethylamin versetzt. Bei 0°C werden 15.7 mg (0.12 mmol) Furan-2-carbonsäurechlorid hinzugefügt. Nach 3 h Rühren bei RT wird das Reaktionsgemisch mittels präparativer HPLC getrennt. Die Produktfraktion wird im Vakuum eingeengt und mit 4 M HCl in Dioxan codestilliert. Man erhält 12 mg (34.7% d.Th.) der Titelverbindung.
¹H-NMR (400 MHz, D₂O): δ = 7.70 (d, 1H), 7.67 (m, 1H), 7.63 (m, 1H), 7.55 (s, 1H), 7.28 (m, 1H), 7.14 (m, 1H), 6.60 (m, 1H), 4.24 (m, 1H), 3.70 (m, 1H), 3.51-3.21 (m, 5H), 2.28 (m, 1H), 2.20 (m, 1H), 2.10 (m, 2H), 2.00 (m, 1H) ppm.
HPLC: Rₜ = 3.6 min (Methode H)
LC-MS (Methode F): *m*/*z* = 396 (M+H)⁺ (freie Base), Rₜ = 2.62 min.

### Beispiel 72

### N-[(3R)-1-Azabicyclo[2.2.2]oct-3-yl]-5-(benzoylamino)-1-benzothiophen-2-carboxamid-Hydrochlorid.

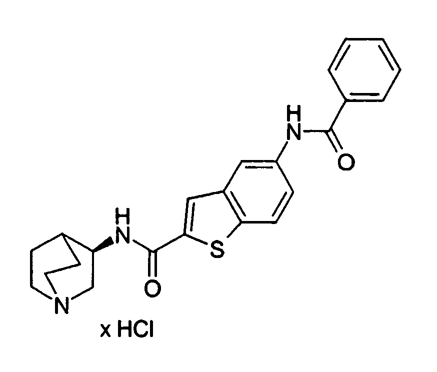

30 mg (0.08 mmol) N-[(3R)-1-Azabicyclo[2.2.2]oct-3-yl]-5-amino-1-benzothiophen-2-carboxamid-Dihydrochlorid werden in 1 ml DMF gelöst und mit 33.5 µl (0.24 mmol) Triethylamin versetzt. Bei 0°C werden 16.9 mg (0.12 mmol) Benzoylchlorid hinzugefügt. Nach 3 h Rühren bei RT wird das Reaktionsgemisch mittels präparativer HPLC getrennt. Die Produktfraktion wird im Vakuum eingeengt und mit 4 M HCl in Dioxan codestilliert. Man erhält 9 mg (25.4% d.Th.) der Titelverbindung.
LC-MS (Methode F): *m*/*z* = 406 (M+H)⁺ (freie Base), Rₜ = 2.82 min.

### Beispiel 73

### 6-Amino-N-[(3R)-1-azabicyclo[2.2.2]oct-3-yl]-1-benzothiophen-2-carboxamid-Dihydrochlorid

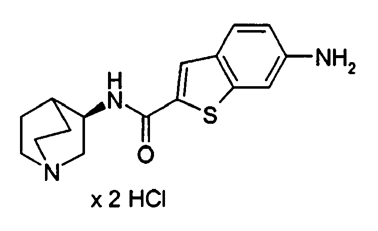

### Methode A):

15 mg (0.05 mmol) 6-[(tert.-Butoxycarbonyl)amino]-1-benzothiophen-2-carbonsäure, 10.2 mg (0.05 mmol) R-3-Aminochinuklidin-Dihydrochlorid, 21.4 mg (0.06 mmol) HATU, 21.8 mg (0.17 mmol) N,N-Diisopropylethylamin und 1 ml DMF werden gemäß der allgemeinen Arbeitsvorschrift (Variante B) umgesetzt. Das Reaktionsgemisch wird durch präparative HPLC gereinigt. Das Produkt wird mit 5 ml 4 M HCl in Dioxan versetzt und 30 min. bei RT gerührt. Es wird eingeengt und im Hochvakuum getrocknet. Man erhält 17 mg (98% d.Th.) der Titelverbindung.

### Methode B):

247 mg (0.67 mmol) N-[(3R)-1-Azabicyclo[2.2.2]oct-3-yl]-6-nitro-1-benzothiophen-2-carboxamid-Hydrochlorid werden in 1.6 ml 1 N Salzsäure und 4.3 ml Methanol suspendiert und unter Argon mit 25.6 mg Palladium auf Kohle (5%-ig) versetzt. Unter einer Wasserstoffatmosphäre (Normaldruck) wird für 2 h gerührt. Der Kolbeninhalt wird über Kieselgur filtriert und das Filtrat im Vakuum zur Trockene eingeengt. Man erhält 241 mg (95.6% d.Th.) der Titelverbindung.

### Methode C):

730 mg (1.76 mmol) N-[(3R)-1-Azabicyclo[2.2.2]oct-3-yl]-6-brom-1-benzothiophen-2-carboxamid-Hydrochlorid, 638.9 mg (3.53 mmol) Benzophenonimin, 109.8 mg (0.18 mmol) *rac*-BINAP, 508.2 mg (5.29 mmol) Natrium-tert.-butylat und 161.4 mg (0.18 mmol) Pd₂(dba)₃ werden unter Argon in einen ausgeheizten Kolben gegeben. 10 ml einer 1:1-Mischung aus THF und Toluol werden hinzugefügt und die Reaktionsmischung über Nacht auf 85°C erhitzt. Der Kolbeninhalt wird auf ca. 7 ml eingeengt und mittels präparativer HPLC aufgereinigt. Das entstandene Benzophenonimin-Addukt wird in 5 ml Methanol und 3 ml 1 N wässriger Salzsäure gelöst und für 1 h bei RT gerührt. Nach dem Einengen der Lösung erfolgt eine Umkristallisation aus Methanol/Diethylether sowie eine weitere Reinigung mittels präparativer HPLC. Man versetzt die Produktfraktionen mit 1 N wässriger Salzsäure. Nach Einengen und Trocknung im Hochvakuum werden 67 mg (10.1% d.Th.) der Titelverbindung erhalten.
¹H-NMR (400 MHz, D₂O): δ = 7.95 (m, 2H), 7.88 (m, 1H), 7.32 (m, 1H), 4.37 (m, 1H), 3.80-3.69 (m, 2H), 3.40-3.18 (m, 4H), 2.32 (m, 1H), 2.16 (m, 1H), 2.00 (m, 2H), 1.89 (m, 1H) ppm.
HPLC: Rₜ = 2.7 min (Methode H)
MS (ESIpos): *m*/*z* = 302 (M+H)⁺ (freie Base).

### Beispiel 74

### N-[(3R)-1-Azabicyclo[2.2.2]oct-3-yl]-5-fluor-1-benzothiophen-2-carboxamid-Hydrochlorid

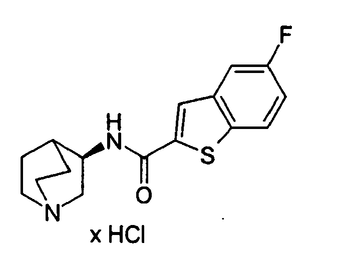

162.6 mg (0.83 mmol) 5-Fluor-1-benzothiophen-2-carbonsäure, 150.0 mg (0.75 mmol) *R*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 144.0 mg (56.1 % d.Th.) der Titelverbindung.
¹H-NMR (300 MHz, DMSO-d₆): δ = 10.01 (s, 1H, br), 9.03 (d, 1H), 8.27 (s, 1H), 8.08 (dd, 1H), 7.81 (dd, 1H), 7.38 (ddd, 1H), 4.32 (m, 1H), 3.67 (m, 1H), 3.43-3.15 (m, 5H), 2.24 (m, 1H), 2.13 (m, 1H), 1.93 (m, 2H), 1.77 (m, 1H) ppm.
HPLC: Rₜ = 3.8 min (Methode H)
MS (ESIpos): *m*/*z* = 305 (M+H)⁺ (freie Base).

### Beispiel 75

### N-[(3S)-1-Azabicyclo[2.2.2]oct-3-yl]-5-fluor-1-benzothiophen-2-carboxamid-Hydrochlorid

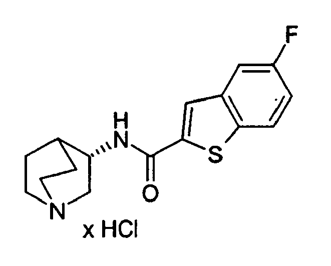

162.6 mg (0.83 mmol) 5-Fluor-1-benzothiophen-2-carbonsäure, 150 mg (0.75 mmol) *S*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus 4 M HCl in Dioxan und Methanol gelöst und anschließend wieder eingeengt. Man erhält 127.6 mg (49.7% d.Th.) der Titelverbindung. Die analytischen Daten stimmen mit denen der enantiomeren Verbindung aus Beispiel 74 überein.

### Beispiel 76

### N-[(3R)-1-Azabicyclo[2.2.2]oct-3-yl]-7-methoxy-1-benzothiophen-2-carboxamid-Hydrochlorid

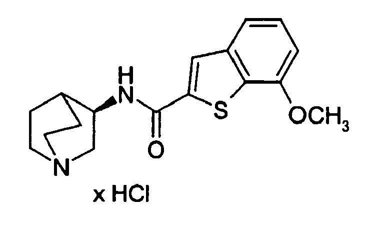

115 mg (0.55 mmol) 7-Methoxy-1-benzothiophen-2-carbonsäure, 100 mg (0.50mmol) *R*-3-Aminochinuklidin-Dihydrochlorid, 229.1 mg (0.60 mmol) HATU, 233.7 mg (1.81 mmol) N.N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 176.9 mg (95.6% d.Th.) der Titelverbindung.
¹H-NMR (200 MHz, DMSO-d₆): δ = 9.45 (m, 1H), 8.86 (d, 1H), 8.20 (s, 1H), 7.77 (d, 1H), 7.43 (dd, 1H), 7.06 (d, 1H), 4.31 (m, 1H), 3.98 (s, 3H), 3.80-3.10 (m, 6H), 2.21 (m, 1H), 2.13 (m, 1H), 1.93 (m, 2H), 1.78 (m, 1H) ppm.
HPLC: Rₜ = 3.8 min (Methode H)
MS (ESIpos): *m*/*z* = 317 (M+H)⁺ (freie Base).

### Beispiel 77

### N-[(3S)-1-Azabicyclo[2.2.2]oct-3-yl]-7-methoxy-1-benzothiophen-2-carboxamid-Hydrochlorid

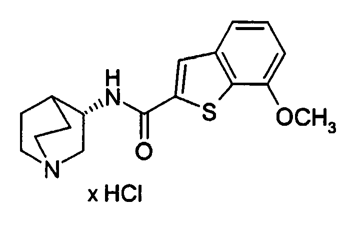

103.5 mg (0.50 mmol) 7-Methoxy-1-benzothiophen-2-carbonsäure, 90 mg (0.45 mmol) *S*-3-Aminochinuklidin-Dihydrochlorid, 206.2 mg (0.54 mmol) HATU, 210.3 mg (1.62 mmol) N,N-Diisopropylethylamin und 2.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 105.6 mg (66.2% d.Th.) der Titelverbindung. Die analytischen Daten stimmen mit denen der enantiomeren Verbindung aus Beispiel 76 überein.

### Beispiel 78

### N-[(3R)-1-Azabicyclo[2.2.2]oct-3-yl]-4-fluor-1-benzothiophen-2-carboxamid-Hydrochlorid

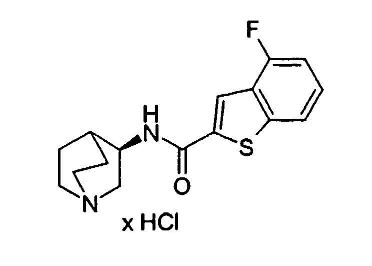

197.1 mg (1.00 mmol) 4-Fluor-1-benzothiophen-2-carbonsäure, 125.0 mg (0.63 mmol) *R*-3-Aminochinuklidin-Dihydrochlorid, 286.4 mg (0.75 mmol) HATU, 292.1 mg (2.26 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 160.1 mg (73.3% d.Th.) der Titelverbindung.
HPLC: Rₜ = 3.8 min (Methode H)
MS (ESIpos): *m*/*z* = 305 (M+H)⁺ (freie Base).

### Beispiel 79

### N-[(3S)-1-Azabicyclo[2.2.2]oct-3-yl]-4-fluor-1-benzothiophen-2-carboxamid-Hydrochlorid

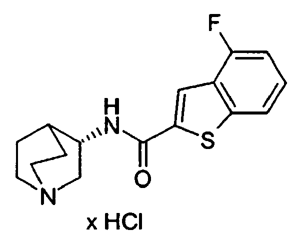

197.1 mg (1.00 mmol) 4-Fluor-1-benzothiophen-2-carbonsäure, 125.0 mg (0.63 mmol) S-3-Aminochinuklidin-Dihydrochlorid, 286.4 mg (0.75 mmol) HATU, 292.1 mg (2.26 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 85.7 mg (40.1% d.Th.) der Titelverbindung. Die analytischen Daten stimmen mit denen der enantiomeren Verbindung aus Beispiel 78 überein.

### Beispiel 80

### N-[(3R)-1-Azabicyclo[2.2.2]oct-3-yl]-5,7-difluor-1-benzothiophen-2-carboxamid-Hydrochlorid

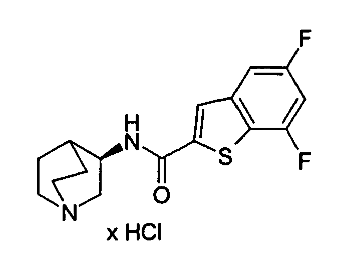

177.5 mg (0.83 mmol) 5,7-Difluor-1-benzothiophen-2-carbonsäure, 150.0 mg (0.75 mmol) *R*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 110.4 mg (40.8% d.Th.) der Titelverbindung.
¹H-NMR (200 MHz, DMSO-d₆): δ = 10.14 (s, 1H, br), 9.26 (d, 1H), 8.40 (d, 1H), 7.78 (dd, 1H), 7.52 (ddd, 1H), 4.31 (m, 1H), 3.65 (m, 1H), 3.50-3.07 (m, 5H), 2.22 (m, 1H), 2.14 (m, 1H), 1.90 (m, 2H), 1.75 (m, 1H) ppm.
HPLC: Rₜ = 3.9 min (Methode H)
MS (ESIpos): *m*/*z* = 323 (M+H)⁺ (freie Base).

### Beispiel 81

### N-[(3S)-1-Azabicyclo[2.2.2]oct-3-yl]-5,7-difluor-1-benzothiophen-2-carboxamid-Hydrochlorid

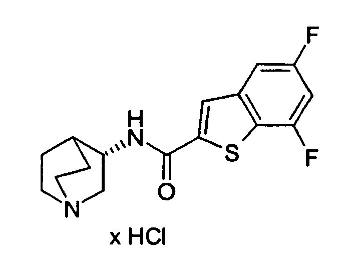

177.5 mg (0.83 mmol) 5,7-Difluor-1-benzothiophen-2-carbonsäure, 150.0 mg (0.75 mmol) *S*-3-Aminochinuklidin-Dihydrochlorid, 343.7 mg (0.90 mmol) HATU, 350.5 mg (2.71 mmol) N,N-Diisopropylethylamin und 3.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 109.3 mg (40.4% d.Th.) der Titelverbindung. Die analytischen Daten stimmen mit denen der enantiomeren Verbindung aus Beispiel 80 überein.

### Beispiel 82

### N-[(3R)-1-Azabicyclo[2.2.2]oct-3-yl]-6-methoxy-1-benzothiophen-2-carboxamid-Hydrochlorid

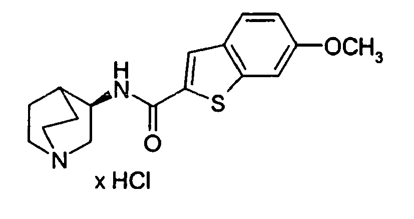

100 mg (0.48 mmol) 6-Methoxy-l-benzothiophen-2-carbonsäure, 86.9 mg (0.44 mmol) *R*-3-Aminochinuklidin-Dihydrochlorid, 199.2 mg (0.52 mmol) HATU, 203.13 mg (1.57 mmol) N,N-Diisopropylethylamin und 1.5 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 118.2 mg (76.7% d.Th.) der Titelverbindung.
¹H-NMR (200 MHz, DMSO-d₆): δ = 9.77 (s, 1H, br), 8.83 (d, 1H), 8.17 (s, 1H), 7.85 (d, 1H), 7.60 (d, 1H), 7.07 (dd, 1H), 4.30 (m, 1H), 3.84 (s, 3H), 3.79-3.45 (m, 2H), 3.39-3.10 (m, 4H), 2.20 (m, 1H), 2.10 (m, 1H), 1.90 (m, 2H), 1.75 (m, 1H) ppm.
HPLC: Rₜ = 3.8 min (Methode H)
MS (ESIpos): *m*/*z* = 317 (M+H)⁺ (freie Base).

### Beispiel 83

### N-[(3S)-1-Azabicyclo[2.2.2]oct-3-yl]-6-methoxy-1-benzothiophen-2-carboxamid-Hydrochlorid

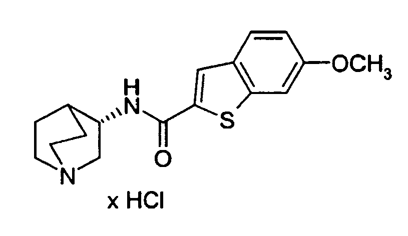

100 mg (0.48 mmol) 6-Methoxy-1-benzothiophen-2-carbonsäure, 86.9 mg (0.44 mmol) *S*-3-Aminochinuklidin-Dihydrochlorid, 199.2 mg (0.52 mmol) HATU, 203.13 mg (1.57 mmol) N,N-Diisopropylethylamin und 1.5 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 112.5 mg (73% d.Th.) der Titelverbindung. Die analytischen Daten stimmen mit denen der enantiomeren Verbindung aus Beispiel 82 überein.

### Beispiel 84

### N-[(3R)-1-Azabicyclo[2.2.2]oct-3-yl]-6-cyano-1-benzothiophen-2-carboxamid-Hydrochlorid

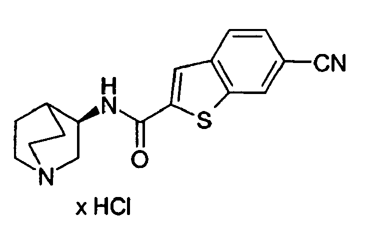

320.8 mg (1.1 mmol) 6-Cyano-1-benzothiophen-2-carbonsäure, 200 mg (1.0 mmol) *R*-3-Aminochinuklidin-Dihydrochlorid, 458.3 mg (1.21 mmol) HATU, 467.3 mg (3.62 mmol) N,N-Diisopropylethylamin und 4.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 222.1 mg (63.6% d.Th.) der Titelverbindung.
¹H-NMR (300 MHz, DMSO-d₆): δ = 9.80 (m, 1H), 9.12 (d, 1H), 8.68 (s, 1H), 8.37 (s, 1H), 8.16 (d, 1H), 7.83 (dd, 1H), 4.33 (m, 1H), 3.76-3.05 (m, 6H), 2.23 (m, 1H), 2.13 (m, 1H), 1.92 (m, 2H), 1.76 (m, 1H) ppm.
HPLC: Rₜ = 3.6 min (Methode H)
MS (ESIpos): *m*/*z* = 312 (M+H)⁺ (freie Base).

### Beispiel 85

### N-[(3S)-1-Azabieyclo[2.2.2]oct-3-yl]-6-cyano-1-benzothiophen-2-carboxamid-Hydrochlorid

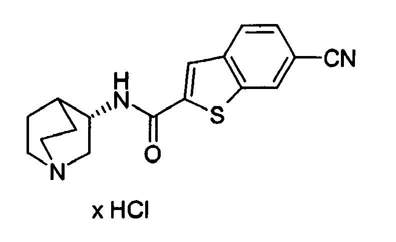

112.2 mg (0.39 mmol) 6-Cyano-l-benzothiophen-2-carbonsäure, 70 mg (0.35 mmol) *S*-3-Aminochinuklidin-Dihydrochlorid, 160.4 mg (0.42 mmol) HATU, 163.5 mg (1.26 mmol) N,N-Diisopropylethylamin und 1.5 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 250.1 mg (41% d.Th.) der Titelverbindung. Die analytischen Daten stimmen mit denen der enantiomeren Verbindung aus Beispiel 84 überein.

### Beispiel 86

### N-[(3R)-1-Azabicyclo[2.2.2]oct-3-yl]-4-nitro-1-benzothiophen-2-carboxamid-Hydrochlorid

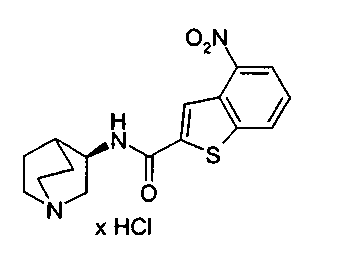

246.6 mg (1.10 mmol) 4-Nitro-1-benzothiophen-2-carbonsäure, 200 mg (1.00 mmol) *R*-3-Aminochinuklidin-Dihydrochlorid, 458.3 mg (1.21 mmol) HATU, 467.4 mg (3.62 mmol) N,N-Diisopropylethylamin und 4.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Man erhält 134.3 mg (35.6% d.Th.) der Titelverbindung.
¹H-NMR (200 MHz, DMSO-d₆): δ = 9.70 (s, 1H, br), 9.23 (d, 1H), 8.49 (s, 1H), 8.57 (d, 1H), 8.43 (dd, 1H), 7.73 (dd, 1H), 4.32 (m, 1H), 3.82-3.10 (m, 6H), 2.27 (m, 1H), 2.13 (m, 1H), 1.93 (m, 2H), 1.77 (m, 1H) ppm.
HPLC: Rₜ = 3.8 min (Methode H)
MS (ESIpos): *m*/*z* = 332 (M+H)⁺ (freie Base).

### Beispiel 87

### N-[(3S)-1-Azabicyclo[2.2.2]oct-3-yl]-4-nitro-1-benzothiophen-2-carboxamid-Hydrochlorid

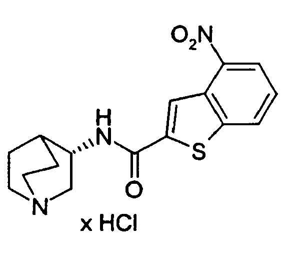

246.6 mg (1.10 mmol) 4-Nitro-1-benzothiophen-2-carbonsäure, 200 mg (1.00 mmol) *S*-3-Aminochinuklidin-Dihydrochlorid, 458.3 mg (1.21 mmol) HATU, 467.4 mg (3.62 mmol) N,N-Diisopropylethylamin und 4.0 ml DMF werden gemäß der allgemeinen Arbeitsvorschrift (Variante B) umgesetzt. Das Reaktionsgemisch wird durch präparative HPLC gereinigt. Das Produkt wird in einem Gemisch aus Methanol und 4 M HCl in Dioxan gelöst, anschließend wieder eingeengt und im Hochvakuum getrocknet. Nach Umkristallisation aus Methanol erhält man 128.5 mg (34.8% d.Th.) der Titelverbindung. Die analytischen Daten stimmen mit denen der enantiomeren Verbindung aus Beispiel 86 überein.

### Beispiel 88

### 6-(Acetylamino)-N-[(3R)-1-azabicyclo[2.2.2]oct-3-yl]-1-benzothiophen-2-carboxamid-Hydrochlorid

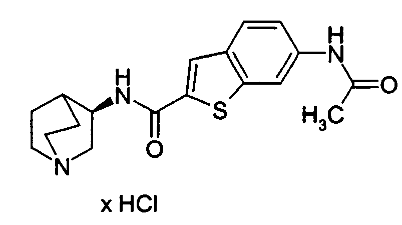

50 mg (0.12 mmol) 6-Amino-N-[(3R)-1-azabicyclo[2.2.2]oct-3-yl]-1-benzothiophen-2-carboxamid-Dihydrochlorid werden in 0.3 ml DMF vorgelegt und mit 18.7 mg (0.18 mmol) Triethylamin sowie 11.6 mg (0.15 mmol) Acetylchlorid versetzt. Nach 16 h Rühren bei RT werden weitere 18.7 mg (0.18 mmol) Triethylamin sowie 11.6 mg (0.15 mmol) Acetylchlorid hinzugefügt. Nach weiteren 12 h Rühren bei RT wird die überstehende Lösung des Reaktionsgemisches mittels präparativer HPLC gereinigt. Die Produktfraktionen werden eingeengt, der Rückstand in einem Gemisch aus Methanol und 4 M HCl in Dioxan gelöst, anschließend wieder eingeengt und im Hochvakuum getrocknet. Man erhält 8 mg (17.1% d.Th.) der Titelverbindung.
¹H-NMR (400 MHz, Methanol-d₄): δ = 8.34 (m, 1H), 8.06 (s, 1H), 7.83 (d, 1H), 7.47 (dd, 1H), 4.45 (m, 1H), 3.83 (m, 1H), 3.49 (m, 1H), 3.42-3.26 (m, 4H), 2.38 (m, 1H), 2.28 (m, 1H), 2.17 (s, 3H), 2.10 (m, 2H), 1.95 (m, 1H) ppm.
HPLC: Rₜ = 3.3 min (Methode H)
MS (ESIpos): *m*/*z* = 344 (M+H)⁺ (freie Base).

### Beispiel 89

### N-[(3R)-1-Azabicyclo[2.2.2]oct-3-yl]-4-amino-1-benzothiophen-2-carboxamid-Dihydrochlorid

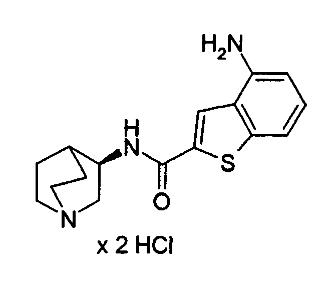

70 mg (0.19 mmol) 4-Nitro-1-benzothiophen-2-carbonsäure werden mit 2 ml Methanol und 460 µl 1 N Salzsäure versetzt. Nach Zugabe von 7 mg Palladium auf Kohle (10%-ig) wird das Reaktionsgemisch für 2 h bei RT und Normaldruck hydriert. Das Reaktionsgemisch wird über Kieselgur filtriert und durch präparative HPLC gereinigt. Die Produktfraktionen werden eingeengt, mit einem Gemisch aus Methanol und 4 M HCl in Dioxan versetzt, anschließend erneut eingeengt und im Hochvakuum getrocknet. Man erhält 75.5 mg (98.7% d.Th.) der Titelverbindung.
HPLC: Rₜ = 2.9 min (Methode H)
MS (ESIpos): *m*/*z* = 302 (M+H)⁺ (freie Base).

### Beispiel 90

### N-[(3R)-1-Azabicyclo[2.2.2]oct-3-yl]-3-chlor-6-amino-1-benzothiophen-2-carboxamid-Dihydrochlorid

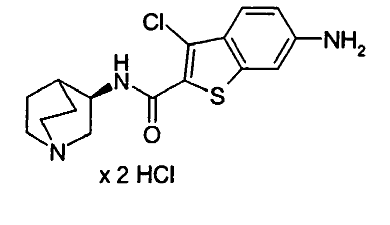

83 mg (0.23 mmol) N-[(3R)-1-Azabicyclo[2.2.2]oct-3-yl]-3-chlor-6-nitro-1-benzothiophen-2-carboxamid-Hydrochlorid werden in 1.5 ml 2 M Zinn(II)chlorid-Lösung in DMF gelöst und fiir 14 h bei RT gerührt. Das Reaktionsgemisch wird durch präparative HPLC gereinigt. Die Produktfraktionen werden eingeengt, in einem Gemisch aus Methanol und 4 M HCl in Dioxan gelöst, anschließend erneut eingeengt und im Hochvakuum getrocknet. Man erhält 53 mg (57.2% d.Th.) der Titelverbindung.
¹H-NMR (300 MHz, Methanol-d₄): δ = 7.69 (d, 1H), 7.20 (d, 1H), 7.02 (dd, 1H), 4.46 (m, 1H), 3.83 (m, 1H), 3.52-3.25 (m, 5H), 2.42 (m, 1H), 2.27 (m, 1H), 2.11 (m, 2H), 2.02 (m, 1H) ppm.
HPLC: Rₜ = 3.0 min (Methode H)
MS (ESIpos): *m*/*z* = 336 (M+H)⁺ (freie Base).

### Beispiel 91

### N-[(3R)-1-Azabicyclo[2.2.2]oct-3-yl]-6-(isopropylamino)-1-benzothiophen-2-carboxamid-Dihydrochlorid

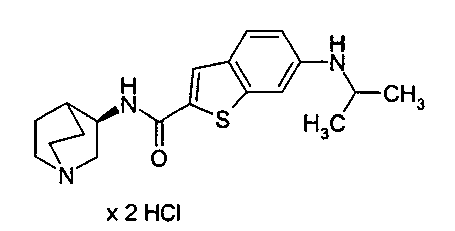

Eine Lösung aus 150 mg (0.40 mmol) 6-Amino-N-[(3R)-1-azabieyclo[2.2.2]oct-3-yl]-1-benzothiophen-2-carboxamid-Dihydrochlorid und 48 µl (0.65 mmol) Aceton in 1.5 ml 1,2-Dichlorethan wird mittels Essigsäure auf pH 4 eingestellt. Es werden 254.8 mg (1.20 mmol) Natriumtriacetoxyborhydrid hinzugefügt und bei RT 6 h lang gerührt. Der Kolbeninhalt wird im Vakuum eingeengt und mittels präparativer HPLC gereinigt. Die Produktfraktionen werden eingeengt, der Rückstand in einem 5:1-Gemisch aus Acetonitril und 1 N Salzsäure gelöst, anschließend erneut eingeengt und im Hochvakuum getrocknet. Man erhält 49 mg (29.4% d.Th.) der Titelverbindung.
¹H-NMR (300 MHz, Methanol-d₄): δ = 8.32 (s, 1H), 8.15 (m, 2H), 7.53 (dd, 1H), 4.49 (m, 1H), 3.88 (m, 1H), 3.83 (m, 1H), 3.56 (m, 1H), 3.50-3.23 (m, 4H), 2.39 (m, 1H), 2.31 (m, 1H), 2.11 (m, 2H), 1.95 (m, 1H), 1.39 (d, 6H) ppm.
HPLC: Rₜ = 3.1 min (Methode H)
MS (ESIpos): *m*/*z* = 344 (M+H)⁺ (freie Base).

### Beispiel 92

### 6-[(Z)-Amino(hydroxyimino)methyl]-N-[(3R)-1-azabicyclo[2.2.2]oct-3-yl]-1-benzothiophen-2-carboxamid-Dihydrochlorid

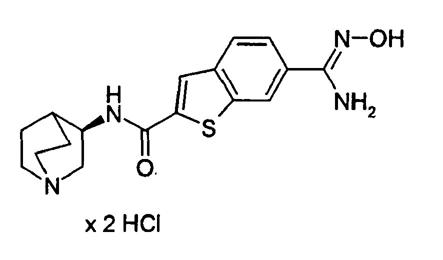

800 mg (2.0 mmol) N-[(3R)-1-Azabicyclo[2.2.2]oct-3-yl]-6-cyano-1-benzothiophen-2-carboxamid-Hydrochlorid, 278.1 mg (4.0 mmol) Hydroxylamin-Hydrochlorid und 829.5 mg (6.0 mmol) Kaliumcarbonat werden in 8 ml eines 8:1-Gemisches aus Wasser und Ethanol für 3 h auf 80°C erhitzt. Das Gemisch wird säulenchromatographisch an Kieselgel aufgereinigt (Laufmittel: Dichlormethan/Methanol/25% Ammoniak 100:20:4). Die Produktfraktionen werden vereinigt, eingeengt, mit Methanol und 4 M HCl in Dioxan versetzt, anschließend erneut eingeengt und im Hochvakuum getrocknet. Man erhält 447.3 mg (53.6% d.Th.) der Titelverbindung.
¹H-NMR (200 MHz, DMSO-d₆): δ = 11.15 (m, 1H), 10.22 (m, 1H), 9.36 (d, 1H), 8.52 (s, 1H), 8.46 (m, 1H), 8.14 (d, 1H), 7.73 (dd, 1H), 4.33 (m, 1H), 3.93-3.10 (m, 6H), 2.32-2.05 (m, 2H), 1.93 (m, 2H), 1.75 (m, 1H) ppm.
HPLC: Rₜ = 2.9 min (Methode H)
MS (ESIpos): *m*/*z* = 345 (M+H)⁺ (freie Base).

### Beispiel 93

### N-[(3R)-1-Azabicyclo[2.2.2]oct-3-yl]-7-cyano-1-benzothiophen-2-carboxamid-Hydrochlorid

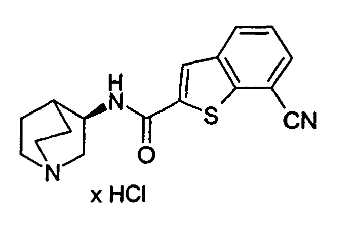

1.5 g (7.38 mmol) 7-Cyano-1-benzothiophen-2-carbonsäure und 1.47 g (7.38 mmol) *R*-3-Aminochinuklidin-Dihydrochlorid werden in 25 ml DMF vorgelegt. Bei 0°C wird die Lösung mit 1.70 g (8.86 mmol) EDC, 1.20 g (8.86 mmol) HOBt und 3.70 ml (26.6 mmol) Triethylamin versetzt. Bei RT wird für 18 h gerührt. Die Reaktion wird durch Zugabe von 10%-iger wässriger Natriumhydrogencarbonat-Lösung beendet und mit Essigsäureethylester versetzt. Der entstehende Niederschlag wird abgesaugt. Die Mutterlauge wird zweimal mit Essigsäureethylester extrahiert. Die organischen Phasen werden vereinigt und eingeengt. Das Rohprodukt wird an Kieselgel säulenchromatographisch gereinigt (Laufmittel: Dichlormethan/Methanol/25% Ammoniak 100:20:4). Die Produktfraktionen werden eingeengt, in einem Gemisch aus Methanol und 1 N Salzsäure gelöst, anschließend erneut eingeengt und im Hochvakuum getrocknet. Man erhält 655.1 mg (24.5% d.Th.) der Titelverbindung.
HPLC: Rₜ = 3.7 min (Methode H)
MS (ESIpos): *m*/*z* = 312 (M+H)⁺ (freie Base).

### Beispiel 94

### N-[(3R)-1-Azabicyclo[2.2.2]oct-3-yl]-7-nitro-1-benzothiophen-2-carboxamid-Hydrochlorid

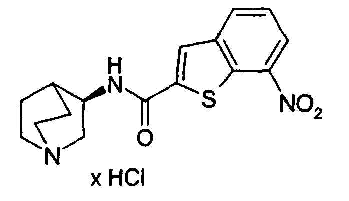

2.0 g (8.96 mmol) 7-Nitro-1-benzothiophen-2-carbonsäure und 1.78 g (8.96 mmol) *R*-3-Aminochinuklidin-Dihydrochlorid werden in 25 ml DMF vorgelegt. Bei 0°C wird die Lösung mit 2.06 g (10.75 mmol) EDC, 1.45 g (10.75 mmol) HOBt und 4.50 ml (32.26 mmol) Triethylamin versetzt. Bei RT wird für 18 h gerührt. Die Reaktion wird durch Zugabe von 10%-iger wässriger Natriumhydrogencarbonat-Lösung beendet und mit Essigsäureethylester versetzt. Der entstehende Niederschlag wird abgesaugt. Die Mutterlauge wird zweimal mit Essigsäureethylester extrahiert. Die organischen Phasen werden vereinigt und eingeengt. Sowohl der Niederschlag als auch die eingeengte Mutterlauge werden an Kieselgel säulenchromatographisch gereinigt (Laufmittel: Dichlormethan/Methanol/25% Ammoniak 100:10:2). Die Produktfraktionen werden eingeengt, in einem Gemisch aus Methanol und 1 N Salzsäure gelöst, anschließend erneut eingeengt und im Hochvakuum getrocknet. Der entstandene Feststoff wird mit Acetonitril verrührt, abgesaugt und im Vakuum bei 50°C getrocknet. Man erhält 1.10 g (32.8% d.Th.) der Titelverbindung.
¹H-NMR (500 MHz, DMSO-d₆): δ = 9.90 (s, 1H, br), 9.25 (d, 1H), 8.56 (d, 1H), 8.50 (s, 1H), 8.49 (d, 1H), 7.78 (dd, 1H), 4.37 (m, 1H), 3.69 (m, 1H), 3.62-3.19 (m, 5H), 2.23 (m, 1H), 2.14 (m, 1H), 1.93 (m, 2H), 1.78 (m, 1H) ppm.
HPLC: Rₜ = 3.8 min (Methode H)
MS (ESIpos): *m*/*z* = 332 (M+H)⁺ (freie Base).

### Beispiel 95

### N-[(3R)-1-Azabicyclo[2.2.2]oct-3-yl]-6-[(methylsulfonyl)amino]-1-benzothiophen-2-carboxamid-Hydrochlorid

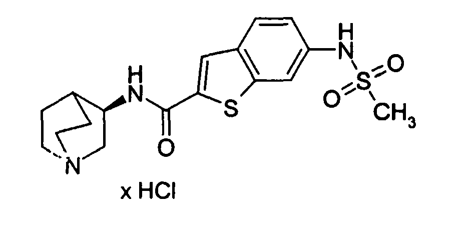

40 mg (0.11 mmol) N-[(3R)-1-Azabicylo[2.2.2]oct-3-yl]-6-amino-1-benzothiophen-2-carboxamid-Hydrochlorid werden in 2 ml DMF gelöst, mit 6.5 mg (0.05 mmol) N,N-Dimethyl-4-aminopyridin, 59.6 µl (0.43 mmol) Triethylamin und 16.6 1 (0.21 mmol) Methansulfonsäurechlorid versetzt und 16 h bei Raumtemperatur gerührt. Das Reaktionsgemisch wird durch präparative HPLC gereinigt. Das Produkt wird in einem Gemisch aus 1 N wässriger Salzsäure und Acetonitril gelöst, anschließend wieder eingeengt und im Hochvakuum getrocknet. Man erhält 15 mg (33.8% d.Th.) der Titelverbindung.
¹H-NMR (400 MHz, Methanol-d₄): δ = 8.07 (s, 1H), 7.87 (d, 1H), 7.81 (d, 1H), 7.32 (dd, 1H), 4.44 (m, 1H), 3.83 (m, 1H), 3.48 (m, 1H), 3.42-3.24 (m, 4H), 3.00 (s, 3H), 2.37 (m, 1H), 2.27 (m, 1H), 2.10 (m, 2H), 1.95 (m, 1H) ppm.
HPLC: Rₜ = 3.3 min (Methode H)
MS (ESIpos): *m*/*z* = 380 (M+H)⁺ (freie Base).

### Beispiel 96

### N-[(3R)-1-Azabieyclo[2.2.2]oct-3-yl]-6-[bis(phenylsulfonyl)amino]-1-benzothiophen-2-carboxamid-Hydrochlorid

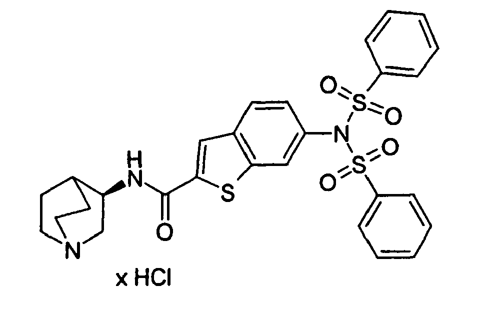

40 mg (0.11 mmol) N-[(3R)-1-Azabicyclo[2.2.2]oct-3-yl]-6-amino-1-benzothiophen-2-carboxamid-Hydrochlorid werden in 2 ml DMF gelöst, mit 6.5 mg (0.05 mmol) N,N-Dimethyl-4-aminopyridin, 59.6 µl (0.43 mmol) Triethylamin und 27.2 µl (0.21 mmol) Benzolsulfonsäurechlorid versetzt und 16 h bei Raumtemperatur gerührt. Das Reaktionsgemisch wird durch präparative HPLC gereinigt. Das Produkt wird in einem Gemisch aus 1 N wässriger Salzsäure und Acetonitril gelöst, anschließend erneut eingeengt und im Hochvakuum getrocknet. Man erhält 33 mg (50% d.Th.) der Titelverbindung.
¹H-NMR (400 MHz, Methanol-d₄): δ = 8.13 (s, 1H), 7.94-7.82 (m, 5H), 7.78 (m, 2H), 7.68-7.55 (m, 5H), 7.01 (dd, 1H), 4.45 (m, 1H), 3.84 (m, 1H), 3.47 (m, 1H), 3.42-3.22 (m, 4H), 2.39 (m, 1H), 2.28 (m, 1H), 2.10 (m, 2H), 1.95 (m, 1H) ppm.
HPLC: Rₜ = 4.4 min (Methode H)
MS (ESIpos): *m*/*z* = 582 (M+H)⁺ (freie Base).

### Beispiel 97

### N-[(3R)-1-Azabicyclo[2.2.2]oct-3-yl]-6-(benzoylamino)-1-benzothiophen-2-carboxamid-Hydrochlorid

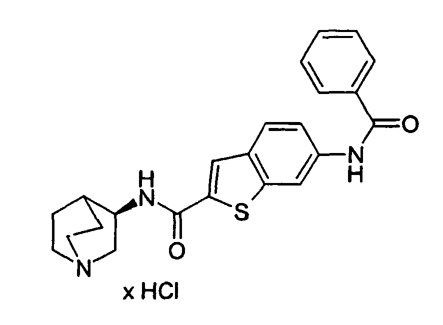

40 mg (0.11 mmol) N-[(3R)-1-Azabicyclo[2.2.2]oct-3-yl]-6-amino-1-benzothiophen-2-carboxamid-Dihydrochlorid werden in 2 ml DMF gelöst und mit 59.6 µl (0.43 mmol) Triethylamin versetzt. Es werden 30 mg (0.21 mmol) Benzoylchlorid hinzugefügt. Nach 18 h Rühren bei RT wird das Reaktionsgemisch mittels präparativer HPLC getrennt. Die Produktfraktion wird im Vakuum eingeengt, mit einem Gemisch aus Acetonitril und 1 N Salzsäure versetzt, anschließend erneut eingeengt und im Hochvakuum getrocknet. Man erhält 32 mg (67.8% d.Th.) der Titelverbindung.
¹H-NMR (400 MHz, Methanol-d₄): δ = 8.46 (s, 1H), 8.11 (s, 1H), 8.01-7.93 (m, 2H), 7.89 (d, 1H), 7.49 (dd, 1H), 7.58 (d, 1H), 7.56-7.48 (m, 2H), 4.46 (m, 1H), 3.83 (m, 1H), 3.50 (m, 1H), 3.44-3.24 (m, 4H), 2.38 (m, 1H), 2.28 (m, 1H), 2.10 (m, 2H), 1.95 (m, 1H) ppm.
HPLC: Rₜ = 4.6 min (Methode H)
MS (ESIpos): *m*/*z* = 406 (M+H)⁺ (freie Base).

### Beispiel 98

### 7-[(Z)-Amino(hydroxyimino)methyl]-N-[(3R)-1-azabicyclo[2.2.2]oct-3-yl]-1-benzothiophen-2-carboxamid-Dihydrochlorid

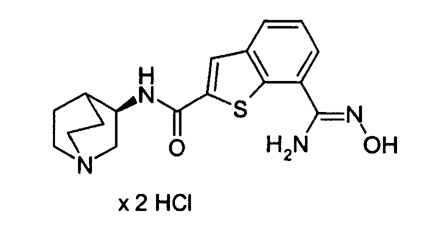

120 mg (0.43 mmol) N-[(3R)-1-Azabicyclo[2.2.2]oct-3-yl]-7-cyano-1-benzothiophen-2-carboxamid-Hydrochlorid, 45.0 mg (0.65 mmol) Hydroxylamin-Hydrochlorid und 119.2 mg (0.86 mmol) Kaliumcarbonat werden in 1.5 ml eines 8:1-Gemisches aus Wasser und Ethanol für 18 h auf 80°C erhitzt. Das Gemisch wird mittels präparativer HPLC aufgereinigt. Die Produktfraktionen werden vereinigt, eingeengt, mit Acetonitril und 1 N wässriger Salzsäure (3:1) versetzt, anschließend erneut eingeengt und im Hochvakuum getrocknet. Man erhält 58 mg (30.3% d.Th.) der Titelverbindung.
¹H-NMR (300 MHz, Methanol-d₄): δ = 8.34 (s, 1H), 8.23 (d, 1H), 7.73 (d, 1H), 7.65 (dd, 1H), 4.48 (m, 1H), 3.82 (m, 1H), 3.56 (m, 1H), 3.48-3.16 (m, 4H), 2.39 (m, 1H), 2.30 (m, 1H), 2.10 (m, 2H), 1.96 (m, 1H) ppm.
HPLC: Rₜ = 2.8 min (Methode H)
MS (ESIpos): *m*/*z* = 345 (M+H)⁺ (freie Base).

## Patentansprüche

1. Verbindungen der Formel 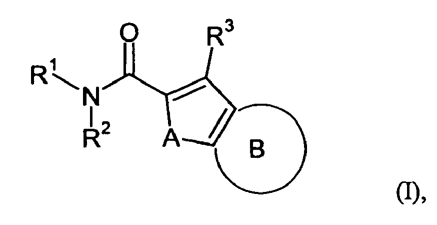 in welcher
R¹ für 1-Aza-bicyclo[2.2.2]oct-3-yl steht,
R² für Wasserstoff oder C₁-C₆-Alkyl steht,
R³ für Wasserstoff, Halogen oder C₁-C₆-Alkyl steht,
A für Sauerstoff oder Schwefel steht,
und
der Ring B für Benzo, Pyrimido, Pyridazo oder Pyridazino, die gegebenenfalls durch Reste ausgewählt aus der Gruppe Wasserstoff, Halogen, C₁-C₆-Alkanoyl, Carbamoyl, Cyano, Trifluormethyl, Trifluormethoxy, Nitro, Amino, C₁-C₆-Acylamino, C₁-C₆-Alkyl, C₁-C₆-Alkoxy, C₁-C₆-Alkylthio, C₁-C₆-Alkylamino, Heteroarylcarbonylamino, Arylcarbonylamino, C₁-C₄-Alkylsulfonylamino, Di-(C₁-C₄-alkylsulfonyl)amino, Arylsulfonylamino, Di-(arylsulfonyl)amino, C₃-C₆-Cycloalkylcarbonylmethyl, 1,3-Dioxa-propan-1,3-diyl, Amino-(hydroxyimino)methyl und Benzo substituiert sind, steht,
und deren Salze, Solvate und Solvate der Salze.

2. Verbindungen der Formel (I) nach Anspruch 1,
in welcher
R¹ für 1-Aza-bicyclo[2.2.2]oct-3-yl steht,
R² für Wasserstoff oder (C₁-C₆)-Alkyl steht,
R³ für Wasserstoff, Halogen oder (C₁-C₆)-Alkyl steht,
A für Sauerstoff oder Schwefel steht,
und
der Ring B für Benzo, Pyrimido, Pyridazo oder Pyridazino, die gegebenenfalls durch Reste ausgewählt aus der Gruppe Wasserstoff, Halogen, Formyl, Carbamoyl, Cyano, Trifluormethyl, Trifluormethoxy, Nitro, Amino, Formamido, Acetamido, (C₁-C₆)-Alkyl, (C₁-C₆)-Alkoxy, (C₁-C₆)-Alkylthio und Benzo substituiert sind, steht,
und deren Salze, Solvate und Solvate der Salze.

3. Verbindungen der Formel (I) nach Anspruch 1,
in welcher
R¹ für 1-Aza-bicyclo[2.2.2]oct-3-yl steht,
R² für Wasserstoff steht,
R³ für Wasserstoff, Chlor, Fluor oder Methyl steht,
A für Sauerstoff oder Schwefel steht,
und
der Ring B für Benzo, welches gegebenenfalls durch 1 bis 3 Reste ausgewählt aus der Gruppe Wasserstoff, Halogen, Formyl, Carbamoyl, Cyano, Trifluormethyl, Trifluormethoxy, Nitro, Amino, Formamido, Acetamido, C₁-C₄-Alkyl, C₁-C₄-Alkoxy, C₁-C₄-Alkylthio, C₁-C₄-Alkylamino, Furylcarbonylamino, Phenylcarbonylamino, Methylsulfonylamino, Di-(phenylsulfonyl)amino, Cyclopropylcarbonylmethyl, 1,3-Dioxapropan-1,3-diyl, Amino(hydroxyimino)methyl und Benzo substituiert sind, steht,
und deren Salze, Solvate und Solvate der Salze.

4. Verbindungen nach Anspruch 1 der Formel (I), wobei
R¹ für (3*R*)-1-Aza-bicyclo[2.2.2]oct-3-yl steht,
und R², R³, A und der Ring B die in Anspruch 1 angegebenen Bedeutungen haben.

5. Verbindungen nach Anspruch 1 der Formel (I), wobei
R¹ für (3*R*)-1-Aza-bicyclo[2.2.2]oct-3-yl steht,
R² und R³ für Wasserstoff stehen,
A für Schwefel steht,
und
der Ring B für Benzo, welches gegebenenfalls durch 1 bis 3 Reste ausgewählt aus der Gruppe Wasserstoff, Halogen, Cyano, Trifluormethyl, Trifluormethoxy, Nitro, Amino, Formamido, Acetamido und C₁-C₄-Alkyl substituiert sind,
und deren Salze, Solvate und Solvate der Salze.

6. Verbindungen nach Anspruch 1 der Formel 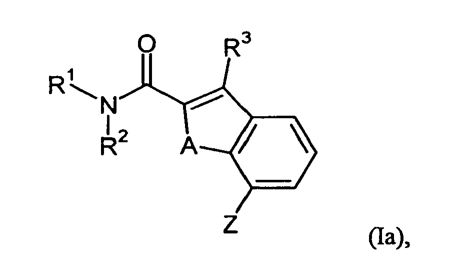 in welcher
R¹ für 1-Aza-bicyclo[2.2.2]oct-3-yl steht,
R² für Wasserstoff oder C₁-C₆-Alkyl steht,
R³ für Wasserstoff, Halogen oder C₁-C₆-Alkyl steht,
A für Sauerstoff oder Schwefel steht,
Z für Wasserstoff, Halogen, Formyl, Carbamoyl, Cyano, Trifluormethyl, Trifluormethoxy, Nitro, Amino, Formamido, Acetamido, C₁-C₆-Alkyl, C₁-C₆-Alkoxy, C₁-C₆-Alkylthio, C₁-C₆-Alkylamino, Heteroarylcarbonylamino, Arylcarbonylamino, C₁-C₄-Alkylsulfonylamino, Di-(arylsulfonyl)amino, C₃-C₆-Cycloalkylcarbonylmethyl oder Amino-(hydroxyimino)methyl steht,
und deren Salze, Solvate und Solvate der Salze.

7. Verbindungen nach Anspruch 1 der Formel (Ia),
in welcher
R¹ für 1-Aza-bicyclo[2.2.2]oct-3-yl steht,
R² für Wasserstoff steht,
R³ für Wasserstoff, Chlor, Fluor oder Methyl steht,
A für Sauerstoff oder Schwefel steht,
und
Z für Wasserstoff, Halogen, Formyl, Carbamoyl, Cyano, Trifluormethyl, Trifluormethoxy, Nitro, Amino, Formamido, Acetamido, Methyl, Ethyl, Methoxy, Ethoxy, C₁-C₄-Alkylamino, Furylcarbonylamino, Phenylcarbonylamino, Methylsulfonylamino, Di-(phenylsulfonyl)amino, Cyclopropylcarbonylmethyl oder Amino(hydroxyimino)methyl steht,
und deren Salze, Solvate und Solvate der Salze.

8. Verbindungen nach Anspruch 1 der Formel (Ia),
in welcher
R¹ für (*3R*)-1-Aza-bicyclo[2.2.2]oct-3-yl steht,
R² für Wasserstoff steht,
R³ für Wasserstoff, Chlor, Fluor oder Methyl steht,
A für Sauerstoff oder Schwefel steht,
und
Z für Wasserstoff, Halogen, Formyl, Carbamoyl, Cyano, Trifluormethyl, Trifluormethoxy, Nitro, Amino, Formamido, Acetamido, Methyl, Ethyl, Methoxy, Ethoxy, C₁-C₄-Alkylamino, Furylcarbonylamino, Phenylcarbonylamino, Methylsulfonylamino, Di-(phenylsulfonyl)amino, Cyclopropylcarbonylmethyl oder Amino(hydroxyimino)-methyl steht,
und deren Salze, Solvate und Solvate der Salze.

9. Verbindungen nach Anspruch 1 der Formel (I) ausgewählt aus der Gruppe N-[(3R)-1-Azabicyclo[2.2.2]oct-3-yl]-6-amino-1-benzofuran-2-carboxamid, N-[(3R)-1-Azabicyclo[2.2.2]oct-3-yl]-5-fluor-1-benzofuran-2-carboxamid-Hydrochlorid, N-[(3R)-1-Azabicyclo[2.2.2]oct-3-yl]-7-fluor-1-benzofuran-2-carboxamid-Hydrochlorid, N-[(3R)-1-Azabicyclo[2.2.2]oct-3-yl]-3-fluor-1-benzothiophen-2-carboxamid-Hydrochlorid, N-[(3R)-1-Azabicyclo[2.2.2]oct-3-yl]-7-trifluormethyl-1-benzothiophen-2-carboxamid-Hydrochlorid, N-[(3R)-1-Azabicyclo[2.2.2]oct-3-yl]-7-amino-1-benzothiophen-2-carboxamid-Dihydrochlorid, N-[(3R)-1-Azabicyclo[2.2.2]oct-3-yl]-7-chlor-1-benzothiophen-2-carboxamid-Hydrochlorid, 6-Amino-N-[(3R)-1-azabicyclo[2.2.2]oct-3-yl]-1-benzothiophen-2-carboxamid-Dihydrochlorid, N-[(3R)-1-Azabicyclo[2.2.2]oct-3-yl]-5-fluor-1-benzothiophen-2-carboxamid-Hydrochlorid, N-[(3R)-1-Azabicyclo[2.2.2]oct-3-yl]-7-methoxy-1-benzothiophen-2-carboxamid-Hydrochlorid, N-[(3R)-1-Azabicyclo[2.2.2]oct-3-yl]-5,7-difluor-1-benzothiophen-2-carboxamid-Hydrochlorid, 6-(Acetylamino)-N-[(3R)-1-azabicyclo[2.2.2]oct-3-yl]-1-benzothiophen-2-carboxamid-Hydrochlorid, N-[(3R)-1-Azabicyclo[2.2.2]oct-3-yl]-6-(isopropylamino)-1-benzothiophen-2-carboxamid-Dihydrochlorid, N-[(3R)-1-Azabicyclo[2.2.2]oct-3-yl]-7-cyano-1-benzothiophen-2-carboxamid-Hydrochlorid und N-[(3R)-1-Azabicyclo[2.2.2]oct-3-yl]-6-[(methylsulfonyl)amino]-1-benzothiophen-2-carboxamid-Hydrochlorid und deren Salze, Solvate und Solvate der Salze.

10. Verfahren zur Herstellung von Verbindungen der Formel (I) nach Anspruch 1, **dadurch gekennzeichnet, dass** man Verbindungen der Formel
R¹R²NH (II),
in welcher R¹ und R² die in Anspruch 1 genannte Bedeutung haben,
mit einer Verbindung der Formel 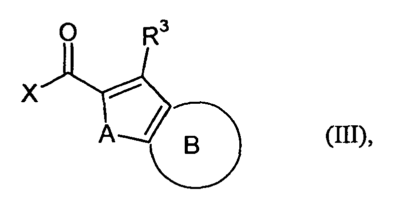 in welcher
R³, A und der Ring B die in Anspruch 1 genannten Bedeutungen haben, und
X für Hydroxy oder eine geeignete Abgangsgruppe steht,
in einem inerten Lösungsmittel gegebenenfalls in Gegenwart eines Kondensationsmittels und gegebenenfalls in Gegenwart einer Base umsetzt.

11. Verbindungen nach einem der Ansprüche 1 bis 9 zur Behandlung und/oder Prophylaxe von Krankheiten.

12. Arzneimittel enthaltend mindestens eine der Verbindungen nach einem der Ansprüche 1 bis 9 in Zusammenmischung mit mindestens einem pharmazeutisch verträglichen, im wesentlichen nichtgiftigen Träger oder Exzipienten.

13. Verwendung von Verbindungen nach einem der Ansprüche 1 bis 9 zur Herstellung eines Arzneimittels zur Verbesserung der Wahrnehmung, Konzentrationsleistung, Lernleistung und/oder Gedächtnisleistung.

14. Verwendung von Verbindungen nach einem der Ansprüche 1 bis 9 zur Herstellung eines Arzneimittels zur Behandlung und/oder Prophylaxe von Störungen der Wahrnehmung, Konzentrationsleistung, Lernleistung und/oder Gedächtnisleistung.

15. Arzneimittel nach Anspruch 12 zur Behandlung und/oder Prophylaxe von Störungen der Wahrnehmung, Konzentrationsleistung, Lernleistung und/oder Gedächtnisleistung.

## Claims

1. Compound of the formula 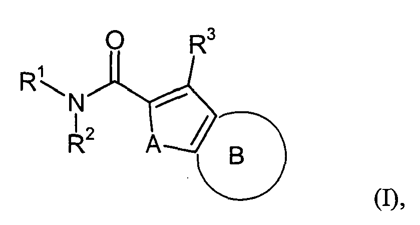 in which
R¹ represents 1-azabicyclo[2.2.2]oct-3-yl,
R² represents hydrogen or C₁-C₆-alkyl,
R³ represents hydrogen, halogen or C₁-C₆-alkyl,
A represents oxygen or sulfur,
and
the ring B represents benzo, pyrimido, pyridazo or pyridazino which are optionally substituted by radicals selected from the group consisting of hydrogen, halogen, C₁-C₆-alkanoyl, carbamoyl, cyano, trifluoromethyl, trifluoromethoxy, nitro, amino, C₁-C₆-acylamino, C₁-C₆-alkyl, C₁-C₆-alkoxy, C₁-C₆-alkylthio, C₁-C₆-alkylamino, heteroarylcarbonylamino, arylcarbonylamino, C₁-C₄-alkylsulfonylamino, di-(C₁-C₄-alkylsulfonyl)amino, arylsulfonylamino, di(arylsulfonyl)amino, C₃-C₆-cycloalkylcarbonylmethyl, 1,3-dioxapropane-1,3-diyl, amino(hydroxyimino)methyl and benzo,
or a salt, a solvate or a solvate of a salt thereof.

2. Compound of the formula (I) according to Claim 1,
in which
R¹ represents 1-azabicydo[2.2.2]oct-3-yl,
R² represents hydrogen or (C₁-C₆)-alkyl,
R³ represents hydrogen, halogen or (C₁-C₆)-alkyl,
A represents oxygen or sulfur,
and
the ring B represents benzo, pyrimido, pyridazo or pyridazino which are optionally substituted by radicals selected from the group consisting of hydrogen, halogen, formyl, carbamoyl, cyano, trifluoromethyl, trifluoromethoxy, nitro, amino, formamido, acetamido, (C₁-C₆)-alkyl, (C₁-C₆)-alkoxy, (C₁-C₆)-alkylthio and benzo,
or a salt, a solvate or a solvate of a salt thereof.

3. Compound of the formula (I) according to Claim 1,
in which
R¹ represents 1-azabicyclo[2.2.2]oct-3-yl,
R² represents hydrogen,
R³ represents hydrogen, chlorine, fluorine or methyl,
A represents oxygen or sulfur,
and
the ring B represents benzo which is optionally substituted by 1 to 3 radicals selected from the group consisting of hydrogen, halogen, formyl, carbamoyl, cyano, trifluoromethyl, trifluoromethoxy, nitro, amino, formamido, acetamido, C₁-C₄-alkyl, C₁-C₄-alkoxy, C₁-C₄-alkylthio, C₁-C₄-alkylamino, furylcarbonylamino, phenylcarbonylamino, methylsulfonylamino, di(phenylsulfonyl)amino, cyclopropylcarbonylmethyl, 1,3-dioxapropane-1,3-diyl, amino(hydroxyimino)methyl and benzo,
or a salt, a solvate or a solvate of a salt thereof.

4. Compound according to Claim 1 of the formula (I), where
R¹ represents (3*R*)-1-azabicyclo[2.2.2]oct-3-yl,
and R², R³, A and the ring B are as defined in claim 1.

5. Compound according to Claim 1, of the formula (I), where
R¹ represents (3*R*)-1-azabicyclo[2.2.2]oct-3-yl,
R² and R³ represent hydrogen,
A represents sulfur,
and
the ring B represents benzo which is optionally substituted by 1 to 3 radicals selected from the group consisting of hydrogen, halogen, cyano, trifluoromethyl, trifluoromethoxy, nitro, amino, formamido, acetamido and C₁-C₄-alkyl,
or a salt, a solvate or a solvate of a salt thereof.

6. Compound according to Claim 1 of the formula 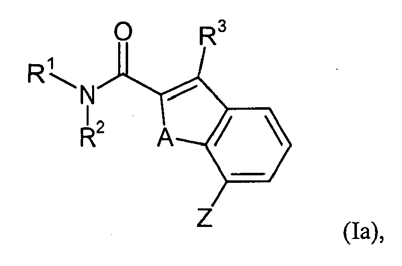 in which
R¹ represents 1-azabicyclo[2.2.2]oct-3-yl,
R² represents hydrogen or C₁-C₆-alkyl,
R³ represents hydrogen, halogen or C₁-C₆-alkyl,
A represents oxygen or sulfur,
Z represents hydrogen, halogen, formyl, carbamoyl, cyano, trifluoromethyl, trifluoromethoxy, nitro, amino, formamido, acetamido, C₁-C₆-alkyl, C₁-C₆-alkoxy, C₁-C₆-alkylthio, C₁-C₆-alkylamino, heteroarylcarbonylamino, arylcarbonylamino, C₁-C₄-alkylsulfonylamino, di(arylsulfonyl)amino, C₃-C₆-cycloalkylcarbonylmethyl or amino(hydroxyimino)methyl,
or a salt, a solvate or a solvate of a salt thereof.

7. Compound according to Claim 1 of the formula (Ia),
in which
R¹ represents 1-azabicyclo[2.2.2]oct-3-yl,
R² represents hydrogen,
R³ represents hydrogen, chlorine, fluorine or methyl,
A represents oxygen or sulfur,
and
Z represents hydrogen, halogen, formyl, carbamoyl, cyano, trifluoromethyl, trifluoromethoxy, nitro, amino, formamido, acetamido, methyl, ethyl, methoxy, ethoxy, C₁-C₄-alkylamino, furylcarbonylamino, phenylcarbonylamino, methylsulfonylamino, di(phenylsulfonyl)amino, cyclopropylcarbonylmethyl or amino(hydroxyimino)methyl,
or a salt, a solvate or a solvate of a salt thereof.

8. Compound according to Claim 1 of the formula (Ia),
in which
R¹ represents (3*R*)-1-azabicyclo[2.2.2]oct-3-yl,
R² represents hydrogen,
R³ represents hydrogen, chlorine, fluorine or methyl,
A represents oxygen or sulfur,
and
Z represents hydrogen, halogen, formyl, carbamoyl, cyano, trifluoromethyl, trifluoromethoxy, nitro, amino, formamido, acetamido, methyl, ethyl, methoxy, ethoxy, C₁-C₄-alkylamino, furylcarbonylamino, phenylcarbonylamino, methylsulfonylamino, di(phenylsulfonyl)amino, cyclopropylcarbonylmethyl or amino(hydroxyimino)methyl,
or a salt, a solvate or a solvate of a salt thereof.

9. Compound according to Claim 1 of the formula (I) selected from the group consisting of N-[(3R)-1-azabicyclo[2.2.2]oct-3-yl]-6-amino-1-benzofuran-2-carboxamide, N-[(3R)-1-azabicyclo[2.2.2]oct-3-yl]-5-fluoro-1-benzofuran-2-carboxamide hydrochloride, N-[(3R)-1-azabicyao[2.2.2]oct-3-yl]-7-fluoro-1-benzofuran-2-carboxamide hydrochloride, N-[(3R)-1-azabicyclo[2.2.2]oct-3-yl]-3-fluoro-1-benzothiophene-2-carboxamide hydrochloride, N-[(3R)-1-azabicyclo[2.2.2]oct-3-yl]-7-trifluoromethyl-1-benzothiophene-2-carboxamide hydrochloride, N-[(3R)-1-azabicyclo[2.2.2]oct-3-yl]-7-amino-1-benzothiophene-2-carboxamide dihydrochloride, N-[(3R)-1-azabicyclo[2.2.2]oct-3-yl]-7-chloro-1-benzothiophene-2-carboxamide hydrochloride, 6-amino-N-[(3R)-1-azabicyclo[2.2.2]oct-3-yl]-1-benzothiophene-2-carboxamide dihydrochloride, N-[(3R)-1-azabicyclo[2.2.2]oct-3-yl]-5-fluoro-1-benzothiophene-2-carboxamide hydrochloride, N-[(3R)-1-azabicyclo[2.2.2]oct-3-yl]-7-methoxy-1-benzothiophene-2-carboxamide hydrochloride, N-[(3R)-1-azabicyclo[2.2.2]oct-3-yl]-5,7-difluoro-1-benzothiophene-2-carboxamide hydrochloride, 6-(acetylamino)-N-[(3R)-1-azabicyclo[2.2.2]oct-3-yl]-1-benzothiophene-2-carboxamide hydrochloride, N-[(3R)-1-azabicyclo[2.2.2]oct-3-yl]-6-(isopropylamino)-1-benzothiophene-2-carboxamide dihydrochloride, N-[(3R)-1-azabicyclo-[2.2.2]oct-3-yl]-7-cyano-1-benzothiophene-2-carboxamide hydrochloride and N-[(3R)-1-azabicyclo[2.2.2]oct-3-yl]-6-[(methylsulphonyl)amino]-1-benzothiophene-2-carboxamide hydrochloride and their salts, solvates and solvates of the salts.

10. Process for preparing compounds of the formula (I) according to Claim 1, **characterized in that**
compounds of the formula
R¹R²NH (II),
in which R¹ and R² are as defined in Claim 1
are reacted with a compound of the formula 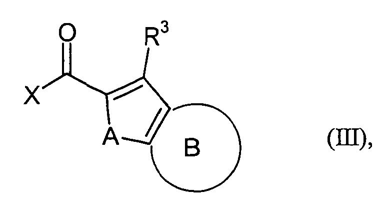 in which
R³, A and the ring B are as defined in Claim 1 and
X represents hydroxy or a suitable leaving group,
in an inert solvent, if appropriate in the presence of a condensing agent and if appropriate in the presence of a base.

11. A compound according to any of Claims 1 to 9 for the treatment and/or prophylaxis of diseases.

12. A medicament comprising at least one of the compounds according to any of Claims 1 to 9 in a mixture with at least one pharmaceutically acceptable, essentially nontoxic carrier or excipient.

13. The use of compounds according to any of Claims 1 to 9 for preparing a medicament for improving perception, concentration, learning and/or memory.

14. The use of compounds according to any of Claims 1 to 9 for preparing a medicament for the treatment and/or prophylaxis of impairments of perception, concentration, learning and/or memory.

15. A medicament according to Claim 12 for the treatment and/or prophylaxis of impairments of perception, concentration, learning and/or memory.

## Revendications

1. Composés de formule 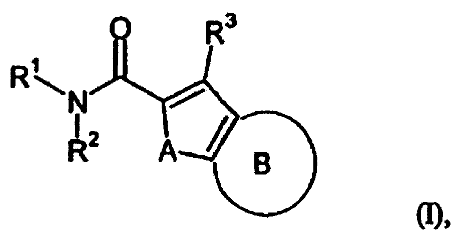 dans laquelle
R¹ représente un groupe 1-aza-bicyclo[2.2.2]oct-3-yle,
R² représente l'hydrogène ou un reste alkyle en C₁ à C₆,
R³ représente l'hydrogène, un halogène ou un reste alkyle en C₁ à C₆,
A représente l'oxygène ou le soufre,
et
le noyau B représente un noyau benzo, un noyau pyrimido, un noyau pyridazo ou un noyau pyridazino, qui sont éventuellement substitués par des restes choisis dans le groupe des restes hydrogène, halogène, alcanoyle en C₁ à C₆, carbamoyle, cyano, trifluorométhyle, trifluorométhoxy, nitro, amino, acylamino en C₁ à C₆, alkyle en C₁ à C₆, alkoxy en C₁ à C₆, alkylthio en C₁ à C₆, alkylamino en C₁ à C₆, hétéroarylcarbonylamino, arylcarbonylamino, alkylsulfonylamino en C₁ à C₄, di(alkylsulfonyle en C₁ à C₄)amino, arylsulfonylamino, di(arylsulfonyl)amino, (cycloalkyle en C₃ à C₆)-carbonylméthyle, 1,3-dioxa-propane-1,3-diyle, amino-(hydroxyimino)méthyle et benzo,
et leurs sels, leurs produits de solvatation et les produits de solvatation des sels.

2. Composés de formule (I) suivant la revendication 1,
dans laquelle
R¹ représente un groupe 1-aza-bicyclo[2.2.2]oct-3-yle,
R² représente l'hydrogène ou un reste alkyle en C₁ à C₆,
R³ représente l'hydrogène, un halogène ou un reste alkyle en C₁ à C₆,
A représente l'oxygène ou le soufre,
et
le noyau B représente un noyau benzo, un noyau pyrimido, un noyau pyridazo ou un noyau pyridazino, qui sont éventuellement substitués par des restes choisis dans le groupe des restes hydrogène, halogène, formyle carbamoyle, cyano, trifluorométhyle, trifluorométhoxy, nitro, amino, formamido, acétamido, alkyle en C₁ à C₆, alkoxy en C₁ à C₆, alkylthio en C₁ à C₆ et benzo,
et leurs sels, leurs produits de solvatation et les produits de solvatation des sels.

3. Composés de formule (I) suivant la revendication 1, dans laquelle
R¹ représente un groupe 1-aza-bicyclo[2.2.2]oct-3-yle,
R² représente l'hydrogène,
R³ représente l'hydrogène, le chlore, le fluor ou un reste méthyle,
A représente l'oxygène ou le soufre,
et
le noyau B représente un noyau benzo qui est éventuellement substitué par 1 à 3 restes choisis dans le groupe des restes hydrogène, halogène, formyle, carbamoyle, cyano, trifluorométhyle, trifluorométhoxy, nitro, amino, formamido, acétamido, alkyle en C₁ à C₄, alkoxy en C₁ à C₄, alkylthio en C₁ à C₄, alkylamino en C₁ à C₄, furylcarbonylamino, phénylcarbonylamino, méthylsulfonylamino, di(phénylsulfonyl)amino, cyclopropylcarbonylméthyle, 1,3-dioxapropane-1,3-diyle, amino(hydroximino)méthyle et benzo,
et leurs sels, leurs produits de solvatation et les produits de solvatation des sels.

4. Composés suivant la revendication 1, de formule (I) dans laquelle
R¹ représente un groupe (3*R*)-1-aza-bicyclo[2.2.2]oct-3-yle,
et R², R³, A et le noyau B ont les définitions indiquées dans la revendication 1.

5. Composés suivant la revendication 1, de formule (I) dans laquelle
R¹ représente un groupe (3*R*)-1-aza-bicyclo[2.2.2]oct-3-yle,
R² et R³ représentent l'hydrogène,
A représente le soufre,
et
le noyau B représente un noyau benzo qui est éventuellement substitué par 1 à 3 restes choisis dans le groupe des restes hydrogène, halogène, cyano, trifluorométhyle, trifluorométhoxy, nitro, amino, formamido, acétamido et alkyle en C₁ à C₄,
et leurs sels, leurs produits de solvatation et les produits de solvatation des sels.

6. Composés suivant la revendication 1, de formule 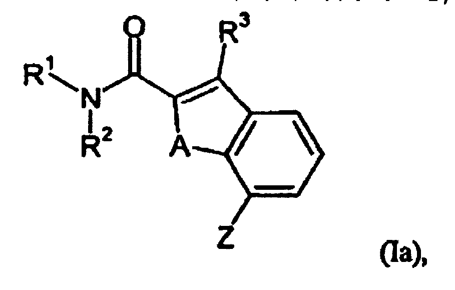 dans laquelle
R¹ représente un groupe 1-aza-bicyclo[2.2.2]oct-3-yle,
R² représente l'hydrogène ou un reste alkyle en C₁ à C₆,
R³ représente l'hydrogène, un halogène ou un reste alkyle en C₁ à C₆,
A représente l'oxygène ou le soufre,
Z représente l'hydrogène, un halogène, un reste formyle, carbamoyle, cyano, trifluorométhyle, trifluorométhoxy, nitro, amino, formamido, acétamido, alkyle en C₁ à C₆, alkoxy en C₁ à C₆, alkylthio en C₁ à C₆, alkylamino en C₁ à C₆, hétéroarylcarbonylamino, arylcarbonylamino, alkylsulfonylamino en C₁ à C₆, di(arylsulfonyl)amino, (cycloalkyle en C₃ à C₆)carbonylméthyle ou amino(hydroxyimino)méthyle,
et leurs sels, leurs produits de solvatation et les produits de solvatation des sels.

7. Composés suivant la revendication 1, de formule (Ia)
dans laquelle
R¹ représente un groupe 1-aza-bicyclo[2.2.2]oct-3-yle,
R² représente l'hydrogène,
R³ représente l'hydrogène, le chlore, le fluor ou un reste méthyle,
A représente l'oxygène ou le soufre,
et
Z représente l'hydrogène, un halogène, un reste formyle, carbamoyle, cyano, trifluorométhyle, trifluorométhoxy, nitro, amino, formamido, acétamido, méthyle, éthyle, méthoxy, éthoxy, alkylamino en C₁ à C₄, furylcarbonylamino, phénylcarbonylamino, méthylsulfonylamino, di(phénylsulfonyl)amino, cyclopropylcarbonylméthyle ou amino(hydroxyimino)méthyle,
et leurs sels, leurs produits de solvatation et les produits de solvatation des sels.

8. Composés suivant la revendication 1, de formule (Ia)
dans laquelle
R¹ représente un groupe (3*R*)-1-aza-bicyclo[2.2.2]oct-3-yle,
R² représente l'hydrogène,
R³ représente l'hydrogène, le chlore, le fluor ou un reste méthyle,
A représente l'oxygène ou le soufre,
et
Z représente l'hydrogène, un halogène, un reste formyle, carbamoyle, cyano, trifluorométhyle, trifluorométhoxy, nitro, amino, formamido, acétamido, méthyle, éthyle, méthoxy, éthoxy, alkylamino en C₁ à C₄, furylcarbonylamino, phénylcarbonylamino, méthylsulfonylamino, di(phénylsulfonyl)amino, cyclopropylcarbonylméthyle et amino(hydroxyimino)méthyle,
et leurs sels, leurs produits de solvatation et les produits de solvatation des sels.

9. Composés suivant la revendication 1 de formule (I), choisis dans le groupe des composés N-[(3R)-1-azabicyclo[2.2.2]oct-3-yl]-6-amino-1-benzofuranne-2-carboxamide, chlorhydrate de N-[(3R)-1-azabicyclo[2.2.2]oct-3-yl]-5-fluoro-1-benzofuranne-2-carboxamide, chlorhydrate de N-[(3R)-1-azabicyclo[2.2.2]oct-3-yl]-7-fluoro-1-benzofuranne-2-carboxamide, chlorhydrate de N-[(3R)-1-azabicyclo-[2.2.2]oct-3-yl]-3-fluoro-1-benzothiophène-2-carboxamide, chlorhydrate de N-[(3R)-1-azabicyclo[2.2.2]oct-3-yl]-7-trifluorométhyl-1-benzothiophène-2-carboxamide, dichlorhydrate de chlorhydrate de N-[(3R)-1-azabicyclo[2.2.2]oct-3-yl]-7-amino-1-benzothiophène-2-carboxamide, chlorhydrate de N-[(3R)-1-azabicyclo[2.2.2]oct-3-yl]-7-chloro-1-benzothiophène-2-carboxamide, dichlorhydrate de 6-amino-N-[(3R)-1-azabicyclo[2.2.2]oct-3-yl]-1-benzothiophène-2-carboxamide, chlorhydrate de N-[(3R)-1-azabicyclo[2.2.2]oct-3-yl]-5-fluoro-1-benzothiophène-2-carboxamide, chlorhydrate de N-[(3R)-1-azabicyclo[2.2.2]oct-3-yl]-7-méthoxy-1-benzothiophène-2-carboxamide, chlorhydrate de N-[(3R)-1-azabicyclo[2.2.2]oct-3-yl]-5,7-difluoro-1-benzothiophène-2-carboxamide, chlorhydrate de 6-(acétylamino)-N-[(3R)-1-azabicyclo[2.2.2]oct-3-yl]-1-benzothiophène-2-carboxamide, dichlorhydrate de N-[(3R)-1-azabicyclo[2.2.2]oct-3-yl]-6-(isopropylamino)-1-benzothiophène-2-carboxamide, chlorhydrate de N-[(3R)-1-azabicyclo-[2.2.2]oct-3-yl]-7-cyano-1-benzothiophène-2-carboxamide, et chlorhydrate de N-[(3R)-1-azabicyclo[2.2.2]oct-3-yl]-6-[(méthylsulfonyl)amino]-1-benzothiophène-2-carboxamide et leurs sels, leurs produits de solvatation et les produits de solvatation des sels.

10. Procédé de production de composés de formule (I) suivant la revendication 1, **caractérisé en ce qu'**on fait réagir des composés de formule
R¹R²NH (II),
dans laquelle R¹ et R² ont la définition indiquée ci-dessus,
avec un composé de formule 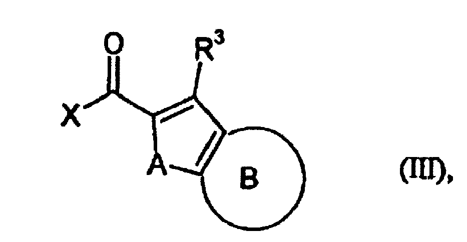 dans laquelle
R³, A et le noyau B ont les définitions indiquées dans la revendication 1, et
X est un groupe hydroxy ou un groupe partant convenable,
dans un solvant inerte, éventuellement en présence d'un agent de condensation et, le cas échéant, en présence d'une base.

11. Composés suivant l'une des revendications 1 à 9, destinés au traitement et/ou à la prophylaxie de maladies.

12. Médicament contenant au moins l'un des composés selon l'une des revendications 1 à 9 en combinaison avec au moins un support ou excipient essentiellement non toxique, pharmaceutiquement acceptable.

13. Utilisation de composés suivant l'une des revendications 1 à 9, pour la préparation d'un médicament destiné à améliorer la perception, la capacité de concentration, la capacité d'apprentissage et/ou la capacité de mémoire.

14. Utilisation de composés suivant l'une des revendications 1 à 9, pour la préparation d'un médicament destiné au traitement et/ou à la prophylaxie de troubles de la perception, de la capacité de concentration, de la capacité d'apprentissage et/ou de la capacité de mémoire.

15. Médicament suivant la revendication 12, destiné au traitement et/ou à la prophylaxie de troubles de la perception, de la capacité de concentration, de la capacité d'apprentissage et/ou de la capacité de mémoire.
